# EUROPEAN PATENT APPLICATION

(11) **EP 4 570 905 A1**
(43) Date of publication of application: **18.06.2025**
(21) Application number: 23852618.0
(22) Date of filing: 09.08.2023
(51) Int. Cl.: C12N 5/10, C12N 5/02

(54) **METHOD FOR PRODUCING PROLIFERATIVE MACROPHAGE-LIKE CELLS (PMAC)**

(30) Priority: 09.08.2022 JP 2022127477
(71) Applicant: AGC INC., Chiyoda-ku, Tokyo 1008405 (JP); National Cancer Center, Tokyo 104-0045 (JP)
(72) Inventor: FUKUDA, Kyoko, Kashiwa-shi, Chiba 277-8577 (JP); UEMURA, Yasushi, Kashiwa-shi, Chiba 277-8577 (JP); HAGIYA, Yuichiro, Tokyo 100-8405 (JP); IDE, Masamichi, Tokyo 100-8405 (JP); KARIYA, Masahiro, Tokyo 100-8405 (JP)
(74) Representative: Müller-Boré & Partner Patentanwälte PartG mbB
(86) International application number: PCT/JP2023/029198
(87) International publication number: WO 2024/034656

(57) **Abstract**

The present invention provides a proliferating macrophage-like cell (pMAC) characterized by proliferation in a cytokine-dependent manner, a production method thereof, and a method for producing CAR-pMAC, including (1) a step of, in a pluripotent stem cell, deleting the gene that inhibits a macrophage-like function or suppressing expression of the gene, and then inducing differentiation into a myeloid cell to obtain a macrophage-like cell (MAC) (step 1), (2) a step of expressing the gene that confers proliferating property in the MAC obtained in step 1 to obtain a proliferating macrophage-like cell (pMAC) (step 2), and (3) a step of introducing a chimeric antigen receptor (CAR) (step 3). Furthermore, the present invention provides a pharmaceutical use of CAR-pMAC. According to the present invention, it is possible to provide a novel CAR-loaded cell platform, particularly, a novel CAR-loaded cell platform superior in stable quality, stable supply, and economic efficiency, particularly, a novel CAR-loaded cell platform superior as a medicament.

## Description

### [Technical Field]

The present invention relates to proliferating macrophage-like cells (pMAC) derived from pluripotent stem cells and a production method thereof, a method for producing CAR-pMAC including introducing a chimeric antigen receptor (CAR) gene into the pMAC, CAR-pMAC produced by the method, and the like.

### [Background Art]

One of the immunotherapies for cancer patients is a treatment method using chimeric antigen receptor (CAR) T cells (hereinafter also simply referred to as CAR-T cells), in which T cell receptor (TCR) of cytotoxic T cells (CTL) is genetically modified to directly and selectively recognize tumor cells and an antitumor effect is exerted (Non Patent Literature 1). Cancer treatment using CAR-T cells kills cancer cells based on a mechanism different from that of conventional anticancer drugs and radiation therapy, and is therefore expected to be effective against intractable or treatment-resistant cancers. CAR-T cells have already been formulated for some blood tumors, such as leukemia and malignant lymphoma. However, their effect on solid cancers is limited.

CAR-T cells therapy has the following essential problems.
1. No targeting to cancer tissue (no receptors to approach cancer tissue)
2. No infiltration into cancer tissue
3. Decreased function and dysfunction (exhaustion) due to interaction with tumor microenvironment (TME)
4. Loss of target antigen due to genetic mutation in cancer = cancer escape (treatment resistance)
5. Concerns about cytokine release syndrome (CRS)

In light of these problems, attempts have been made both in Japan and abroad to improve the therapy. Examples include the introduction of cancer-directed receptors (CCR2, CXCR2), improvements to intracellular signaling domain of CAR, immune checkpoint control (anti-PD-1 antibody, PD-1-CD28), endowment with cytokine production ability (IL7, 12, 15, 18), rejuvenated T/NK cells (iPS-T/NK cells, SCM-T cells), dual antigen recognition systems, and the like.

However, there are currently no reports showing remarkable clinical effects on solid cancers.

In recent years, preclinical test results have shown that a cell platform that loads CAR on macrophages, rather than T cells, is effective in treating solid cancers (Non Patent Literature 2).

This is based on the following characteristics/functions inherent to macrophages.
1. express cancer-targeting receptors and accumulate and infiltrate into cancers
2. not suffer from PD-1/PD-L1-mediated functional decline
3. induce secondary immune response through activation (type 1 macrophage (M1) transformation)

In addition, Patent Literatures 1, 2 describe monocytes, macrophages, and dendritic cells carrying CAR, and pharmaceutical compositions containing these cells.

Thus, CAR-macrophage therapy can be a very promising cell immunotherapy, but in order to produce a cell formulation, stable quality, standardization (stable supply), and mass production (economic efficiency) are required. From the aspects of stable quality and stable supply, it is preferable that the cells carrying the CAR are allogeneic. Patent Literatures 2, 3 describe methods for differentiating pluripotent stem cells into myeloid cells.

In addition, a technology has been developed to introduce CAR into iMAC based on iPS cell-derived macrophages (Non Patent Literature 3). However, this method does not allow the final product to proliferate, and the differentiation induction performed from time to time is complicated, which raises concerns about soaring production costs.

### [Citation List]

### [Patent Literature]

[Patent Literature 1]
   JP-A-2021-511809
[Patent Literature 2]
   JP-A-2022-28831
[Patent Literature 3]
   WO 2021/230304

### [Non Patent Literature]

[Non Patent Literature 1]
   Eshhar Z. et al., Proc Natl Acad Sci U S A, 1993, 90: 720-724.
[Non Patent Literature 2]
   Klichinsky M. et al., Nat Biotechnol. 38(8):947-953, 2020
[Non Patent Literature 3]
   Su S. et al., Cells. 2022, 11: 1652

### [Summary of Invention]

### [Technical Problem]

The present invention aims to provide a novel CAR-loaded cell platform, in particular, to provide a CAR-loaded macrophage-like cell platform that is superior in stable quality, stable supply, and economic efficiency.

### [Solution to Problem]

In view of the above-mentioned problems, the present inventors have focused on macrophage cells as immune cells that carry a CAR. They have found that allogeneic cells can be used by using macrophage cells induced from pluripotent stem cells as macrophage cells that carry a CAR, and that large-scale production can be achieved by imparting cytokine-dependent proliferation activity to the macrophage cells, which resulted in the completion of the present invention.

Accordingly, the present invention provides the following.

[1] A proliferating macrophage-like cell (pMAC) that proliferates in a cytokine-dependent manner.
[2] The pMAC of [1], wherein the cytokine is a granulocyte macrophage colony stimulating factor (GM-CSF) and/or a macrophage colony stimulating factor (M-CSF).
[3] The pMAC of [1] or [2], wherein CD14 and CD83 are negative.
[4] The pMAC of any of [1] to [3], that is derived from a pluripotent stem cell.
[5] The pMAC of [4], wherein the aforementioned pluripotent stem cell is an induced pluripotent stem cell or an embryonic stem cell.
[6] The pMAC of any of [1] to [5], wherein a gene that inhibits a macrophage-like function is deleted or suppressed in expression.
[7] The pMAC of [6], wherein the aforementioned gene that inhibits a macrophage-like function is a signal regulatory protein α (SIRPα) gene.
[8] The pMAC of any of [1] to [7], wherein the aforementioned pMAC further comprises a chimeric antigen receptor (CAR).
[9] A method for producing a proliferating macrophage-like cell (pMAC), comprising a step of deleting a gene that inhibits a macrophage-like function or suppressing expression of the gene, and a step of expressing a gene that confers proliferating property.
[10] The production method of [9], comprising
   (1) a step of, in a pluripotent stem cell, deleting the gene that inhibits a macrophage-like function or suppressing expression of the gene, and then inducing differentiation into a myeloid cell to obtain a macrophage-like cell (MAC) (step 1), and
   (2) a step of expressing the gene that confers proliferating property in the MAC obtained in step 1 to obtain a proliferating macrophage-like cell (pMAC) (step 2).
[11] The production method of [9], comprising
   (A) a step of expressing a gene that confers proliferating property in a myeloid cell differentiated from a pluripotent stem cell to obtain a proliferating myeloid cell (step A), and
   (B) a step of, in the proliferating myeloid cell obtained in step A, deleting the gene that inhibits a macrophage-like function or suppressing expression of the gene, and then inducing differentiation to obtain a proliferating macrophage-like cell (pMAC) (step B).
[12] The production method of any of [9] to [11], wherein the gene that inhibits a macrophage-like function is a signal regulatory protein α (SIRPα) gene.
[13] The production method of any of [9] to [12], wherein the gene that confers proliferating property is at least one type selected from the group consisting of c-MYC, BMI1 and MDM2.
[14] A method for producing CAR-pMAC, comprising introducing a chimeric antigen receptor (CAR) into the pMAC obtained by the production method of any of [9] to [13].
[15] A method for producing CAR-pMAC, comprising
   (1) a step of, in a pluripotent stem cell, deleting the gene that inhibits a macrophage-like function or suppressing expression of the gene, and then inducing differentiation into a myeloid cell to obtain a macrophage-like cell (MAC) (step 1),
   (2) a step of expressing the gene that confers proliferating property in the MAC obtained in step 1 to obtain a proliferating macrophage-like cell (pMAC) (step 2), and
   (3) a step of introducing a chimeric antigen receptor (CAR) (step 3).
[16] The production method of [15], wherein the aforementioned pluripotent stem cell is an induced pluripotent stem cell or an embryonic stem cell.
[17] The production method of [15] or [16], wherein the differentiation induction into the myeloid cell in step 1 is performed by the following method (i) or (ii).
   (i) performing a layered culture of embryoid body (EB) induced from the pluripotent stem cells on feeder cells in a medium containing VEGF, and further culturing same in a medium containing VEGF, SCF, and TPO, or
   (ii) performing a monolayer culture of embryoid body (EB) induced from the pluripotent stem cells in a medium containing BMP-4, VEGF, and SCF without feeder cells, and further culturing same in a medium containing VEGF, TPO, and GM-CSF.
[18] The production method of [17], wherein the induction from the pluripotent stem cell into the embryoid body (EB) is performed by the following methods (a) and (b):
   (a) seeding pluripotent stem cells in a container for cell mass-forming culture to form an embryoid body (EB), wherein (b) the aforementioned container has fine spheroid wells at the bottom, and does not have a flat surface between adjacent wells.
[19] A method for producing CAR-pMAC, comprising
   (1) a step of, in a pluripotent stem cell, deleting the gene that inhibits a macrophage-like function or suppressing expression of the gene (step 1-1),
      a step of seeding the pluripotent stem cell obtained in step 1-1, in which the gene that inhibits a macrophage-like function is deleted or expression of the gene is suppressed, in a container for cell mass-forming culture that has fine spheroid wells at the bottom, and does not have a flat surface between adjacent wells, to form an embryoid body (EB) (step 1-2),
      a step of obtaining a macrophage-like cell (MAC) by the following method (i) or (ii) from the EB obtained in step 1-2 (step 1-3),
         (i) performing a layered culture of the aforementioned EB on feeder cells in a medium containing VEGF, and further culturing same in a medium containing VEGF, SCF, and TPO, or
         (ii) performing a monolayer culture of the aforementioned EB in a medium containing BMP-4, VEGF, and SCF without feeder cells, and further culturing same in a medium containing VEGF, TPO, and GM-CSF,
   (2) a step of expressing the gene that confers proliferating property in the MAC obtained in step 1-3 to obtain a proliferating macrophage-like cell (pMAC) (step 2), and
   (3) a step of introducing a chimeric antigen receptor (CAR) into the pMAC obtained in step 2 to obtain CAR-pMAC (step 3).
[20] The production method of any of [15] to [19], wherein the gene that inhibits a macrophage-like function is a signal regulatory protein α (SIRPα) gene.
[21] The production method of any of [15] to [20], wherein the gene that confers proliferating property is at least one type selected from the group consisting of c-MYC, BMI1 and MDM2.
[22] A proliferating macrophage-like cell that satisfies the following conditions when a main component analysis based on the gene expression profile obtained by performing a whole-transcriptome analysis using, as a comparison target, a cell population along the macrophage cell lineage consisting of an iPS cell-derived myeloid cell, an iPS cell-derived macrophage, a biologically derived monocyte cell, a biologically derived macrophage M1, a biologically derived macrophage M2, and a biologically derived macrophage M0 is performed and the top two components with high contribution rates are selected as the analysis targets:
   condition 1: no overlapping with plots of known macrophage cell lineages
   condition 2: PC1 is 100 or more, and PC2 is plotted in the range of -80 to 0.
[23] A pharmaceutical composition comprising the proliferating macrophage-like cell of [8] and a pharmaceutically acceptable carrier.
[24] The pharmaceutical composition of [23], which is for treating at least one disease selected from the group consisting of a disease associated with a tumor or cancer, a neurodegenerative disease, an inflammatory disease, a cardiovascular disease, a fibrotic disease, and amyloidosis.
[25] A method for treating at least one disease selected from the group consisting of a disease associated with a tumor or cancer, a neurodegenerative disease, an inflammatory disease, a cardiovascular disease, a fibrotic disease, and amyloidosis, comprising administering an effective amount of the proliferating macrophage-like cell of [8] to a subject in need thereof.
[26] The proliferating macrophage-like cell of [8] for use in treating at least one disease selected from the group consisting of a disease associated with a tumor or cancer, a neurodegenerative disease, an inflammatory disease, a cardiovascular disease, a fibrotic disease, and amyloidosis.

### [Advantageous Effects of Invention]

According to the method of the present invention, it is possible to produce CAR-loaded proliferating macrophage-like cells (CAR-pMAC) that are stable in quality, can be supplied stably, and are superior in economic efficiency. The CAR-loaded proliferating macrophage-like cells are also effective in treating solid cancers.

### [Brief Description of Drawings]

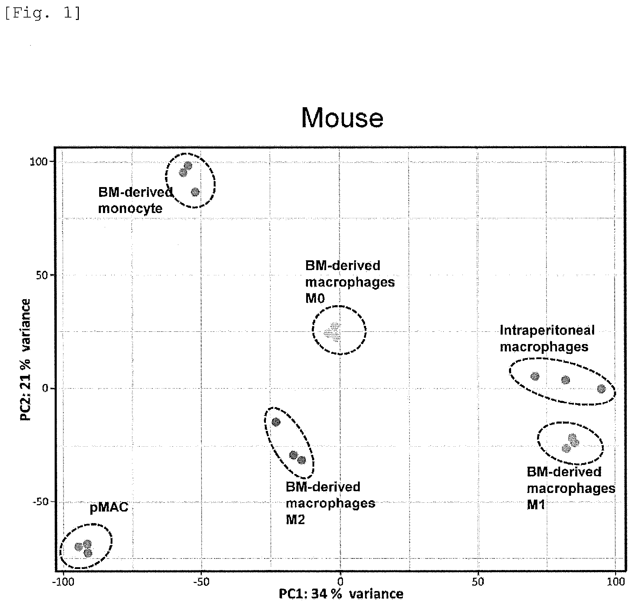

[Fig. 1] A diagram showing the gene expression profile of each cell obtained by RNA sequencing analysis, followed by principal component analysis, of mouse pMAC, bone marrow-derived monocyte (BM-derived monocyte), bone marrow-derived macrophage M0 (BM-derived macrophages M0), mouse bone marrow-derived M1-type macrophage (BM-derived macrophages M1), mouse bone marrow-derived M2-type macrophage (BM-derived macrophages M2) and abdominal cavity-derived macrophage (Intraperitoneal macrophages), in which the first principal component (PC1: contribution rate 34%) is plotted on the horizontal axis, and the second principal component (PC2: contribution rate 21%) is plotted on the horizontal axis. A closer distance between the plots of each cell shows that the gene expression profiles are more similar; conversely, a farther distance between the plots of each cell shows that the gene expression profiles are different.

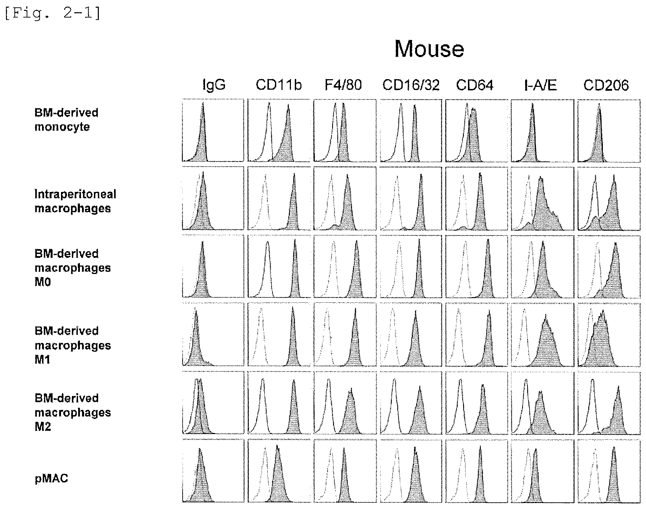

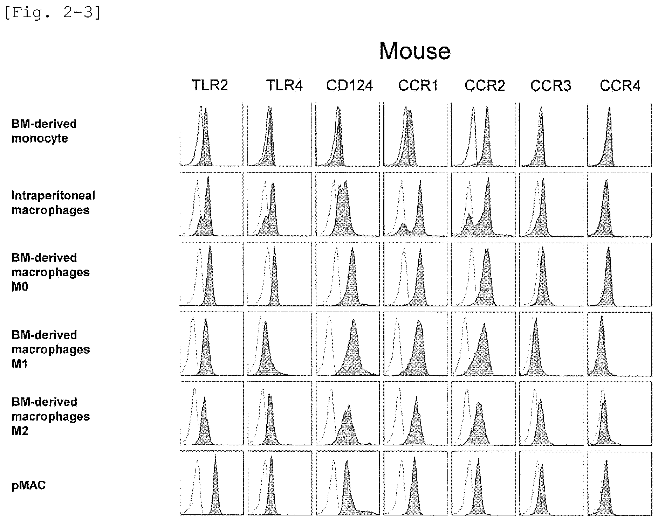

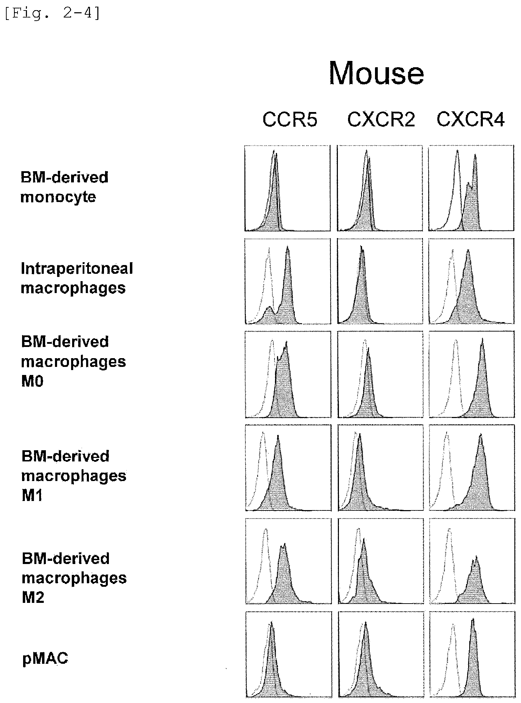

[Fig. 2-1] Histograms showing the results of surface antigen analysis of CD11b, F4/80, CD16/32, CD64, I-A/E, and CD206 by flow cytometry of mouse bone marrow-derived monocyte (BM-derived monocyte), mouse intraperitoneal macrophage (Intraperitoneal macrophages), mouse bone marrow-derived macrophage (BM-derived macrophages M0), mouse bone marrow-derived M1-type macrophage (BM-derived macrophages M1), mouse bone marrow-derived M2-type macrophage (BM-derived macrophages M2) and mouse pMAC. The vertical axis indicates the percentage of cells present, and the horizontal axis indicates the fluorescence intensity of each measured marker.

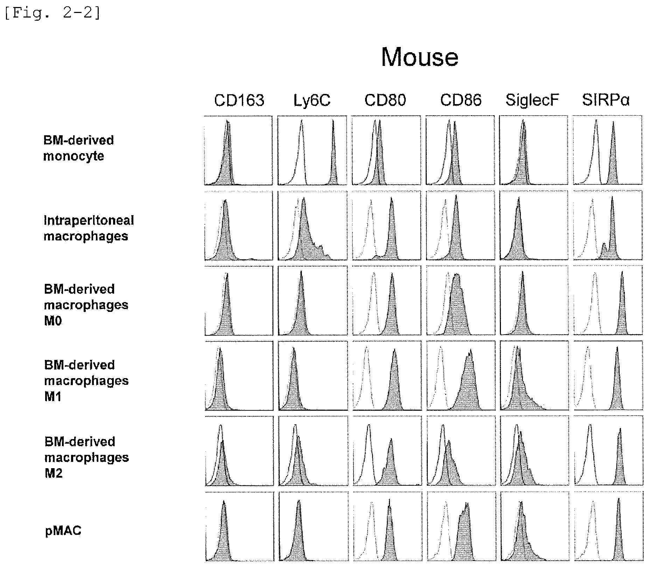

[Fig. 2-2] A histogram showing the analysis results of surface antigens CD163, Ly6C, CD80, CD86, SiglecF and SIRPα by flow cytometry of mouse bone marrow-derived monocyte (BM-derived monocyte), mouse intraperitoneal macrophage (Intraperitoneal macrophages), mouse bone marrow-derived macrophage (BM-derived macrophages M0), mouse bone marrow-derived M1-type macrophage (BM-derived macrophages M1), mouse bone marrow-derived M2-type macrophage (BM-derived macrophage M2) and mouse pMAC. The vertical axis indicates the proportion (%) of cells present, and the horizontal axis indicates the fluorescence intensity of each measured marker.
[Fig. 2-3] Histograms showing the results of surface antigen analysis of TLR2, TLR4, CD124, CCR1, CCR2, CCR3 and CCR4 by flow cytometry of mouse bone marrow-derived monocyte (BM-derived monocyte), mouse intraperitoneal macrophage (Intraperitoneal macrophages), mouse bone marrow-derived macrophage (BM-derived macrophages M0), mouse bone marrow-derived M1-type macrophage (BM-derived macrophages M1), mouse bone marrow-derived M2-type macrophage (BM-derived macrophages M2) and mouse pMAC. The vertical axis indicates the proportion (%) of cells present, and the horizontal axis indicates the fluorescence intensity of each measured marker.
[Fig. 2-4] Histogram showing the results of surface antigen analysis of CCR5, CXCR2 and CXCR4 by flow cytometry of mouse bone marrow-derived monocyte (BM-derived monocyte), mouse intraperitoneal macrophage (Intraperitoneal macrophages), mouse bone marrow-derived macrophage (BM-derived macrophages M0), mouse bone marrow-derived M1-type macrophage (BM-derived macrophages M1), mouse bone marrow-derived M2-type macrophage (BM-derived macrophages M2) and mouse pMAC. The vertical axis indicates the proportion (%) of cells present, and the horizontal axis indicates the fluorescence intensity of each measured marker.

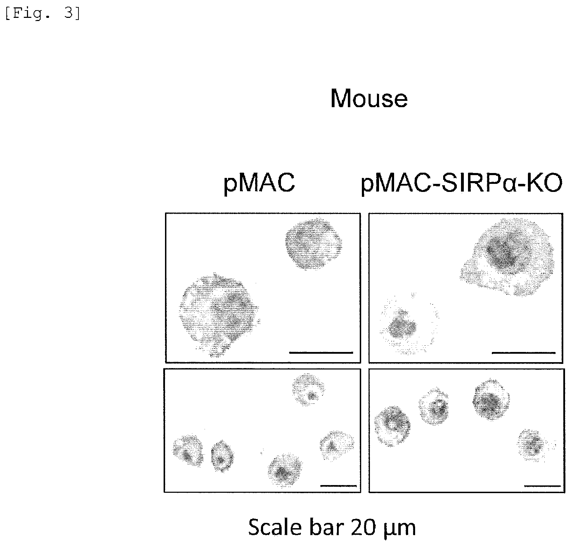

[Fig. 3] Microscope images of mouse pMAC and pMAC-SIRPα-KO lacking the SIRPα gene, that are stained with May-Giemsa. The scale bar indicates 20 µm. Compared to pMAC, no morphological changes characteristic of lack of SIRPα gene were observed.

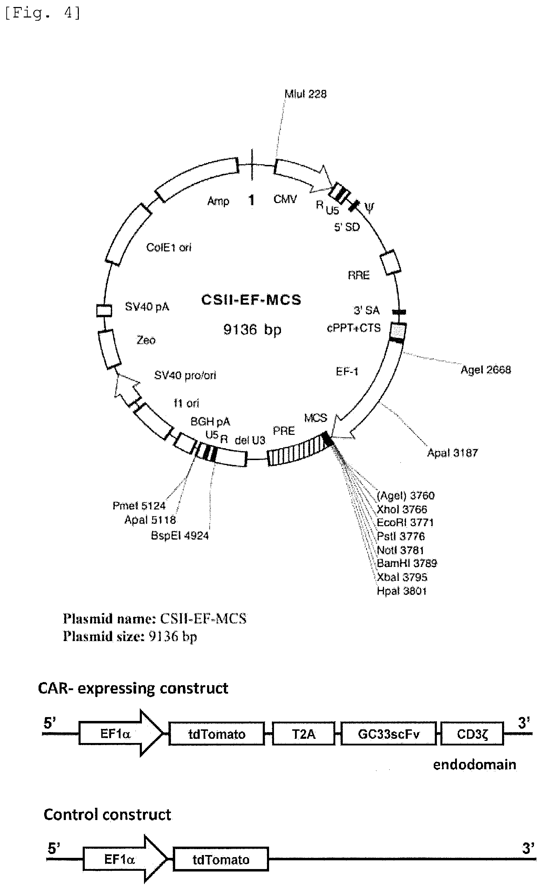

[Fig. 4] A vector map (upper row) of the lentiviral vector used to introduce the GPC3-specific chimeric antigen receptor (GPC3-CAR) gene into pMAC, and CAR-expressing construct (lower row).

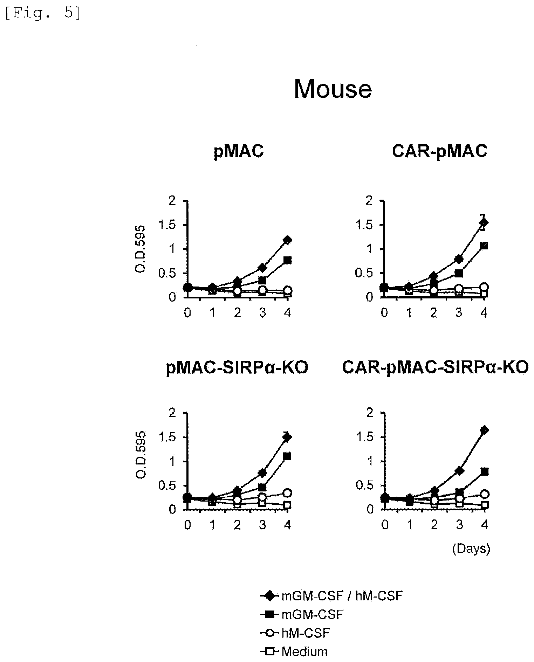

[Fig. 5] Line graphs showing the results of an MTT assay investigating the effect of a medium containing GM-CSF and/or M-CSF (αMEM containing 20% FBS) on the proliferation rate of pMAC. The results of mouse pMAC (upper left), CAR-pMAC (upper right), pMAC-SIRPα-KO (lower left), and CAR-pMAC-SIRPα-KO (lower right) are shown. The vertical axis indicates the concentration of formazan metabolized from MTT reagent (evaluated as absorbance at 595 nm) and the horizontal axis indicates the number of days of culture. There was no change in cytokine-dependent cell proliferation ability due to the deletion of the SIRPα gene and/or the introduction of CAR against the cancer antigen GPC3, which were performed to produce pMAC.

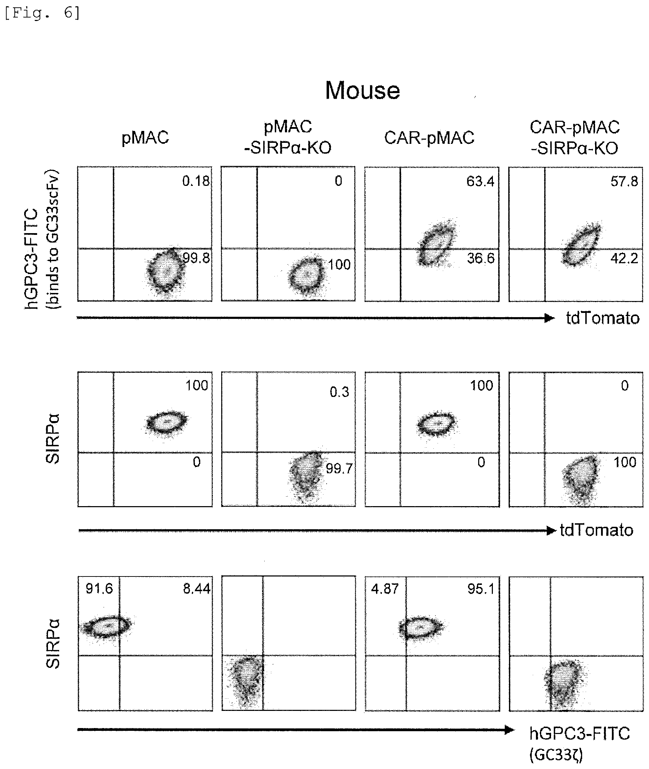

[Fig. 6] Cytograms showing the results of expression analysis of hGPC3-FITC, SIRPα, and tdTomato by flow cytometer in mouse pMAC, pMAC-SIRPα-KO, CAR-pMAC, and CAR-pMAC-SIRPα-KO. The vertical axis indicates the fluorescence intensity of hGPC3-FITC, and the horizontal axis indicates the fluorescence intensity of tdTomato (top). The vertical axis indicates the fluorescence intensity of SIRPα, and the horizontal axis indicates the fluorescence intensity of tdTomato (middle). The vertical axis indicates the fluorescence intensity of SIRPα, and the horizontal axis indicates the fluorescence intensity of hGPC3-FITC (bottom). pMAC expressing GPC3-CAR was obtained as a single cell population, indicating that it is highly pure and contains almost no untargeted cells.

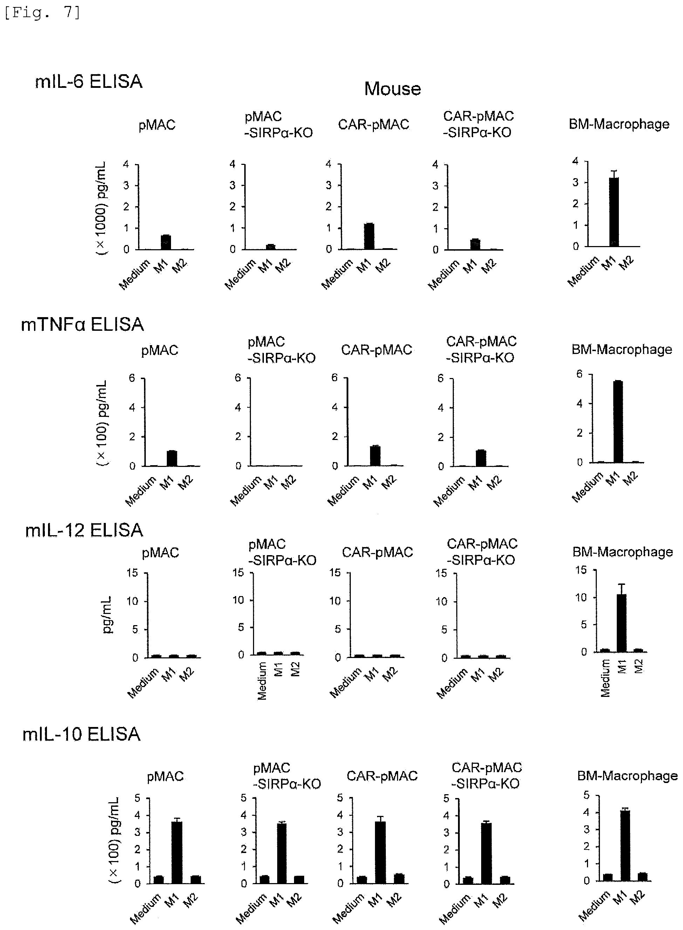

[Fig. 7] ELISA measurement results of production of cytokines IL-6, TNFα, IL-12, and IL-10, when mouse pMAC, pMAC-SIRPα-KO, CAR-pMAC, CAR-pMAC-SIRPα-KO and mouse bone marrow-derived macrophage (BM-Macrophage) were given a medium without stimulating components (Medium), M1 stimulation with interferon-γ and LPS, and M2 stimulation with IL-4. The vertical axis indicates the concentration (pg/mL) of the measured cytokine.

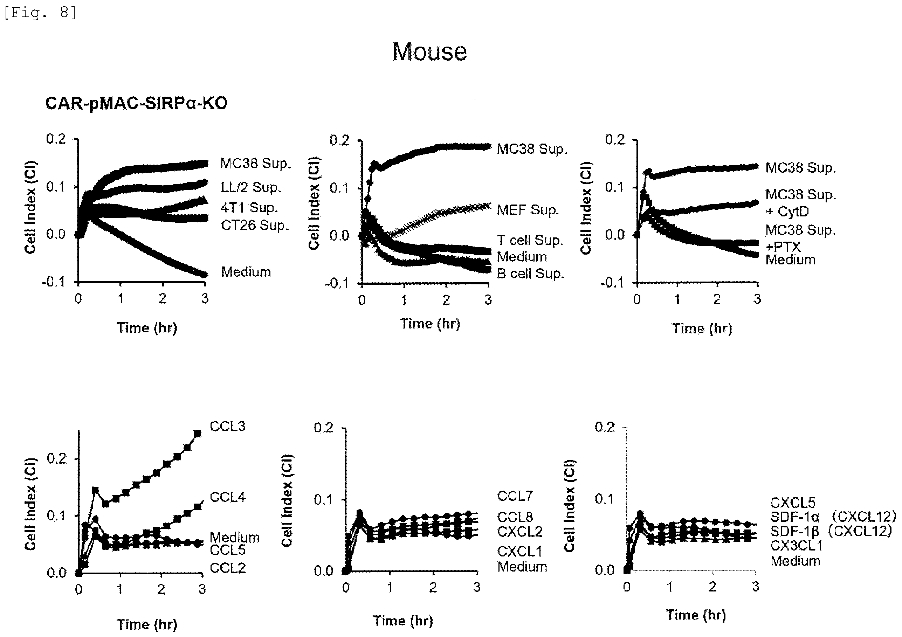

[Fig. 8] Real-time measurement results of cell migration ability of mouse CAR-pMAC-SIRPα-KO using the Boyden chamber-type xCELLigence DP system. Cell migration ability toward culture supernatants derived from mouse cancer cells MC38, LL/2, 4T1, and CT26, and toward fresh medium (Medium) (upper left); cell migration ability toward culture supernatants derived from mouse cancer cell MC38, and mouse embryonic fibroblasts MEF, T cell and B cell, which are mouse normal cells, and toward fresh medium (Medium) (upper center); cell migration ability of mouse CAR-pMAC-SIRPα-KO that was untreated, treated with cytochalasin D (+CytD), or treated with Bordetella pertussis toxin (+PTX) toward culture supernatants from mouse cancer cells MC38, or toward fresh medium (Medium) (upper right). Cell migration ability toward recombinant CCL2, CCL3, CCL4, CCL5, and fresh medium (Medium) (lower left); cell migration ability toward recombinant CCL7, CCL8, CXCL1, CXCL2, and fresh medium (Medium) (lower center); cell migration ability toward recombinant CXCL5, SDF-1α (CXCL12), SDF-1β (CXCL12), CX3CL1, and fresh medium (Medium) (lower right). The horizontal axis indicates the culture time, and the vertical axis indicates Cell Index (CI), which indicates the degree of cell migration ability relative to the start of measurement (0 hr).

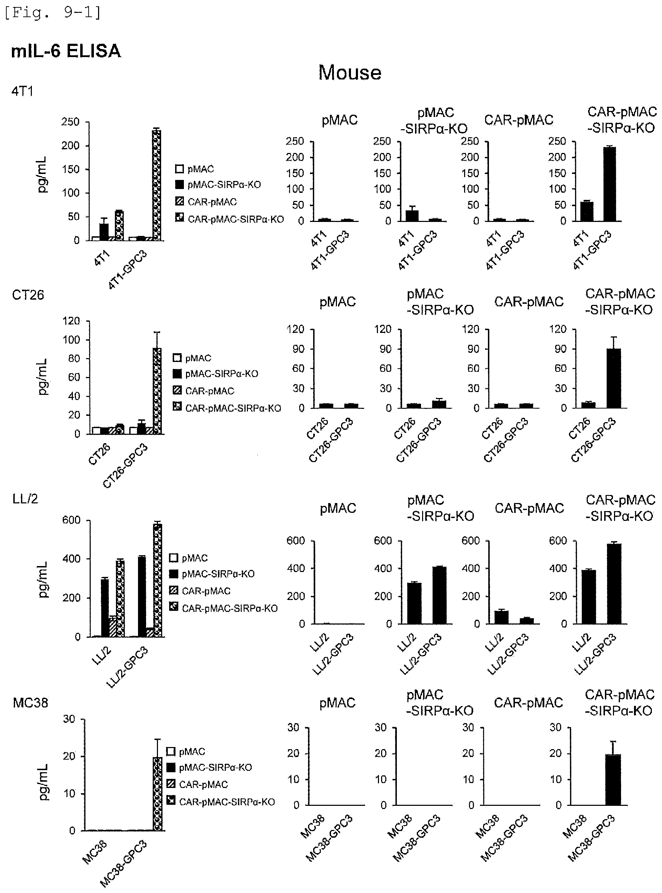

[Fig. 9-1] ELISA measurement results of cytokine IL-6 production in culture supernatants when mouse pMAC, pMAC-SIRPα-KO, CAR-pMAC, or CAR-pMAC-SIRPα-KO were co-cultured with mouse cancer cells (with or without GPC3 expression) for 48 hr. The results of co-culturing the aforementioned four types of pMAC with breast cancer cell line 4T1-mock (without GPC3 expression) or 4T1-GPC3 (with GPC3 expression) (top row), colorectal cancer cell line CT26-mock (without GPC3 expression) or CT26-GPC3 (with GPC3 expression) (second row from the top), lung cancer cell line LL/2-mock (without GPC3 expression) or LL/2-GPC3 (with GPC3 expression) (third row from the top), and colorectal cancer cell line MC38-mock (without GPC3 expression) or MC38-GPC3 (with GPC3 expression) (bottom row).

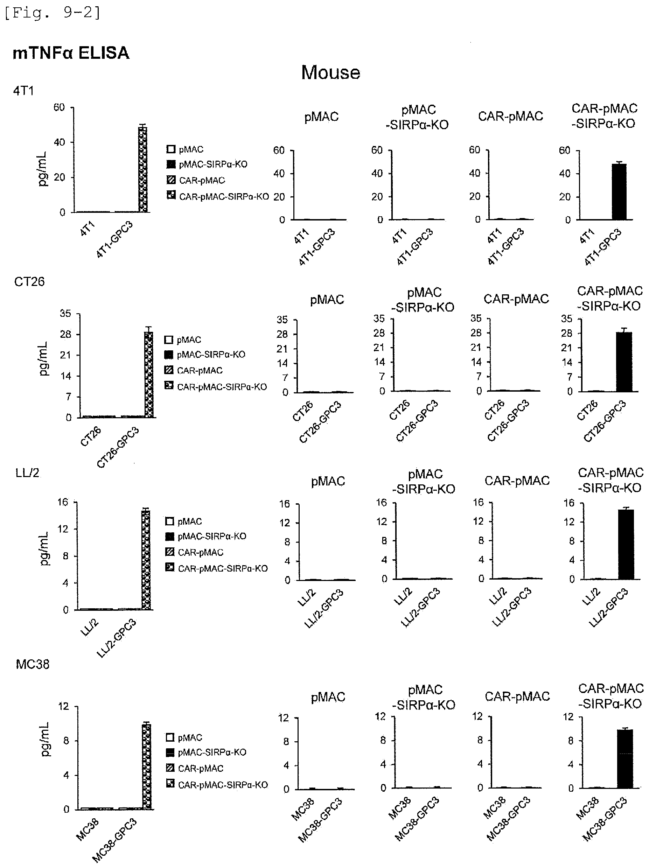

[Fig. 9-2] ELISA measurement results of cytokine TNFα production in culture supernatants when mouse pMAC, pMAC-SIRPα-KO, CAR-pMAC, or CAR-pMAC-SIRPα-KO were co-cultured with mouse cancer cells (with or without GPC3 expression) for 48 hr. The results of co-culturing the aforementioned four types of pMAC with breast cancer cell line 4T1-mock (without GPC3 expression) or 4T1-GPC3 (with GPC3 expression) (top row), colorectal cancer cell line CT26-mock (without GPC3 expression) or CT26-GPC3 (with GPC3 expression) (second row from the top), lung cancer cell line LL/2-mock (without GPC3 expression) or LL/2-GPC3 (with GPC3 expression) (third row from the top), and colorectal cancer cell line MC38-mock (without GPC3 expression) or MC38-GPC3 (with GPC3 expression) (bottom row).

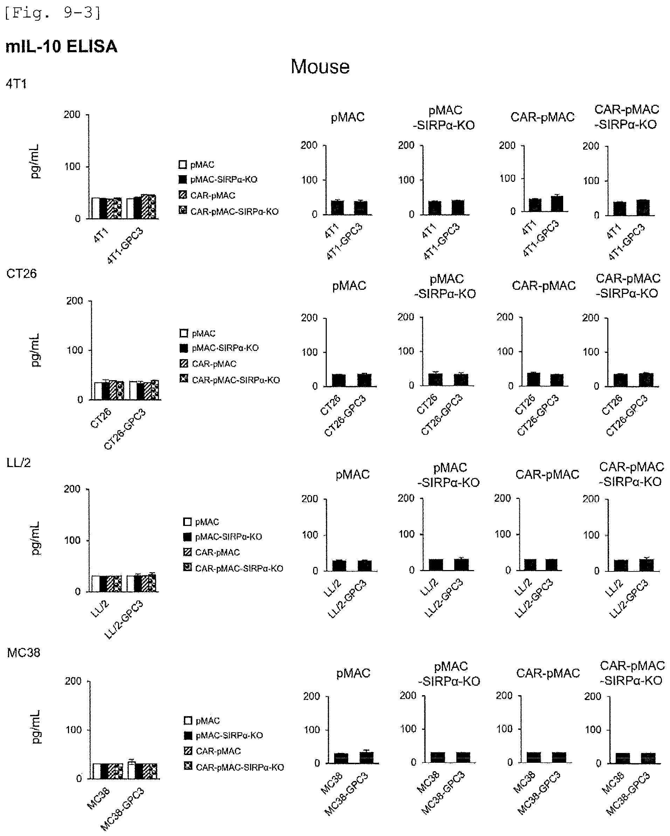

[Fig. 9-3] ELISA measurement results of cytokine IL-10 production in culture supernatants when mouse pMAC, pMAC-SIRPα-KO, CAR-pMAC, or CAR-pMAC-SIRPα-KO were co-cultured with mouse cancer cells (with or without GPC3 expression) for 48 hr. The results of co-culturing the aforementioned four types of pMAC with breast cancer cell line 4T1-mock (without GPC3 expression) or 4T1-GPC3 (with GPC3 expression) (top row), colorectal cancer cell line CT26-mock (without GPC3 expression) or CT26-GPC3 (with GPC3 expression) (second row from the top), lung cancer cell line LL/2-mock (without GPC3 expression) or LL/2-GPC3 (with GPC3 expression) (third row from the top), and colorectal cancer cell line MC38-mock (without GPC3 expression) or MC38-GPC3 (with GPC3 expression) (bottom row).

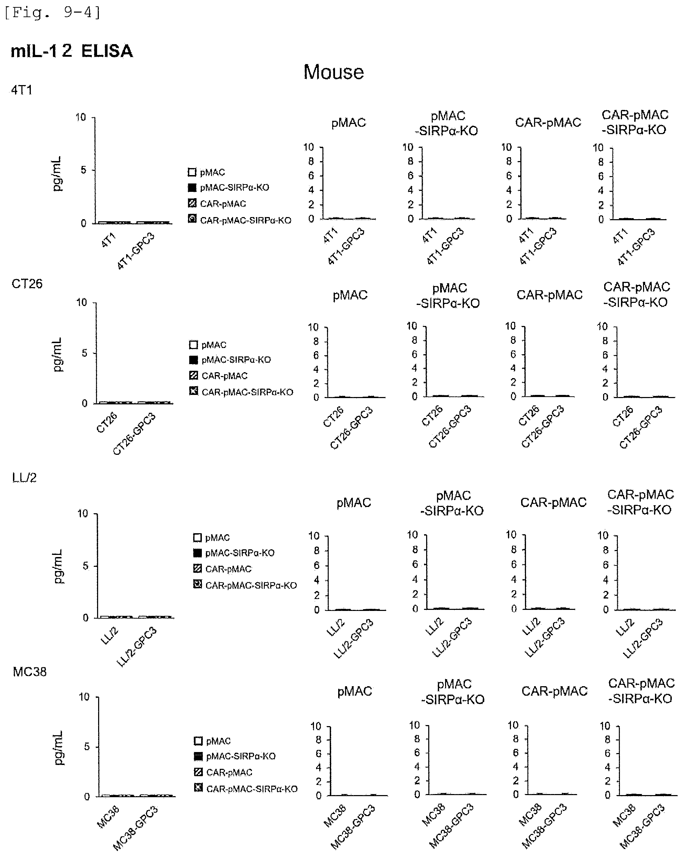

[Fig. 9-4] ELISA measurement results of cytokine IL-12 production in culture supernatants when mouse pMAC, pMAC-SIRPα-KO, CAR-pMAC, or CAR-pMAC-SIRPα-KO were co-cultured with mouse cancer cells (with or without GPC3 expression) for 48 hr. The results of co-culturing the aforementioned four types of pMAC with breast cancer cell line 4T1-mock (without GPC3 expression) or 4T1-GPC3 (with GPC3 expression) (top row), colorectal cancer cell line CT26-mock (without GPC3 expression) or CT26-GPC3 (with GPC3 expression) (second row from the top), lung cancer cell line LL/2-mock (without GPC3 expression) or LL/2-GPC3 (with GPC3 expression) (third row from the top), and colorectal cancer cell line MC38-mock (without GPC3 expression) or MC38-GPC3 (with GPC3 expression) (bottom row).

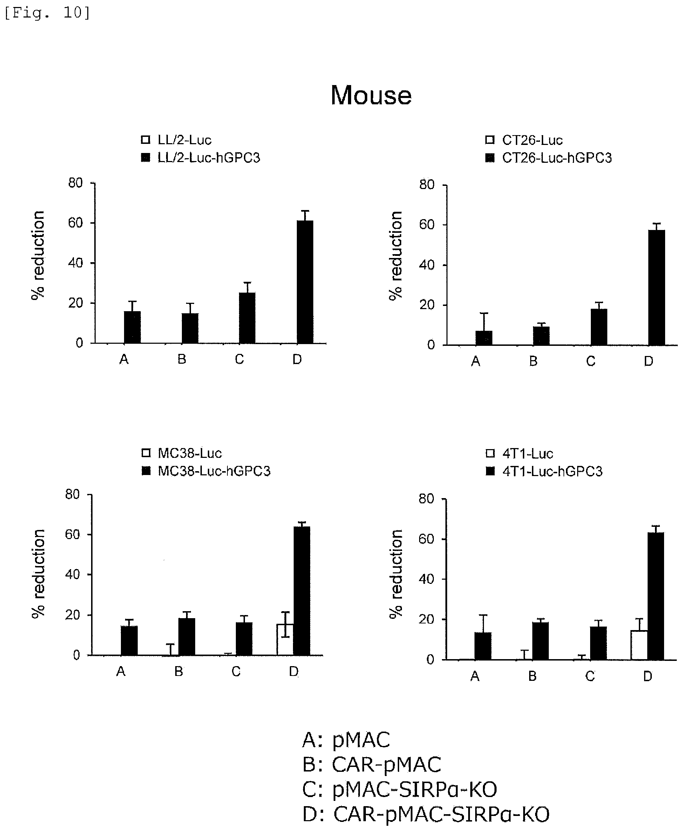

[Fig. 10] Bar graphs quantifying luciferase activity in cancer cells after co-culturing mouse pMAC(A), CAR-pMAC(B), pMAC-SIRPα-KO(C) and CAR-pMAC-SIRPα-KO(D) with lung cancer cell line LL/2 (with or without human GPC3 expression; upper left), colorectal cancer cell line CT26 (with or without human GPC3 expression; upper right), colorectal cancer cell line MC38 (with or without human GPC3 expression; lower left) and breast cancer cell line 4T1 (with or without human GPC3 expression; lower right), each introduced with luciferase (Luc) genes, for 48 hr. The vertical axis indicates the proportion (%) of decrease in cancer cells. White bars represent cancer cells that do not express hGPC3, and black bars represent cancer cells that express hGPC3. It can be seen that CAR-introduced pMAC(D) eliminates cancer cells that express the target cancer antigen human GPC (hGPC).

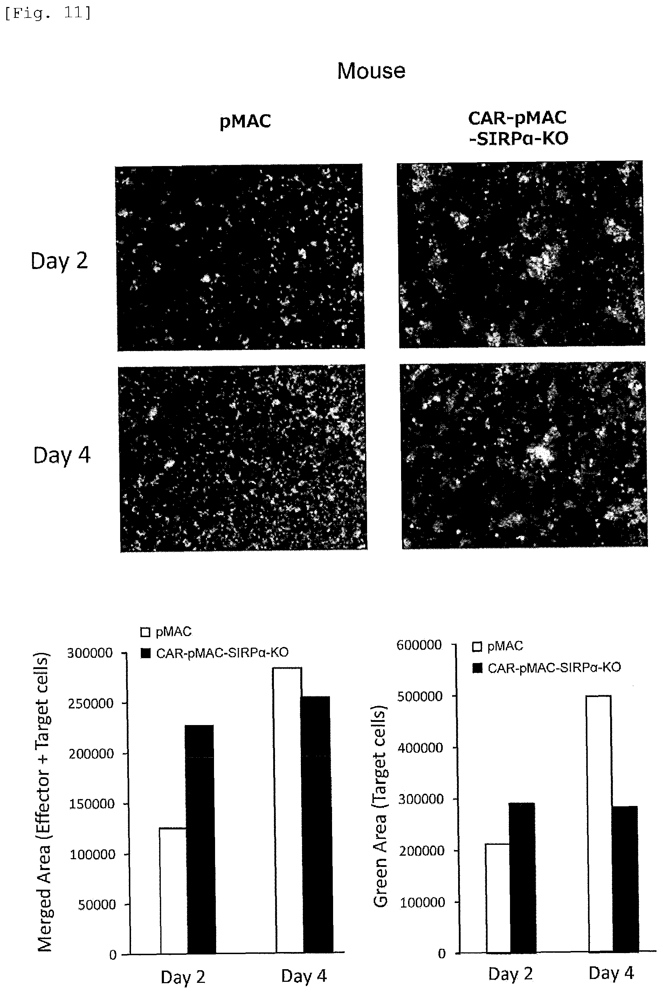

[Fig. 11] Fluorescence microscope observation images (upper row) taken 2 and 4 days after co-culture of mouse pMAC or CAR-pMAC-SIRPα-KO expressing tdTomato with a cancer cell line expressing Venus (with human GPC3 expression). Cancer cells are detected by green fluorescence (excitation wavelength 470 nm, absorption wavelength 525 nm) derived from the fluorescent protein Venus, and pMAC or CAR-pMAC-SIRPα-KO is detected by red fluorescence (excitation wavelength 545 nm, absorption wavelength 605 nm) derived from the fluorescent protein tdTomato. A bar graph plotting the area of the region where the green fluorescent signal of cancer cells and the red fluorescent signal of pMAC or CAR-pMAC-SIRPα-KO are overlapped (lower left). A bar graph plotting the area of the region where the green fluorescent signal of cancer cells is detected (lower right). It is found that cancer cells co-cultured with pMAC proliferated, whereas cancer cells co-cultured with CAR-pMAC-SIRPα-KO were surrounded and phagocytosed by CAR-pMAC-SIRPα-KO, and eliminated.

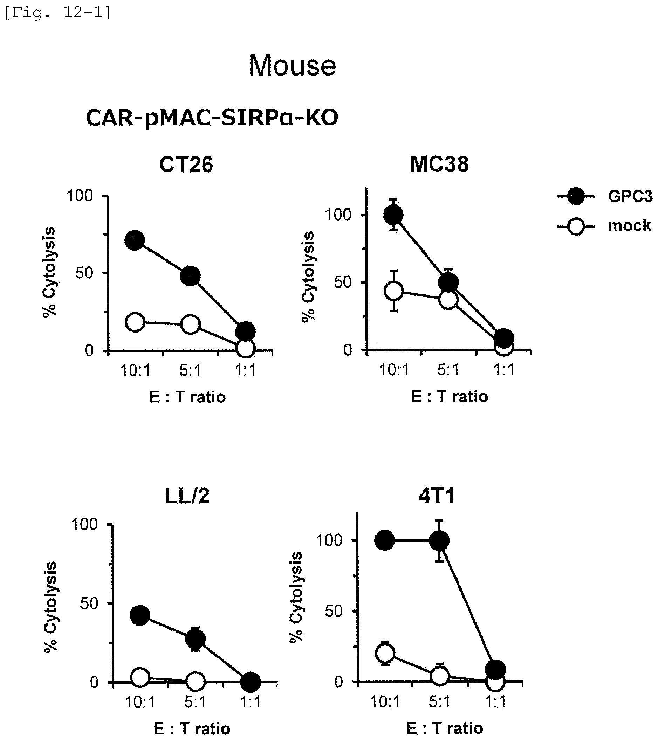

[Fig. 12-1] Graphs showing cytotoxic activity of mouse CAR-pMAC-SIRPα-KO to cancer cells, when mouse CAR-pMAC-SIRPα-KO was used as an effector cell and co-cultured with four types of mouse cancer cells as the target cells. Colorectal cancer cell line CT26 (with or without human GPC3 expression; upper left), colorectal cancer cell line MC38 (with or without human GPC3 expression; upper right), lung cancer cell line LL/2 (with or without human GPC3 expression; lower left), and breast cancer cell line 4T1 (with or without human GPC3 expression; lower right) were cultured for 24 hr, CAR-pMAC-SIRPα-KO was added in a 10-fold, 5-fold, or 1-fold amount of the seeded cancer cells and co-cultured, and the cytotoxic activity (% Cytolysis) after 6 hr was plotted. The vertical axis indicates the cytotoxic activity (% Cytolysis) of cancer cells, and the horizontal axis indicates the mixing ratio (E:T ratio) of effector cells (CAR-pMAC-SIRPα-KO) and target cells (cancer cells).

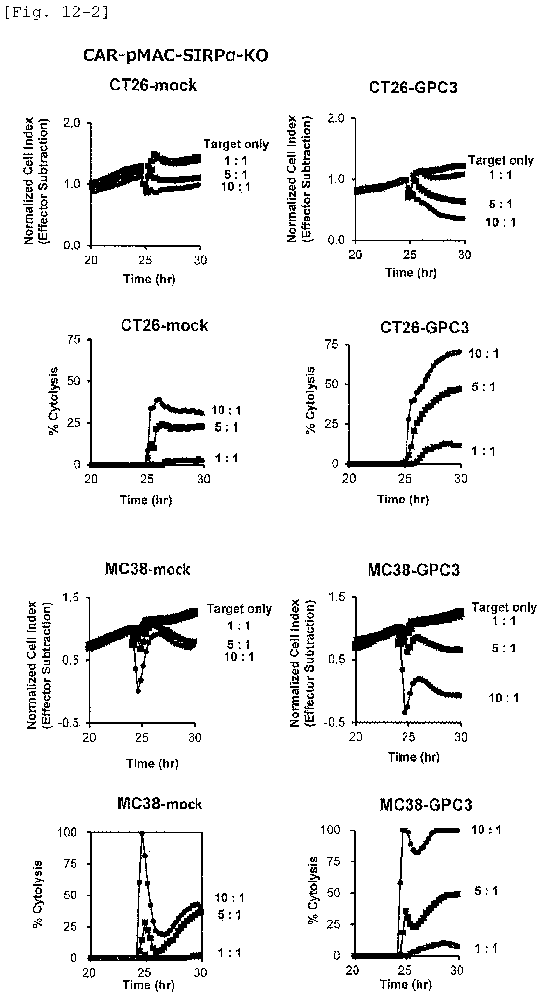

[Fig. 12-2] The results of measurement using the xCELLigence RTCA DP system of the cell survival signal Normalized Cell Index (top row) of the cancer cell CT26 and the cytotoxic activity (% Cytolysis) of mouse CT26 cells (second row from the top) by mouse CAR-pMAC-SIRPα-KO, when mouse CAR-pMAC-SIRPα-KO was used as an effector cell and co-cultured with target mouse cancer cells (with or without human GPC3 expression) at an E:T ratio of 10:1, 5:1, and 1:1. The results of measurement using the xCELLigence RTCA DP system of the cell survival signal Normalized Cell Index (third row from the top) of the cancer cell MC38 and the cytotoxic activity (% Cytolysis) of mouse MC38 cells (bottom row) by mouse CAR-pMAC-SIRPα-KO.

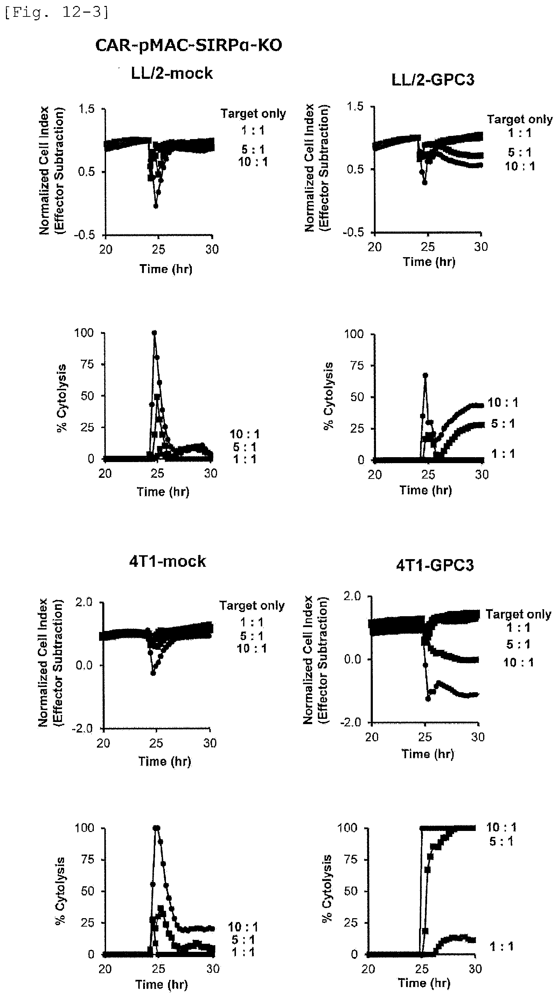

[Fig. 12-3] The results of measurement using the xCELLigence RTCA DP system of the cell survival signal Normalized Cell Index (top row) of the cancer cell LL/2 and the cytotoxic activity (% Cytolysis) of mouse LL/2 cells (second row from the top) by mouse CAR-pMAC-SIRPα-KO, when mouse CAR-pMAC-SIRPα-KO was used as an effector cell and co-cultured with target mouse cancer cells (with or without human GPC3 expression) at an E:T ratio of 10:1, 5:1, and 1:1. The results of measurement using the xCELLigence RTCA DP system of the cell survival signal Normalized Cell Index (third row from the top) of the cancer cell 4T1 and the cytotoxic activity (% Cytolysis) of mouse 4T1 cells (bottom row) by mouse CAR-pMAC-SIRPα-KO.

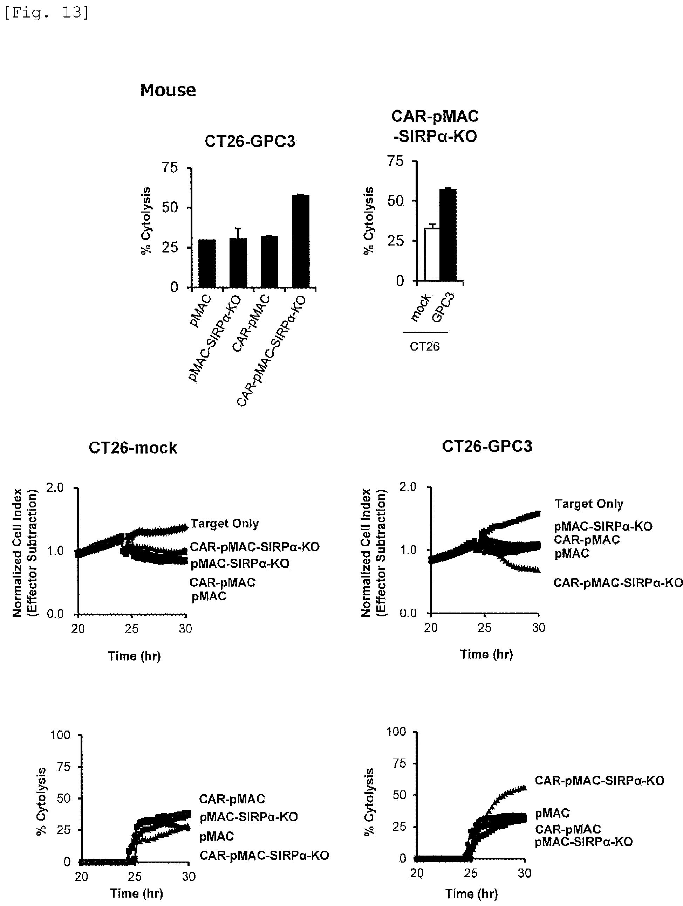

[Fig. 13] Bar graphs in which the cytotoxic activity (% Cytolysis) of CT26 is plotted on the vertical axis (top left), when mouse pMAC, pMAC-SIRPα-KO, CAR-pMAC and CAR-pMAC-SIRPα-KO were used as effector cells and co-cultured with target mouse CT26 cells (with GPC3 expression) at an E:T ratio of 10:1. A bar graph in which the cytotoxic activity (% Cytolysis) of CT26 cells is plotted on the vertical axis (top right), when mouse CAR-pMAC-SIRPα-KO was used as an effector cell and co-cultured with target mouse cancer cells CT26 (with or without GPC3 expression) at an E:T ratio of 10:1. A line graph (middle left) plotting the time course changes of the Normalized Cell Index, which indicates cell survival of CT26 (without GPC3 expression), on the vertical axis and the co-culture time on the horizontal axis, and a line graph (bottom left) plotting the cytotoxic activity (% Cytolysis) of mouse CT26 cells (without GPC3 expression) on the vertical axis, when mouse pMAC, pMAC-SIRPα-KO, CAR-pMAC and CAR-pMAC-SIRPα-KO were used as effector cells and co-cultured with target mouse CT26 cells (without GPC3 expression) at an E:T ratio of 10:1. A line graph (middle right) plotting the time course changes of the Normalized Cell Index, which indicates cell survival of CT26 (with GPC3 expression), on the vertical axis and the co-culture time on the horizontal axis, and a line graph (bottom right) plotting the cytotoxic activity (% Cytolysis) of mouse CT26 cells (with GPC3 expression) on the vertical axis, when mouse pMAC, pMAC-SIRPα-KO, CAR-pMAC and CAR-pMAC-SIRPα-KO were used as effector cells and co-cultured with target mouse CT26 cells (with GPC3 expression) at an E:T ratio of 10:1. It can be seen that CAR-pMAC-SIRPα-KO exhibits higher cytotoxic activity after 6 hr of co-culture with cancer cells, compared to pMAC, CAR-pMAC, and pMAC-SIRPα-KO.

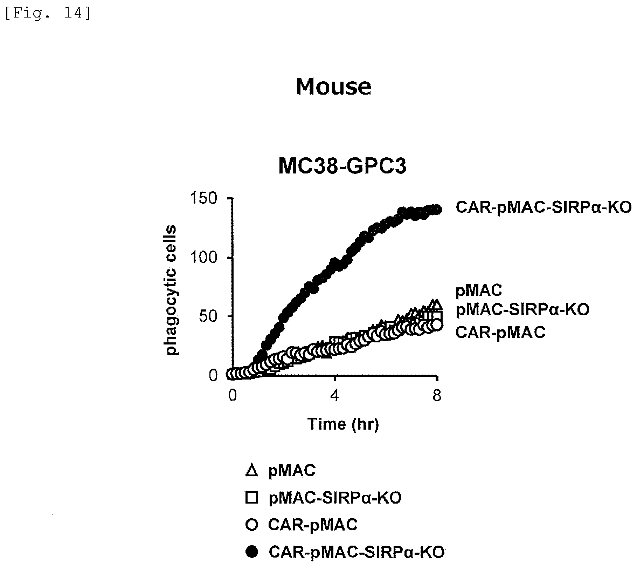

[Fig. 14] A line graph showing the number of phagocytes counted by time-lapse observation after co-culturing mouse pMAC, pMAC-SIRPα-KO, CAR-pMAC, or CAR-pMAC-SIRPα-KO with mouse cancer cell MC38 (GPC3 expression). The horizontal axis indicates the time of co-culture, and the vertical axis indicates the number of phagocytes.

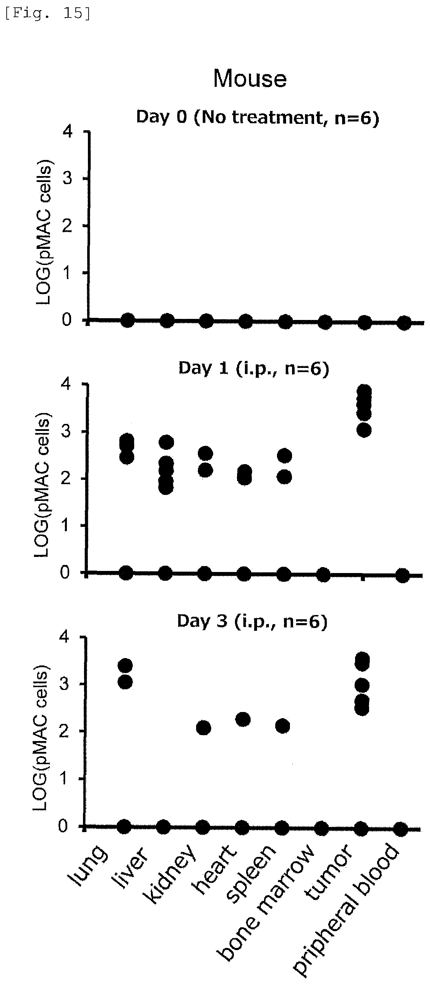

[Fig. 15] The results of pharmacokinetics of CAR-pMAC-SIRPα-KO as measured by quantitative PCR before administration Day 0 (top), after 1 day (middle), and after 3 days (bottom) from the administration of CAR-pMAC-SIRPα-KO to C57BL/6 cancer-bearing mice with peritoneal dissemination formed by transplantation of MC38 (with GPC3 expression). The horizontal axis indicates each organ evaluated, and the vertical axis indicates the common logarithm of the number of CAR-pMAC cells detected in 100 µg of genomic DNA in each organ.

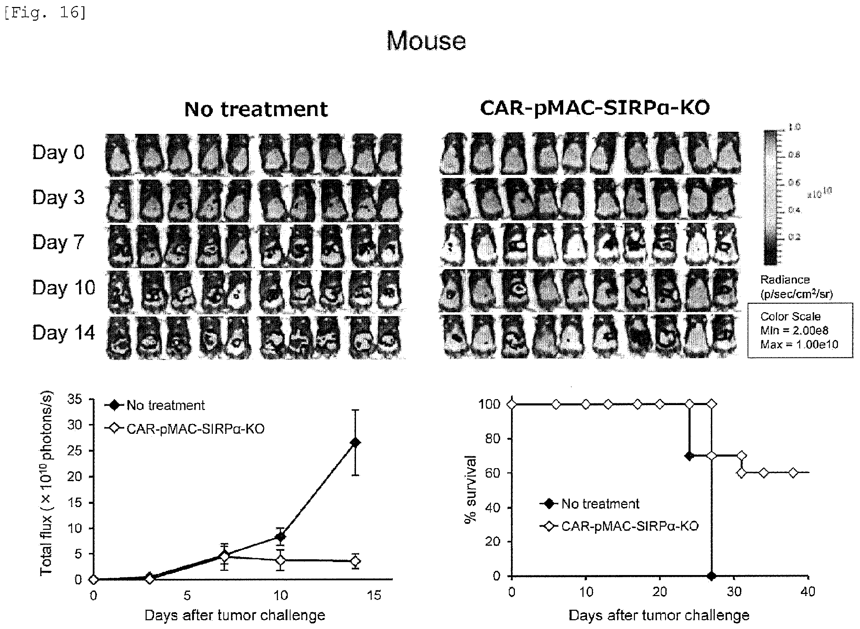

[Fig. 16] Images of in vivo imaging of all individuals in which surviving cancer cells were measured by biochemiluminescence (top), a line graph showing the time-course changes in the total flux of luciferase luminescence (bottom left), and the survival curve of individual cancer-bearing mice (bottom right), in colorectal cancer peritoneal dissemination model mice that were either untreated or administered with CAR-pMAC-SIRPα-KO irradiated with 10 Gy on the following day, day 3, day 7, day 10, day 14, and day 17, after intraperitoneal administration of mouse colorectal cancer cell line MC38, which expresses the luciferase gene and the cancer antigen GPC3, to wild-type C57BL/6 mice. In vivo imaging was performed at the time (Day 0), day 3 (Day 3), day 7 (Day 7), day 10 (Day 10), and day 14 (Day 14) of intraperitoneal administration of MC38. The right side of the photographed images above is a scale showing the luciferase luminescence intensity in chromatic colors, and the minimum luminescence intensity is 2.00×e⁸, and the maximum luminescence intensity is 1.00×e¹⁰ (unit: p/sec/cm²/sr). The vertical axis of the lower left line graph shows the total flux of luciferase luminescence (unit: 10¹⁰photons/s), and the horizontal axis indicates the number of days after cancer cell transplantation. The vertical axis of the graph on the bottom right shows the mouse survival rate, and the horizontal axis shows the number of days after cancer cell transplantation. Cancer cell growth was observed in untreated mice, but in mice administered with CAR-pMAC-SIRPα-KO, the growth of cancer cells was suppressed by administration even after radiation exposure, and it was found that the progression of colorectal cancer peritoneal dissemination was suppressed to prolong the survival of individual mice.

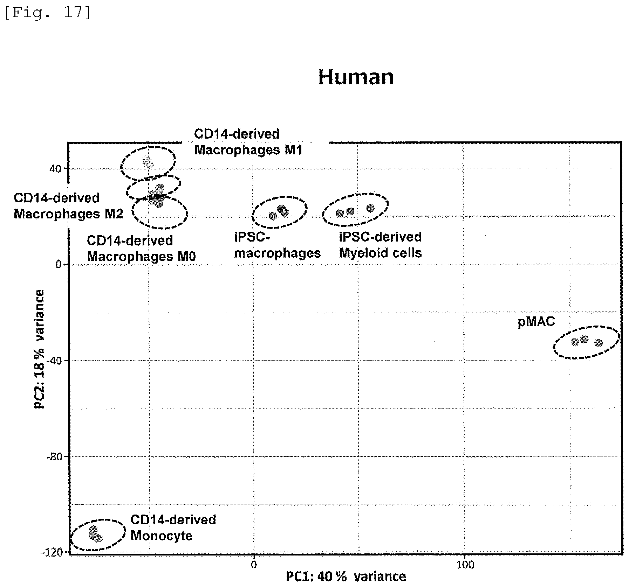

[Fig. 17] A diagram showing the gene expression profile of each cell obtained by RNA sequencing analysis, followed by principal component analysis, of human pMAC, blood collection-derived CD14-positive cell-derived monocyte (CD14-derived monocyte), blood collection-derived CD14 positive cell-derived macrophage M0 (CD14-derived Macrophages M0), blood collection-derived CD14 positive cell-derived M1 type macrophage (CD14-derived Macrophages M1), blood collection-derived CD14 positive cell-derived M2 type macrophage (CD14-derived Macrophages M2), human iPS cell-derived myeloid cell (iPSC-derived Myeloid cells) and human iPS cell-derived macrophage (iPSC-derived macrophages), in which the first principal component (PC1: contribution rate 40%) is plotted on the horizontal axis, and the second principal component (PC2: contribution rate 18%) is plotted on the horizontal axis. A closer distance between the plots of each cell shows that the gene expression profiles are more similar; conversely, a farther distance between the plots of each cell shows that the gene expression profiles are different. It can be seen that the gene expression profile of pMAC is vastly different from that of any known blood collection-derived or iPS cell-derived macrophages.

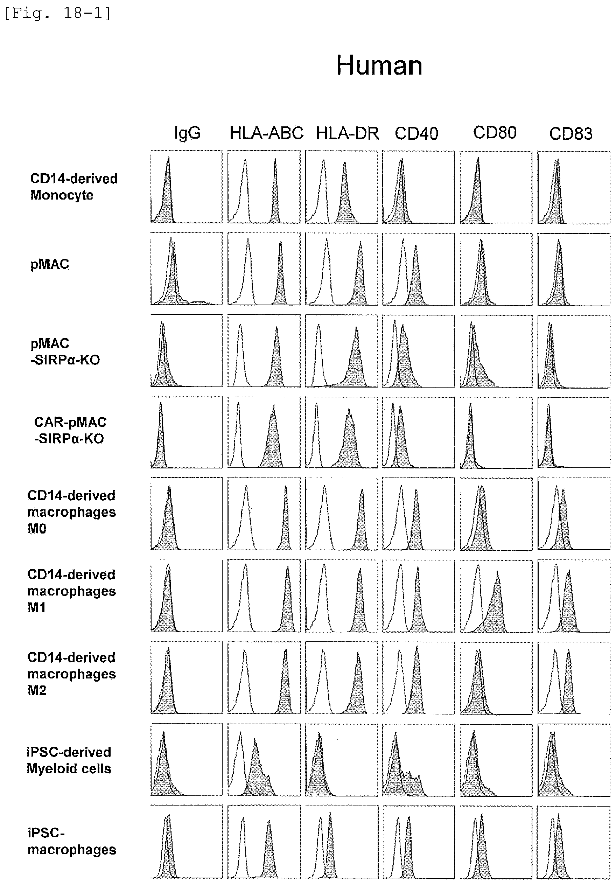

[Fig. 18-1] Histograms showing the results of surface antigen analysis of HLA-ABC, HLA-DR, CD40, CD80, and CD83 using a flow cytometer of human blood collection-derived CD14 positive cell-derived monocyte (CD14-derived Monocyte), pMAC, pMAC-SIRPα-KO, CAR-pMAC-SIRPα-KO, blood collection-derived CD14 positive cell-derived macrophage (CD14-derived macrophages M0), blood collection-derived CD14 positive cell-derived M1 type macrophage (CD14-derived macrophages M1), blood collection-derived CD14 positive cell-derived M2 type macrophage (CD14-derived macrophages M2), iPS cell-derived myeloid cell (iPSC-derived Myeloid cells), iPS cell-derived macrophage (iPSC-derived macrophages). The vertical axis indicates the proportion (%) of each type of cell present, and the horizontal axis indicates the fluorescence intensity of each measured marker.

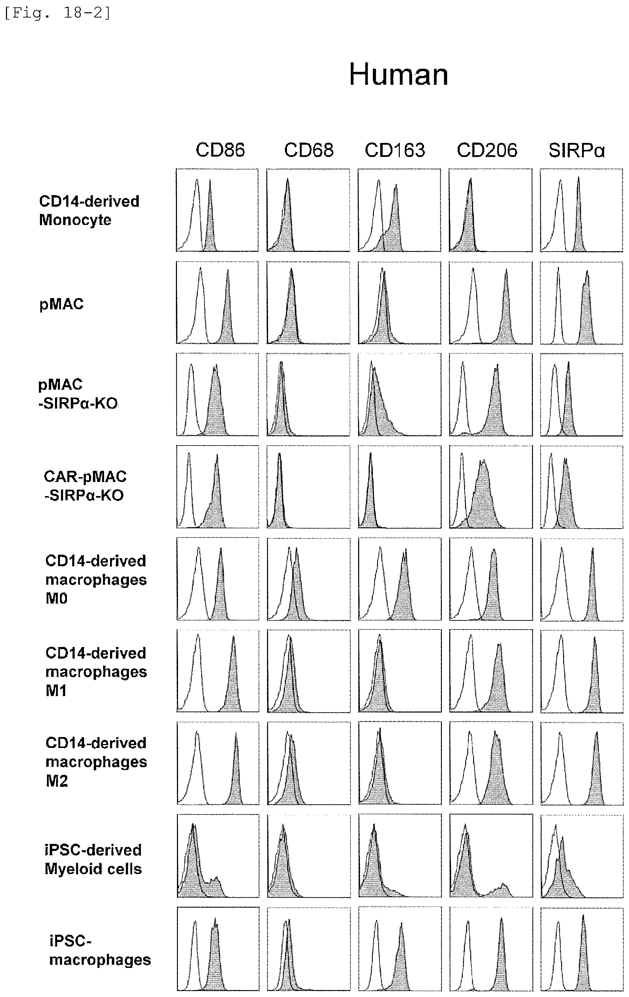

[Fig. 18-2] Histograms showing the results of surface antigen analysis of CD86, CD68, CD163, CD206 and SIRPαusing a flow cytometer of human blood collection-derived CD14 positive cell-derived monocyte (CD14-derived Monocyte), pMAC, pMAC-SIRPα-KO, CAR-pMAC-SIRPα-KO, blood collection-derived CD14 positive cell-derived macrophage (CD14-derived macrophages M0), blood collection-derived CD14 positive cell-derived M1 type macrophage (CD14-derived macrophages M1), blood collection-derived CD14 positive cell-derived M2 type macrophage (CD14-derived macrophages M2), iPS cell-derived myeloid cell (iPSC-derived Myeloid cells), iPS cell-derived macrophage (iPSC-derived macrophages). The vertical axis indicates the proportion (%) of each type of cell present, and the horizontal axis indicates the fluorescence intensity of each measured marker.

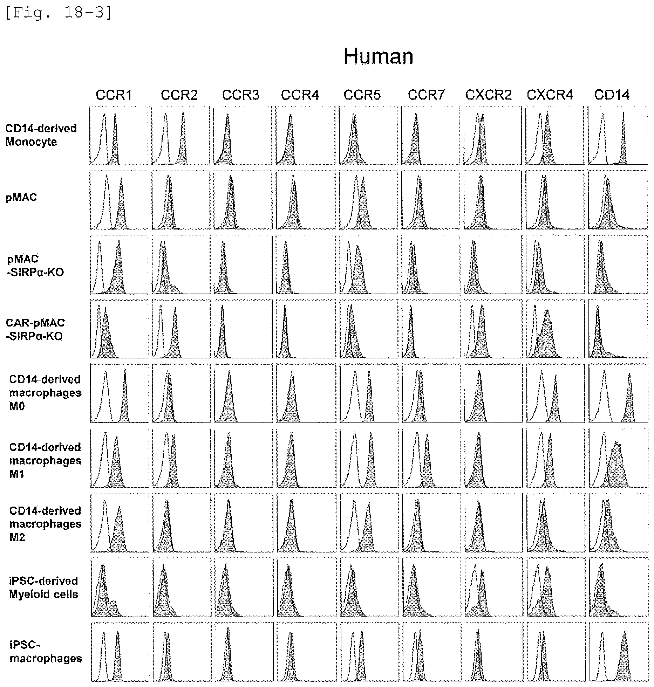

[Fig. 18-3] Histograms showing the results of surface antigen analysis of CCR1, CCR2, CCR3, CCR4, CCR5, CCR7, CXCR2, CXCR4 and CD14 using a flow cytometer of human blood collection-derived CD14 positive cell-derived monocyte (CD14-derived Monocyte), pMAC, pMAC-SIRPα-KO, CAR-pMAC-SIRPα-KO, blood collection-derived CD14 positive cell-derived macrophage (CD14-derived macrophages M0), blood-derived CD14 positive cell-derived M1 type macrophage (CD14-derived macrophages M1), blood collection-derived CD14 positive cell-derived M2 type macrophage (CD14-derived macrophages M2), iPS cell-derived myeloid cell (iPSC-derived Myeloid cells), iPS cell-derived macrophage (iPSC-derived macrophages). The vertical axis indicates the proportion (%) of each type of cell present, and the horizontal axis indicates the fluorescence intensity of each measured marker.

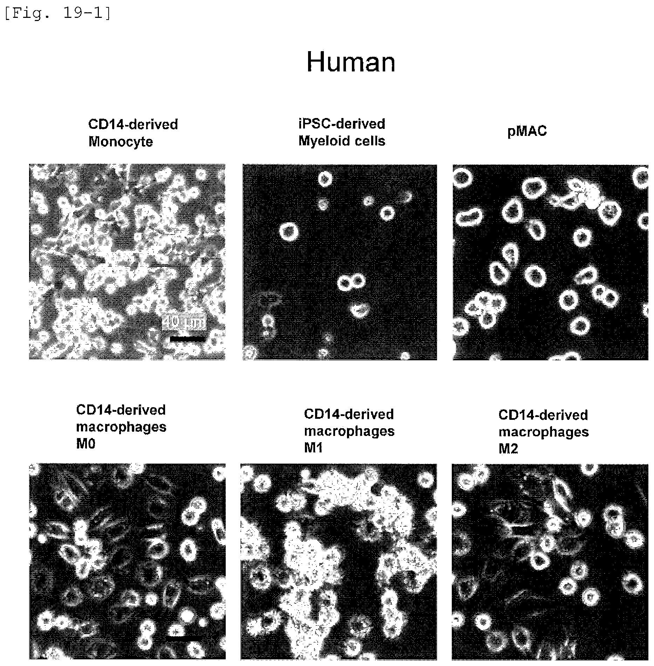

[Fig. 19-1] Phase contrast microscope images of human blood collection-derived CD14 positive cell-derived monocyte (CD14-derived Monocyte), iPS cell-derived myeloid cell (iPSC-derived Myeloid cells), pMAC, blood collection-derived CD14 positive cell-derived macrophage (CD14-derived macrophages M0), blood collection-derived CD14 positive cell-derived M1 type macrophage (CD14-derived macrophages M1), and blood collection-derived CD14 positive cell-derived M2 type macrophage (CD14-derived macrophages M2). The scale bar indicates 40 µm.

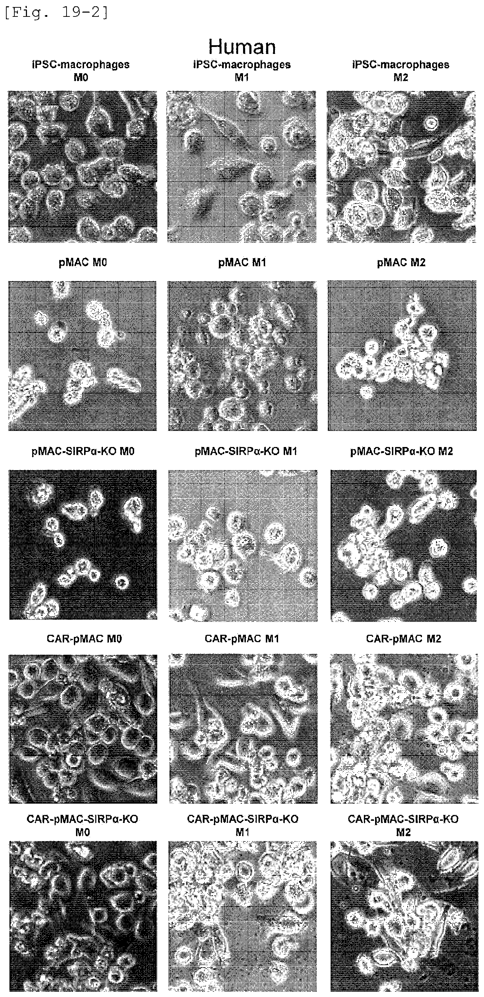

[Fig. 19-2] Phase contrast microscope images of human iPS cell-derived macrophage (iPSC-macrophages), pMAC, pMAC-SIRPα-KO, CAR-pMAC, and CAR-pMAC-SIRPα-KO under conditions of no stimulation (M0), interferon γ and LPS stimulation (M1), and IL-4 stimulation (M2).

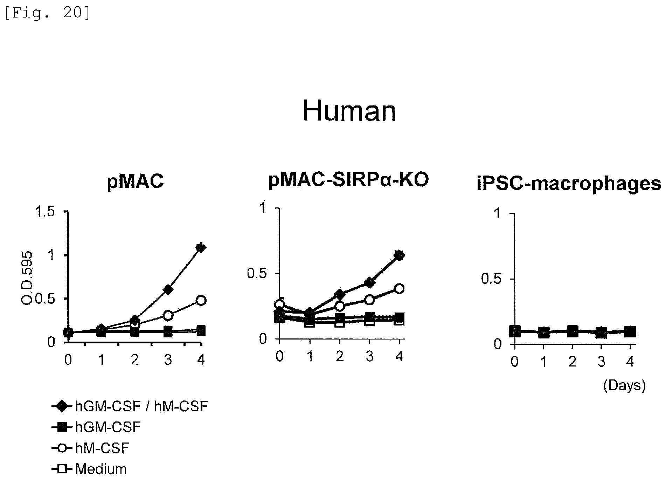

[Fig. 20] Line graphs showing the results of an MTT assay investigating the effect of a medium containing GM-CSF and/or M-CSF (αMEM containing 20% FBS) on the proliferation rate of human pMAC, pMAC-SIRPα-KO, iPS cell-derived macrophage (iPSC-macrophages). The results of human pMAC (left), pMAC-SIRPα-KO (center), iPSC-macrophages (right) are shown. The vertical axis indicates the concentration of formazan metabolized from MTT reagent (evaluated as absorbance at 595 nm) and the horizontal axis indicates the number of days of culture.

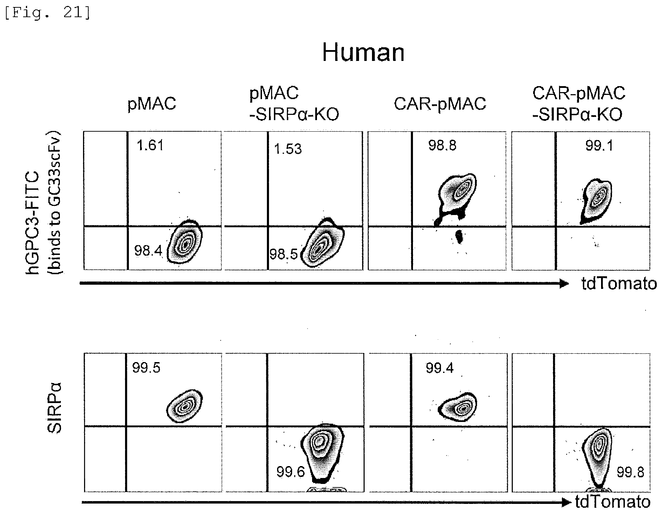

[Fig. 21] Cytograms showing the results of expression analysis of hGPC3-FITC, SIRPα, and tdTomato by flow cytometer in human pMAC, pMAC-SIRPα-KO, CAR-pMAC, and CAR-pMAC-SIRPα-KO. The vertical axis indicates the fluorescence intensity of hGPC3-FITC, and the horizontal axis indicates the fluorescence intensity of tdTomato (top). The vertical axis indicates the fluorescence intensity of SIRPα, and the horizontal axis indicates the fluorescence intensity of tdTomato (bottom). pMAC expressing GPC3-CAR was obtained as a single cell population, indicating that it is highly pure and contains almost no untargeted cells.

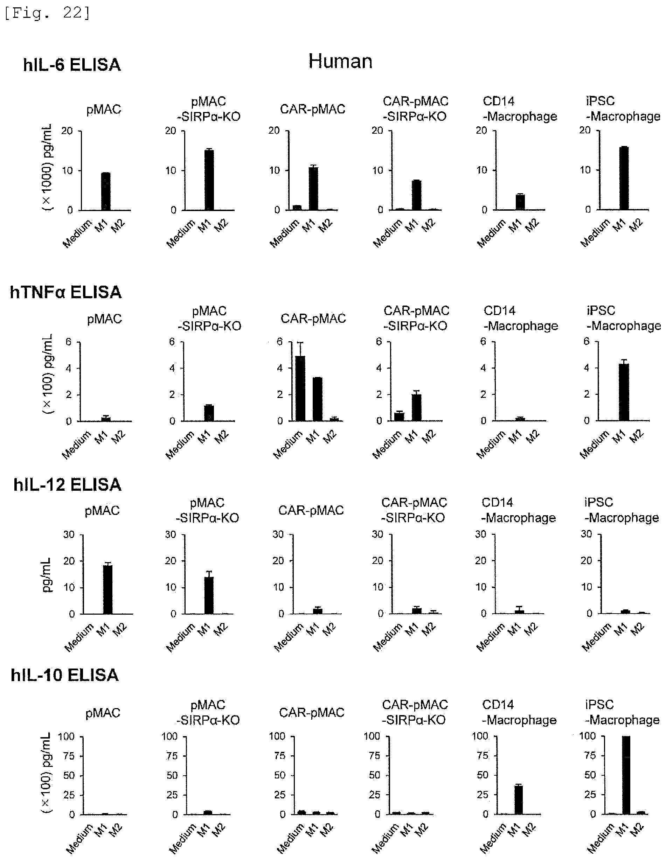

[Fig. 22] ELISA measurement results of production of cytokines IL-6, TNFα, IL-12, and IL-10, when human pMAC, pMAC-SIRPα-KO, CAR-pMAC, CAR-pMAC-SIRPα-KO, blood collection-derived CD14 positive cell-derived macrophage (CD14-Macrophage), and iPS cell-derived macrophage (iPSC-Macrophage) were given a medium without stimulating components (Medium), M1 stimulation with interferon-γ and LPS, and M2 stimulation with IL-4. The vertical axis indicates the concentration (pg/mL) of the measured cytokine.

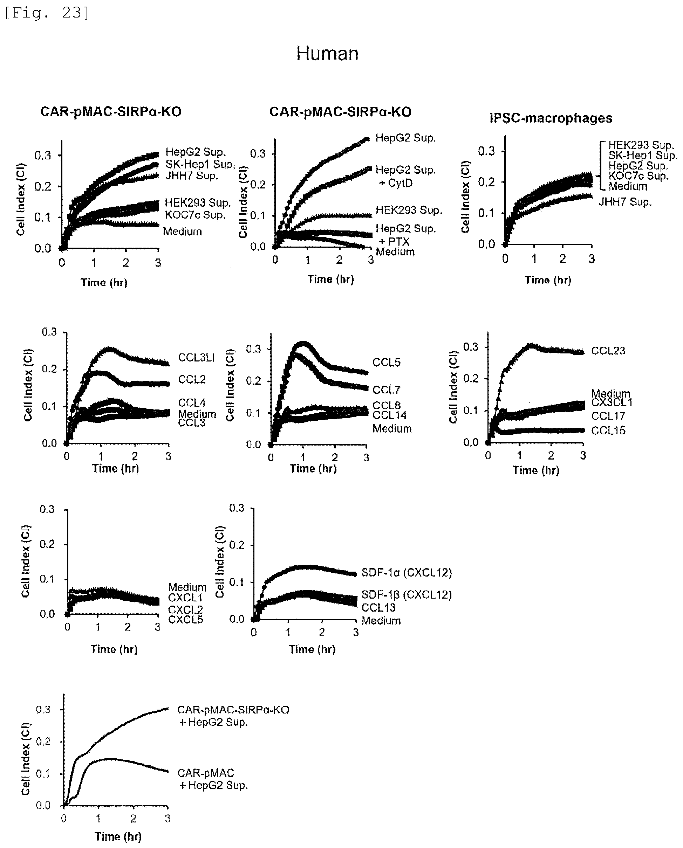

[Fig. 23] Real-time measurement results of cell migration ability of human CAR-pMAC-SIRPα-KO using the Boyden chamber-type xCELLigence DP system. Cell migration ability toward culture supernatants derived from human cancer cell lines HepG2, SK-Hep1, JHH7, KOC7c, human fetal-derived kidney cell line HEK293, and toward fresh medium (Medium) (upper left); cell migration ability of human CAR-pMAC-SIRPα-KO that was untreated, treated with cytochalasin D (+CytD), or treated with Bordetella pertussis toxin (+PTX) toward culture supernatant from human cancer cell line HepG2, culture supernatant from human embryonic kidney cell line HEK293, or toward fresh medium (Medium) (upper center). Cell migration ability of human iPSC-macrophages toward culture supernatants from cancer cell lines HepG2, SK-Hep1, JHH7, KOC7c, and human fetal-derived kidney cell line HEK293, and toward fresh medium (Medium) (top right). Cell migration ability of human CAR-pMAC-SIRPα-KO toward recombinant CCL2, CCL3, CCL3LI, CCL4, and toward fresh medium (Medium) (second row from the top, left). Cell migration ability toward recombinant CCL5, CCL7, CCL8, CCL14, and toward fresh medium (Medium) (second row from the top, center), and cell migration ability toward recombinant CCL15, CCL17, CCL23, CX3CL1, and fresh medium (Medium) (second row from the top, right). Cell migration ability toward recombinant CXCL1, CXCL2, CXCL5, and fresh medium (Medium) (third row from the top, left). Cell migration ability toward recombinant CCL13, SDF-1α(CXCL12), SDF-1β(CXCL12) and fresh medium (Medium) (third row from the top, right). Cell migration ability of CAR-pMAC and CAR-pMAC-SIRPα-KO toward culture supernatants from cancer cell line HepG2 (bottom row). The horizontal axis indicates the culture time, and the vertical axis indicates Cell Index (CI), which indicates the degree of cell migration ability relative to the start of measurement (0 hr).

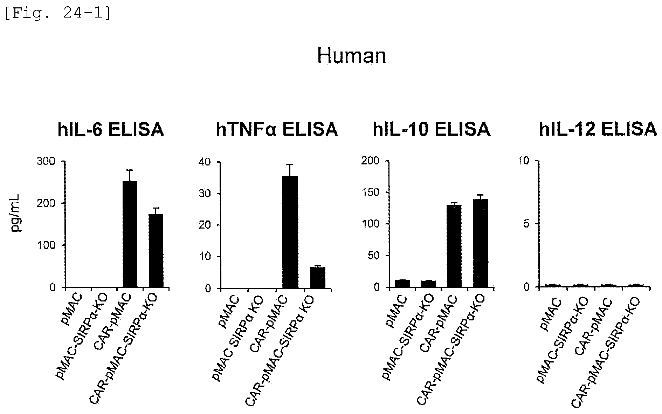

[Fig. 24-1] ELISA measurement results of production of cytokine IL-6 (left), TNFα (second from the left), IL-10 (third from the left), and IL-12 (right) in the culture supernatant when human pMAC, pMAC-SIRPα-KO, CAR-pMAC, or CAR-pMAC-SIRPα-KO was cultured for 48 hr.

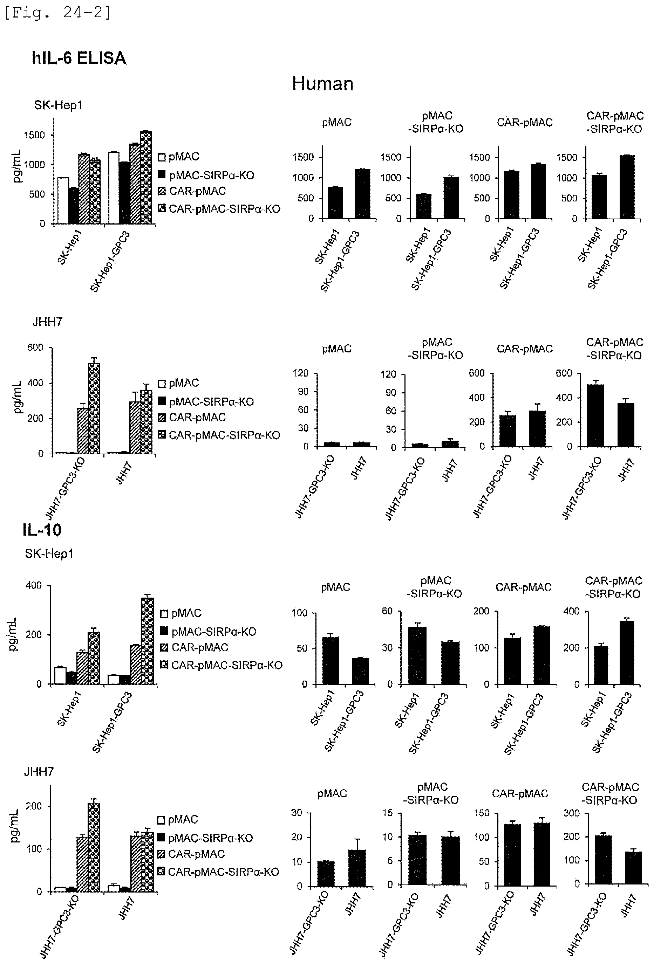

[Fig. 24-2] ELISA measurement results of cytokine IL-6 production in culture supernatants when human pMAC, pMAC-SIRPα-KO, CAR-pMAC or CAR-pMAC-SIRPα-KO was co-cultured with human cancer cell lines (with or without GPC3 expression) for 48 hr. The results of co-culture of the aforementioned four types of pMAC with hepatic cancer cell line SK-Hep1 (without GPC3 expression) or SK-Hep1-GPC3 (with GPC3 expression) (top row), and hepatic cancer cell line JHH7-GPC3-KO (without GPC3 expression) or JHH7 (with GPC3 expression) (second row from the top). ELISA measurement results of cytokine IL-10 production in culture supernatants when human pMAC, pMAC-SIRPα-KO, CAR-pMAC or CAR-pMAC-SIRPα-KO was co-cultured with human cancer cell lines (with or without GPC3 expression) for 48 hr. The results of co-culture of the aforementioned four types of pMAC with hepatic cancer cell line SK-Hep1 (without GPC3 expression) or SK-Hep1-GPC3 (with GPC3 expression) (third row from the top), and hepatic cancer cell line JHH7-GPC3-KO (without GPC3 expression) or JHH7 (with GPC3 expression) (bottom row).

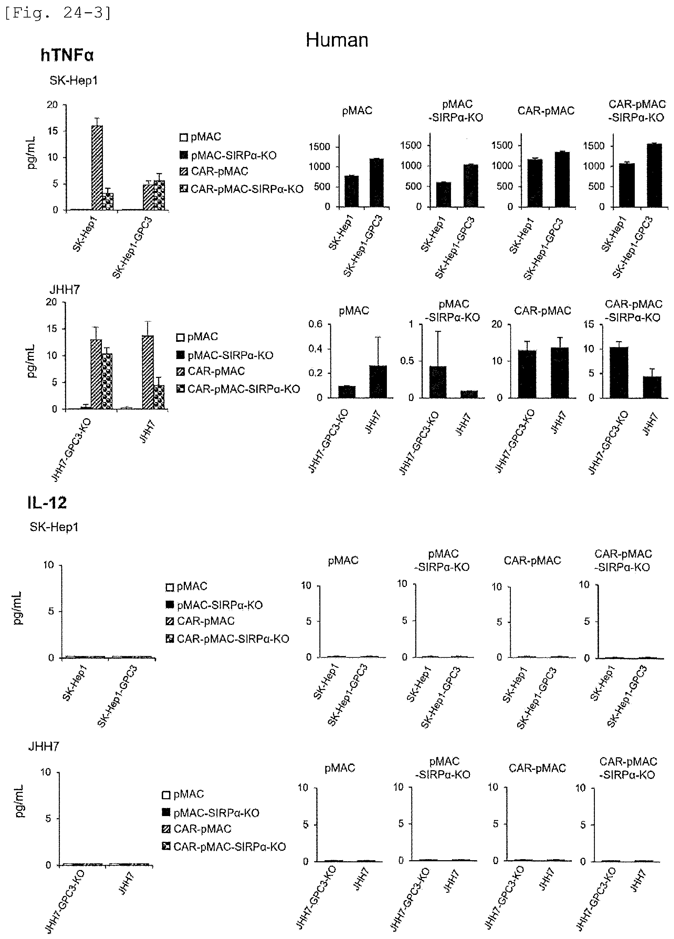

[Fig. 24-3] ELISA measurement results of cytokine TNFα production in culture supernatants when human pMAC, pMAC-SIRPα-KO, CAR-pMAC or CAR-pMAC-SIRPα-KO was co-cultured with human cancer cell lines (with or without GPC3 expression) for 48 hr. The results of co-culture of the aforementioned four types of pMAC with cancer cell line SK-Hep1 (without GPC3 expression) or SK-Hep1-GPC3 (with GPC3 expression) (top row), and cancer cell line JHH7-GPC3-KO (without GPC3 expression) or JHH7 (with GPC3 expression) (second row from the top). ELISA measurement results of cytokine IL-12 production in culture supernatants when human pMAC, pMAC-SIRPα-KO, CAR-pMAC or CAR-pMAC-SIRPα-KO was co-cultured with human cancer cell lines (with or without GPC3 expression) for 48 hr. The results of co-culture of the aforementioned four types of pMAC with cancer cell line SK-Hep1 (without GPC3 expression) or SK-Hep1-GPC3 (with GPC3 expression) (third row from the top), and cancer cell line JHH7-GPC3-KO (without GPC3 expression) or JHH7 (with GPC3 expression) (bottom row).

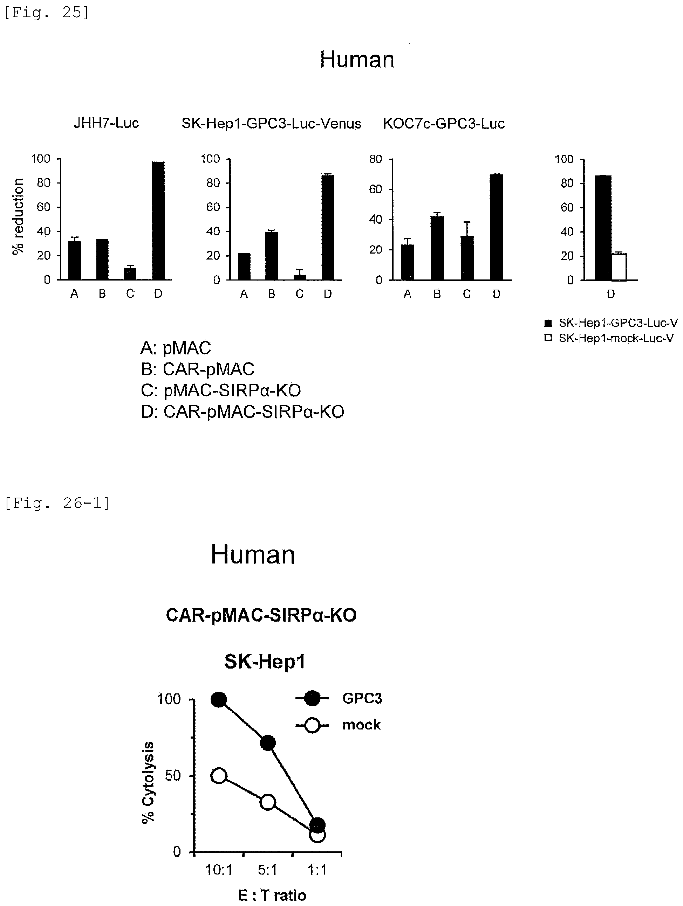

[Fig. 25] Bar graphs showing the results of quantifying luciferase activity in cancer cells after co-culturing human pMAC (A), CAR-pMAC (B), pMAC-SIRPα-KO (C) and CAR-pMAC-SIRPα-KO (D) with human hepatic cancer cell line JHH7-Luc (with human GPC3 expression; left), human hepatic cancer cell line SK-Hep1-GPC3-Luc-Venus (with human GPC3; second from left), and human cancer cell line KOC7c-GPC3-Luc (with human GPC3 expression; third from left), each introduced with the luciferase (Luc) gene, for 48 hr. A bar graph showing the results of quantifying luciferase activity in cancer cells after co-culturing CAR-pMAC-SIRPα-KO(D) and human hepatic cancer cell line SK-Hep1-GPC3-Luc-Venus (with or without human GPC3; right) for 48 hr. The vertical axis indicates the proportion (%) of decrease in cancer cells. White bars represent cancer cells that do not express hGPC3, and black bars represent cancer cells that express hGPC3. It can be seen that CAR-introduced pMAC(D) eliminates cancer cells that express the target cancer antigen human GPC (hGPC).
[Fig. 26-1] A graph showing cytotoxic activity of human CAR-pMAC-SIRPα-KO to cancer cells, when human CAR-pMAC-SIRPα-KO was used as an effector cell and co-cultured with two types of human cancer cells as the target cells. Human hepatic cancer cell line SK-Hep1 (with or without human GPC3 expression) was cultured for 24 hr, CAR-pMAC-SIRPα-KO was added in a 10-fold, 5-fold, or 1-fold amount of the seeded cancer cells and co-cultured, and the cytotoxic activity (% Cytolysis) after 6 hr was plotted. The vertical axis indicates the cytotoxic activity (% Cytolysis) of cancer cells, and the horizontal axis indicates the mixing ratio (E:T ratio) of effector cells (CAR-pMAC-SIRPα-KO) and target cells (cancer cells).

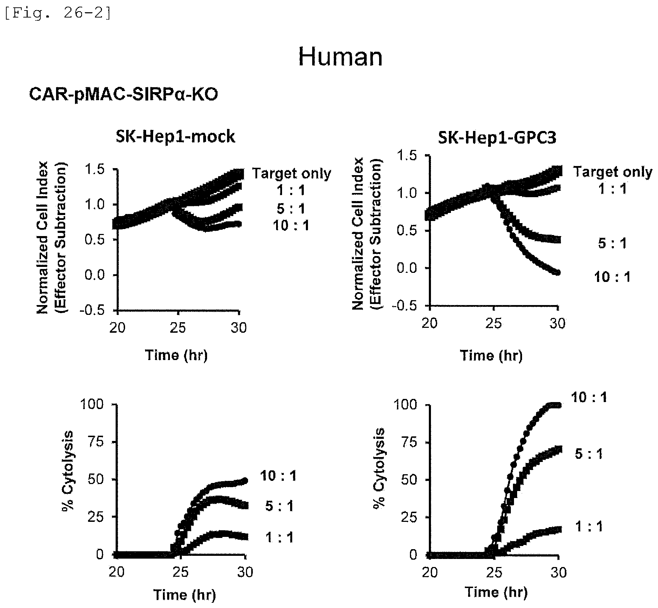

[Fig. 26-2] The results of measurement using the xCELLigence RTCA DP system of the cell survival signal Normalized Cell Index (upper row) of the hepatic cancer cell SK-Hep1 and the cytotoxic activity (% Cytolysis) of human hepatic cancer cell SK-Hep1 (bottom row) by human CAR-pMAC-SIRPα-KO, when human CAR-pMAC-SIRPα-KO was used as an effector cell and co-cultured with target human hepatic cancer cells (with or without human GPC3 expression) at an E:T ratio of 10:1, 5:1, and 1:1.

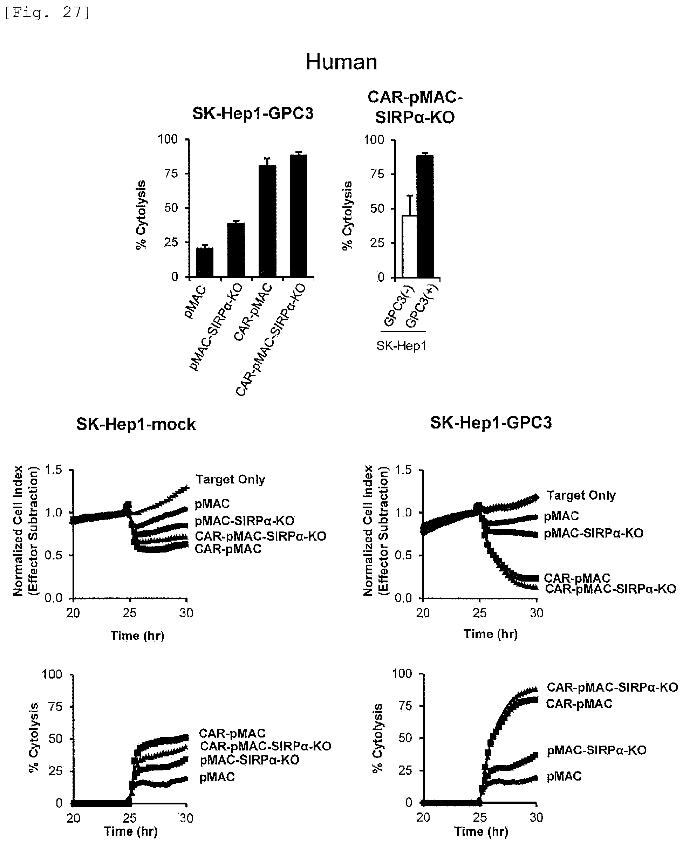

[Fig. 27] A bar graph (top left) in which the cytotoxic activity (% Cytolysis) of SK-Hep1 is plotted on the vertical axis when human pMAC, pMAC-SIRPα-KO, CAR-pMAC, and CAR-pMAC-SIRPα-KO were used as effector cells and co-cultured with target human hepatic cancer cells SK-Hep1 (with expression of GPC3) at an E:T ratio of 10:1. A bar graph (top right) in which the cytotoxic activity (% Cytolysis) of SK-Hep1 is plotted on the vertical axis when human CAR-pMAC-SIRPα-KO was used as an effector cell and co-cultured with target human hepatic cancer cell SK-Hep1 (without expression of GPC3) at an E:T ratio of 10:1. A line graph (middle left) plotting the time course changes of the Normalized Cell Index, which indicates cell survival of SK-Hep1 (without GPC3 expression), on the vertical axis and the co-culture time on the horizontal axis, and a line graph (bottom left) plotting the cytotoxic activity (% Cytolysis) of human hepatic cancer cell SK-Hep1 (without GPC3 expression) on the vertical axis, when human pMAC, pMAC-SIRPα-KO, CAR-pMAC and CAR-pMAC-SIRPα-KO were used as effector cells and co-cultured with target human SK-Hep1 (without GPC3 expression) at an E:T ratio of 10:1. A line graph (middle right) plotting the time course changes of the Normalized Cell Index, which indicates cell survival of SK-Hep1 (with GPC3 expression), on the vertical axis and the co-culture time on the horizontal axis, when human pMAC, pMAC-SIRPα-KO, CAR-pMAC and CAR-pMAC-SIRPα-KO were used as effector cells and co-cultured with target human cancer cell SK-Hep1 (with GPC3 expression) at an E:T ratio of 10:1. A line graph (bottom right) plotting the cytotoxic activity (% Cytolysis) of human hepatic cancer cell SK-Hep1 (with GPC3 expression) on the vertical axis. It can be seen that CAR-pMAC-SIRPα-KO and CAR-pMAC exhibit higher cytotoxic activity after 6 hr of co-culture with cancer cells, compared to pMAC and pMAC-SIRPα-KO.

### [Description of Embodiments]

The present invention is described below. The terms used in the present specification have the meanings generally used in the art unless otherwise specified.

### <Proliferating macrophage-like cell>

The present invention provides macrophage cells characterized by having phagocytic ability and proliferating in a cytokine-dependent manner. The macrophage cells of the present invention have characteristics different from existing macrophages, and are therefore referred to as proliferating macrophage-like cell (pMAC; proliferating Macrophage-like cell) (hereinafter also referred to as pMAC of the present invention). Alternatively, the pMAC of the present invention can also be referred to as cytokine-dependent proliferating macrophage. In one embodiment of the pMAC of the present invention, a gene that inhibits a macrophage-like function has been deleted or expression of the gene has been suppressed.

In the present specification, "proliferating in a cytokine-dependent manner" means that cell proliferation is promoted by cytokine stimulation, culture is possible for three months or more, and proliferating property is significantly superior to cells without cytokine stimulation. Specific examples of cytokine stimulation include a step of culturing cells in a cytokine-containing medium. The cytokine may be added exogenously to the medium, or may be produced intracellularly and secreted into the medium. As the cytokine, one or preferably two selected from the group consisting of granulocyte-macrophage colony-stimulating factor (GM-CSF) and macrophage colony-stimulating factor (M-CSF) are used in combination. The combination of GM-CSF and M-CSF is preferred.

GM-CSF is also known as CSF2, and is produced by cells such as T cells, macrophages, endothelial cells, and fibroblasts in response to immune stimulation, and functions as a hematopoietic growth factor and immunoregulatory factor. GM-CSF induces bone marrow progenitor cells and promotes the proliferation of granulocytes and macrophages.

M-CSF is one type of cytokine that induces the proliferation and differentiation of bone marrow stem cells, particularly the monocyte-macrophage system.

Each cytokine is commercially available. Alternatively, sequence information of various biological species of proteins, genomic DNA or cDNA of each cytokine can be obtained from information sources well known to those skilled in the art, such as the Gene database of the National Center for Biotechnology Information (NCBI). Based on the information, the gene of the desired cytokine can be expressed in cells, the protein can be produced in the cells, and in some cases, the protein can be secreted outside the cells to obtain the desired cytokine.

The pMAC of the present invention is characterized by being negative for CD83 and CD14, in addition to the characteristic cytokine-dependent proliferation.

CD83 is known as a maturation marker of the dendritic cell system that is expressed on the cell surface. It can be detected by immunostaining with an anti-CD83 antibody and analyzing with a flow cytometer.

CD14 is known as a marker of monocytes and macrophages that is expressed on the cell surface. It can be detected by immunostaining with anti-CD14 antibody and analyzing with a flow cytometer.

As used herein, "negative for CD83 and CD14" means that CD83 and CD14 are not expressed or are not substantially expressed. Specifically, it means that the cell surface markers CD83 antigen and CD14 antigen are not detected on the cell surface, are below the detection limit, or are detected only slightly. Specifically, it means that the expression levels of CD83 and CD14 in existing macrophages are 1/5 or less, preferably 1/10 or less, more preferably 1/20 or less, even more preferably 1/30 or less, and most preferably below the detection limit. The expression levels of CD83 and CD14 can be confirmed by known methods such as flow cytometry, quantitative PCR, microarray, northern hybridization, immunohistochemical staining using antibodies against each antigen, and western blotting.

Furthermore, as demonstrated in detail in the following Examples and Drawings, the pMAC of the present invention is characterized by a markedly altered gene expression profile compared to cells belonging to known macrophage cell lineages. Therefore, the pMAC of the present invention can also be defined using statistics based on the gene expression profile. For example, in the case of human pMAC, one example thereof can be defined as follows, as demonstrated in detail in the Examples and Drawings (particularly Fig. 17).

A proliferating macrophage-like cell that satisfies the following conditions when a main component analysis based on the gene expression profile obtained by performing a whole-transcriptome analysis using, as a comparison target, a cell population along the macrophage cell lineage consisting of an iPS cell-derived myeloid cell, an iPS cell-derived macrophage, a biologically derived monocyte cell, a biologically derived macrophage M1, a biologically derived macrophage M2, and a biologically derived macrophage M0 is performed and the top two components with high contribution rates are selected as the analysis targets:
condition 1: no overlapping with plots of known macrophage cell lineages
condition 2: PC1 is 100 or more, and PC2 is plotted in the range of -80 to 0.

In one embodiment, the pMAC of the present invention has a PC1 of 100 or more, preferably 110 or more, 120 or more, 130 or more, or 140 or more, more preferably 150 or more, but is not limited thereto. Furthermore, the pMAC of the present invention may have a PC1 of 250 or less, preferably 220 or less, 210 or less, 200 or less, or 190 or less, more preferably 180 or less, but is not limited thereto. In one embodiment, the pMAC of the present invention has a PC1 of 100 to 250 or less, preferably 110 to 220, 120 to 210, 130 to 200, or 140 to 190, more preferably 150 to 180, but is not limited thereto.

The pMAC of the present invention is derived from a pluripotent stem cell, more preferably derived from an induced pluripotent stem cell or an embryonic stem cells. The pluripotent stem cell refers to a stem cell that can be cultured in vitro while maintaining an undifferentiated state and has the ability (pluripotency) to differentiate into all cells (tissues derived from three germ layers (ectoderm, mesoderm, endoderm)) constituting a living body except the placenta. Embryonic stem cells (ES cells) isolated from embryos after fertilization and before the formation of three germ layers, embryos after the organogenesis stage including primordial germ cells, and fetuses or fetuses are also induced in the "pluripotent stem cells".

In the present invention, the "induced pluripotent stem cell" refers to cells in which pluripotency is induced by directly reprogramming somatic cells that have once lost their pluripotency by expressing several types of genes such as Oct3/4, Sox2, Klf4, Myc, and the like (hereinafter to be also referred to as iPSC or iPS cells). The "induced pluripotent stem cell" is also included in the "pluripotent stem cell". Pluripotent stem cells can be produced by known methods. As for the production methods, for example, the methods for human induced pluripotent stem cells are described in, for example, Cell, 2007, 131(5) pp. 861-872 and Kitayama, S. et. al. (Stem Cell Reports. 6: 213-227, (2016)), and the methods for mouse induced pluripotent stem cells are described in Cell, 2006, 126(4) pp. 663-676. "Pluripotent stem cells" can also be obtained from embryo-engineered animals including cloned embryos, transgenic animals, and genome-edited mutant animals. It is known that pluripotent stem cells can be differentiated into cells of various cell types, including myeloid cells (MC cell).

Human or mouse iPSCs can be maintained and expanded using the regenerative medicine medium StemFit (Ajinomoto). Laminin 511 may be added at this time. In addition, a Rho kinase (ROCK) inhibitor such as Y-27632 may be added to the medium at about 10 µM in order to reduce or remove iPSC damage due to dissociation of cells for passage culture of the cells.

Pluripotent stem cells can be obtained from designated institutions, and commercially available products can also be purchased. For example, human embryonic stem cells KhES-1, KhES-2, and KhES-3 are available from The Institute for Frontier Life and Medical Sciences, Kyoto University. EB5 cells, which are mouse embryonic stem cells, are available from RIKEN, and the D3 strain is available from ATCC.

Pluripotent stem cells can be maintained and cultured by known methods. For example, human pluripotent stem cells can be maintained by culturing in a medium supplemented with Knockout (trade mark) Serum Replacement (Invitrogen). Mouse pluripotent stem cells can be maintained by adding fetal bovine serum (FBS) and Leukemia Inhibitory Factor (LIF) and culturing under feeder free.

### <Production method of proliferating macrophage-like cell>

In the present invention, pMAC can be produced by a method including a step of deleting a gene that inhibits a macrophage-like function or suppressing expression of the gene, and a step of expressing a gene that confers proliferating property. Each step is described in the following.

### 1. Step of deleting the gene that inhibits a macrophage-like function or suppressing expression of the gene

The "gene that inhibits a macrophage-like function" means a gene that inhibits a function that can be provided by macrophages. The "function that can be provided by macrophages" includes phagocytosis (engulfment) that contributes to the uptake and digestion of foreign matter, antigen presentation that promotes the activation of other immune cells such as T cells, and the induction of secondary immune responses by activation. Macrophage activation is broadly divided into classical (M1) activation and selective (M2) activation. M1-activated macrophages (M1 macrophages) are obtained by inducing the differentiation of monocytes with inflammatory cytokines such as tumor necrosis factor (TNF)-α and interferon (IFN)-γ, and produce proinflammatory cytokines that act in defense against pathogenic infections and reduce the infectivity of microorganisms. M2-activated macrophages (M2 macrophages) are obtained by inducing the differentiation with Th2-type cytokines such as IL-4 and IL-13, and produce growth factors and anti-inflammatory cytokines that suppress immune responses in the host, promote wound healing and tissue remodeling, and improve metabolism and endocrine signaling in tissues. The "gene that inhibits a macrophage-like function" is a gene whose expression can inhibit such functions that can be provided by macrophages. Examples of such gene include, but are not limited to, the signal regulatory protein α (SIRPα) gene.

The SIRPα gene is one of the membrane proteins present on the cell membrane that separates the inside and outside of the cell, and is abundant in macrophages. The phagocytic action of SIRPα is weakened when it binds to another membrane protein CD47 on the cell to be the target for phagocytosis by macrophages. That is, for example, cells lacking SIRPα gene or with suppressed expression of the gene have lost the ability to bind to CD47 present on the cell membrane of cancer cells; thus the phagocytic action thereof is not weakened.

The method for deleting the gene or suppressing its expression can be a method generally used in the field.

One embodiment is a nucleic acid that suppresses the expression of the SIRPα gene. The nucleic acid may act at any stage of the SIRPα gene, such as the transcription level, the level of post-transcriptional regulation, the level of translation into protein, or the level of post-translational modification. Thus, examples of the nucleic acid that suppresses the expression of the SIRPα gene include nucleic acids that inhibit the transcription of the SIRPα gene (e.g., antigene), nucleic acids that inhibit the processing of an initial transcription product into mRNA, and nucleic acids that inhibit the translation of mRNA into protein (e.g., antisense nucleic acid, miRNA) or degrade mRNA (e.g., siRNA, ribozyme, miRNA). Any nucleic acid that acts at either stage can be used, but a substance that binds complementarily to mRNA to inhibit translation into protein or degrades mRNA is preferred.

The nucleotide sequence of the SIRPα gene is known, and sequence information can be obtained, for example, from public databases (e.g., NCBI, EMBL, DDBJ, etc.).

Another preferred embodiment is a nucleic acid that suppresses the expression of the SIRPα gene by using genome editing. Specifically, the nucleic acid that suppresses the expression of the SIRPα gene can be a nucleic acid that can inactivate (knock out) the SIRPα gene. Such a nucleic acid can be a nucleic acid that encodes an artificial nuclease that consists of a nucleic acid sequence recognition module (e.g., CRISPR/Cas9, ZF motif, TAL effector, etc.) that can specifically recognize a partial nucleotide sequence in the gene as a target, and a nuclease that introduces a double-strand break (DSB) into the gene within or near the target sequence. After the introduction of DSB, the gene can be knocked out by insertion or deletion mutation due to a non-homologous end joining (NHEJ) repair error. Alternatively, gene knockout can be performed by homologous recombination (HR) repair by combining with a targeting vector in which a marker gene (e.g., a reporter gene such as a fluorescent protein gene, or a selection marker gene such as a drug resistance gene) is inserted into the gene sequence.

Thus, macrophage-like cells can be prepared by deleting a gene that inhibits a macrophage-like function or suppressing expression of the gene.

### 2. Step of expressing gene that confers proliferating property

The "gene that confers proliferating property" refers to a gene having a function that can promote cell proliferation by being introduced into a cell and expressed in the cell, and includes genes of various factors. In the present specification, it is also referred to as proliferation gene. Specifically, examples include, but are not limited to, genes such as c-MYC, BMI1, MDM2, and the like of any biological species, and homologs or orthologs thereof. The biological species is selected to be compatible with the animal species from which the cells to be introduced are derived. Preferably, at least one gene, preferably two genes, more preferably all three genes, selected from the group consisting of c-MYC, BMI1, and MDM2 are introduced and expressed.

Sequence information of various biological species of the proteins, genomic DNAs or cDNAs of "c-MYC", "BMI1", and "MDM2" can be obtained, for example, from sources well known to those skilled in the art, such as the Gene database of the National Center for Biotechnology Information (NCBI).

In the present invention, to induce expression means to produce a desired gene or protein in a cell and, in some cases, to cause secretion outside the cell to exhibit the function of the gene or protein.

In the present invention, "gene introduced" means that a desired gene is introduced into a specific cell, and the gene or a protein encoded by the gene is produced in the cell, or in some cases, secreted outside the cell to exhibit the function of the protein.

As one embodiment of the production method of the pMAC of the present invention, the following method can be mentioned (embodiment 1): a method including (1) a step of, in a pluripotent stem cell, deleting the gene that inhibits a macrophage-like function or suppressing expression of the gene, and then inducing differentiation into a myeloid cell to obtain a macrophage-like cell (MAC) (step 1), and
(2) a step of expressing the gene that confers proliferating property in the MAC obtained in step 1 to obtain a proliferating macrophage-like cell (pMAC) (step 2).

In step 1 of embodiment 1, the above-mentioned "1. Step of deleting the gene that inhibits a macrophage-like function or suppressing expression of the gene" is performed on the pluripotent stem cells. Here, the pluripotent stem cell is preferably an induced pluripotent stem cell or an embryonic stem cell, more preferably an induced pluripotent stem cell. Then, pluripotent stem cells lacking or with suppressed expression of a gene that inhibits a macrophage-like function, for example, pluripotent stem cells (particularly iPS cells) lacking or with suppressed expression of the SIRPα gene, are induced to differentiate into myeloid cells (hereinafter also referred to as "myeloid differentiation"), whereby macrophage-like cells (MACs) can be obtained.

The myeloid cell is a generic term for monocytes, macrophages, dendritic cells, and progenitor cells belonging to the cell lineages that differentiate into these cells, among leukocytes differentiated from stem cells. By causing myeloid differentiation of pluripotent stem cells lacking or with suppressed expression of a gene that inhibits a macrophage-like function, the differentiation-induced myeloid cells become macrophage-like cells. As a method for obtaining a macrophage-like cell (MAC) by myeloid differentiation of a pluripotent stem cells lacking or with suppressed expression of a gene that inhibits a macrophage-like function, the following method can be specifically mentioned.
(i) Performing a layered culture of embryoid body (EB) induced from pluripotent stem cells on feeder cells in a medium containing VEGF, and further culturing same in a medium containing VEGF, SCF, and TPO, or
(ii) Performing a monolayer culture of embryoid body (EB) induced from the pluripotent stem cells in a medium containing BMP-4, VEGF, and SCF without feeder cells, and further culturing same in a medium containing VEGF, TPO, and GM-CSF.

In the above-mentioned (i) and (ii), the pluripotent stem cell is a pluripotent stem cell lacking or with suppressed expression of a gene that inhibits a macrophage-like function.

In step 2 of embodiment 1, the above-mentioned "2. Step of expressing a gene that confers proliferating property" is performed on the MAC obtained in step 1.

As another embodiment of the production method of the pMAC of the present invention, the following method can be mentioned (embodiment 2): a method including (A) a step of expressing a gene that confers proliferating property in a myeloid cell differentiated from a pluripotent stem cell to obtain a proliferating myeloid cell (step A), and (B) a step of, in the proliferating myeloid cell obtained in step A, deleting the gene that inhibits a macrophage-like function or suppressing expression of the gene, and then inducing differentiation to obtain a proliferating macrophage-like cell (pMAC) (step B).

In step A of embodiment 2, the above-mentioned "2. Step of expressing the gene that confers proliferating property" is performed on a myeloid cell differentiated from a pluripotent stem cell (pluripotent stem cell-derived myeloid cell). Here, the pluripotent stem cell is preferably an induced pluripotent stem cell or an embryonic stem cell, more preferably an induced pluripotent stem cell. By this step, a myeloid cell conferred with the proliferating property can be obtained.

For induction of differentiation from pluripotent stem cells into myeloid cells, the following method can be specifically mentioned.
(i) Performing a layered culture of embryoid body (EB) induced from the pluripotent stem cells on feeder cells in a medium containing VEGF, and further culturing same in a medium containing VEGF, SCF, and TPO, or
(ii) performing a monolayer culture of embryoid body (EB) induced from the pluripotent stem cells in a medium containing BMP-4, VEGF, and SCF without feeder cells, and further culturing same in a medium containing VEGF, TPO, and GM-CSF.

In step B of embodiment 2, the above-mentioned "1. Step of deleting the gene that inhibits a macrophage-like function or suppressing expression of the gene" is performed on the proliferating myeloid cell obtained in step A. The proliferating myeloid cell is induced into a macrophage-like cell by deleting a gene that inhibits a macrophage-like function, for example, SIRPα gene, or suppressing expression of the gene, whereby pMAC can be obtained.

The "embryoid body" in the present invention refers to cell mass formed by adhesion of undifferentiated pluripotent stem cells. Due to the close cell-to-cell interaction in the cell mass, the repertoire of cell differentiation of the pluripotent stem cells that constitute the cell mass is limited, which resembles an embryo in the developmental stage in which blastoderm is formed after implantation.

The size of the embryoid body in the present invention is not particularly limited. The diameter is preferably 50 to 1000 µm, further preferably 50 to 500 µm, particularly preferably 50 to 200 µm. When the size of the embryoid body is larger than the above-mentioned range, the oxygen supply to the inside of the embryoid body may be insufficient and cell necrosis may occur. When the size of the embryoid body is smaller than the above-mentioned range, single cells may undergo cell death due to apoptosis without forming an embryoid body. Mouse embryoid body may be cultured using mouse mesenchymal C3H10T1/2 cells or mouse bone marrow-derived interstitial OP9 cells as feeder cells. Human embryoid body can be cultured without using feeder cells.

The "VEGF", "SCF", "TPO", "BMP-4", and/or "GM-CSF" of the present invention refer to VEGF, SCF, TPO, BMP-4, and/or GM-CSF protein of any species, or homologues or orthologues thereof, having an activity of inducing differentiation from pluripotent stem cells of human or mammals other than human into myeloid cells (MC).

As the "VEGF", "SCF", "TPO", "BMP-4", and/or "GM-CSF" in the present invention, VEGF, SCF, TPO, BMP-4 and/or GM-CSF protein of the biological species other than the biological species of the embryoid body to be cultured, or variants of VEGF, SCF, TPO, BMP-4 and/or GM-CSF protein of the same biological species as the biological species of the embryoid body to be cultured can be used, provided that they have the same level of differentiation-inducing activity as that of "VEGF", "SCF", "TPO", "BMP-4" and/or "GM-CSF" of the biological species of the embryoid body to be cultured.

In the present specification, Flt-3L, IL-3, and/or bFGF are/is also used at times together with the aforementioned VEGF, SCF, TPO, BMP-4, and/or GM-CSF, and the like when culturing cells in the present invention. These can be obtained, for example, from sources well known to those skilled in the art, such as the Gene database of the National Center for Biotechnology Information (NCBI). As the gene-recombinant protein reagent, for example, Recombinant Human Flt-3 Ligand/FLT3L Protein (308-FKN), Recombinant Human IL-3 Protein (203-IL), Recombinant Human FGF basic/FGF2/bFGF (145 aa) Protein (3718-FB), Recombinant Human VEGF 165 Protein (293-VE), Recombinant Human SCF Protein (255-SC), Recombinant Human Thrombopoietin Protein (288-TP), Recombinant Human BMP-4 Protein (314-BP), and Recombinant Human GM-CSF Protein (215-GM) may also be purchased from R&D Systems (https://www.rndsystems.com/).

Sequence information of various biological species of the proteins, genomic DNAs or cDNAs of "VEGF", "SCF", "TPO", "BMP-4" and/or "GM-CSF" of the present invention can be obtained, for example, from sources well known to those skilled in the art, such as the Gene database of the National Center for Biotechnology Information (NCBI).

As the aforementioned gene and protein, those of the same kind as the pluripotent stem cells to be used are preferred. For example, when the pluripotent stem cells are derived from human, it is preferable to use VEGF, SCF, TPO, BMP-4, GM-CSF, Flt-3L, IL-3, and/or bFGF derived from human. When the pluripotent stem cells are derived from mouse, it is also preferable to use the gene or protein derived from mouse.

In the present invention, the "medium containing substance X" means a medium supplemented with exogenous substance X or a medium containing exogenous substance X, and the "medium free of substance X" means a medium not supplemented with exogenous substance X or a medium not containing exogenous substance X. As used herein, the "exogenous substance X" means a substance X foreign to the cell or tissue cultured in the medium, and does not include endogenous substance X produced by the cell or tissue.

For example, the "medium containing VEGF" means a medium supplemented with exogenous VEGF or a medium containing exogenous VEGF. While the dose of VEGF may be influenced by other growth factors and the like, 0.2 ng/mL to 200 ng/mL is preferred, 2 ng/mL to 100 ng/mL is further preferred, and 5 ng/mL to 50 ng/mL is particularly preferred. The "medium free of VEGF" is a medium not supplemented with exogenous VEGF or a medium not containing exogenous VEGF.

Similarly, as regards SCF, TPO, BMP-4, and GM-CSF, a medium containing SCF, TPO, BMP-4 and/or GM-CSF means a medium supplemented with exogenous SCF, TPO, BMP-4 and/or GM-CSF or a medium containing exogenous SCF, TPO, BMP-4 and/or GM-CSF. Therefore, while the dose of SCF, TPO, BMP-4 and/or GM-CSF may be influenced by other growth factors and the like, 0.2 ng/mL to 200 ng/mL is preferred, 2 ng/mL to 100 ng/mL is further preferred. In the case of TPO, it is more preferably 5 ng/mL to 15 ng/mL. In the cases of SCF, BMP-4 and GM-CSF, it is more preferably 5 ng/mL to 70 ng/mL.

The "medium free of SCF, TPO, BMP-4 and/or GM-CSF" means a medium not supplemented with exogenous VEGF or a medium not containing exogenous SCF, TPO, BMP-4 and/or GM-CSF.

αMEM supplemented with 20% fetal bovine serum (FBS) can be used as a medium for mouse embryoid body in the present invention. Mouse mesenchymal C3H10T1/2 cells or mouse bone marrow-derived interstitial OP9 cells can be used as feeder cells. A completely synthetic medium, StemFit (Ajinomoto Co., Inc.), can be used as a medium for human embryoid body.

As the medium, αMEM supplemented with 20% FBS and the like can be used.

In the present invention, the "feeder cell" is other cell type used to adjust the culture conditions for undifferentiated maintenance culture and differentiation induction culture of iPS cells. Feeder cells that underwent mitomycin C treatment or radiation irradiation in order to stop the growth thereof are used. Mouse bone marrow-derived interstitial cells OP-9, mouse-derived mesenchymal cells C3H10T1/2, and the like can be used.

In the present invention, the "layered culture" refers to seeding and coculturing the iPS cells or embryoid body of interest in a feeder cell layer culture system cultured in advance in a predetermined culture container. In the present invention, the "monolayer culture" means culturing a single cell type being adhered to a container for tissue culture.

In the present invention, the monolayer culture without feeder cells refers to seeding the iPS cells or embryoid body of interest in a culture container and performing static culture.

In addition, in one embodiment of the present invention, the present invention includes forming an embryoid body from pluripotent stem cells by the following method:
(i) seeding a single cell dispersion of pluripotent stem cells in a container for cell mass-forming culture to form an embryoid body (EB), wherein
(ii) the aforementioned container has fine spheroid wells at the bottom, and does not have a flat surface between adjacent spheroid wells.

"Forming an embryoid body" means dissociating pluripotent stem cells proliferating in an undifferentiated state to obtain a single-cell dispersion of the pluripotent stem cells, and forming qualitatively uniform cell mass by suspension culture or static culture under conditions in which pluripotent stem cells adhere and aggregate. The formation of embryoid body is confirmed by visual observation of the presence of viable cells under a microscope.

In the present specification, the "suspension culture" refers to culturing under conditions in which cells or cell masses are kept in a suspending state so that they do not adhere to substrates such as the bottom, walls, and the like of culture containers.

The incubator used for suspension culture is not particularly limited as long as it is capable of "suspension culture", and can be appropriately determined by those skilled in the art. Examples of such incubator include flask, tissue culture flask, dish, petri dish, tissue culture dish, multidish, microplate, microwell plate, micropore, multiplate, multiwell plate, chamber slide, schale, tube, tray, culture bag, and roller bottle. These incubators are preferably cell non-adhesive so that suspension culture can be performed. As the cell non-adhesive incubator, an incubator free of an artificial surface treatment (for example, coating treatment with an extracellular matrix or the like) for the purpose of improving adhesion to cells and the like can be used.

In the present invention, it is preferable to rapidly aggregate pluripotent stem cells to form pluripotent stem cell-derived embryoid body. When cell masses of pluripotent stem cells are formed in this way, transferred to a medium supplemented with VEGF at a concentration of 50 ng/mL, and cultured, the cells of the cell mass can be differentiated into the cell lineage of myeloid cells in the same manner as blood island in the yolk sac.

Experimental operation for forming embryoid body includes, for example, a method of confining cells in a small space using a culture container such as a plate with a large number of small-diameter holes (wells) (e.g., 96-well plate), a container for cell mass-forming culture and the like, and a method of aggregating cells by briefly centrifuging them in a small centrifuge tube. The culture conditions for aggregates of cells or pluripotent stem cells include rotary culture, shaking culture, and other suspension cultures. In addition, static culture can also be performed on the condition that the adhesion between the pluripotent stem cells and the surface of the substrate of the culture container that comes into contact with the cells is reduced or suppressed by subjecting the substrate surface of the culture container to a treatment in advance to make same hydrophobic or the like.

The formation of embryoid body as cell mass of pluripotent stem cells and the differentiation of embryoid body cells into myeloid cells can be determined using microscopy and FACS based on characteristics including, but not limited to, confirmation of the size and cell number of embryoid body, confirmation of the microscopic morphology of embryoid body under culture, confirmation of histological or cytochemical findings, and/or confirmation of expression of differentiation and undifferentiation markers, uniformity thereof, regulation of expression of differentiation markers, synchronicity thereof, or reproducibility of differentiation efficiency between embryoid bodies, and the like.

In the present invention, when a certain cell is "positive" for a certain marker, an average fluorescence intensity ratio of the positive cells to the negative control, as measured by flow cytometry of the cells, of 10 times or more is +, 2 times or more to less than 10 times is low, and less than 2 times is -.

In the present invention, moreover, high or low expression of a gene in a cell means an expression level of the gene with respect to housekeeping gene β actin, GAPDH (glyceraldehyde-3-phosphate dehydrogenase), HPRT1 (hypoxanthine phosphoribosyltransferase 1), or β2-microglobulin, as measured by the real-time PCR method, and is high when it is 1-fold or more and low when it is less than 1-fold.

In the present invention, the single cell dispersion is prepared by washing cultured cells with PBS or the like, treating them with a cell dissociation solution trypsin-EDTA (Life Technologies) or TrypLE Select (Life Technologies) for 4 to 5 minutes, further washing them with PBS or the like, centrifuging them to obtain pellets of the desired cells, and resuspending the pellets by pipetting with a desired culture solution or the like.

In the present invention, the container for cell mass-forming culture means a container in which cells seeded during culture can form cell aggregates without adhering to the container. Preferably, the container is provided with fine spheroid wells on its bottom, which is the culture surface, and does not have a flat surface between adjacent spheroid wells, in which the adjacent spheroid wells are separated by a curved surface that slopes toward any spheroid well. Since a surface treatment to suppress adhesiveness of cells is applied to the curved surface, most of the cells seeded in the culture container drop into any of the spheroid wells.

An example of the container is a container in which spheroid wells with a diameter of about 400 to 800 µm and a depth of 100 to 800 µm are uniformly placed without gaps on the culture surface (bottom) up to the wall surface. Preferably, the spheroid well has a diameter of 400 to 500 µm and a depth of 50 to 200 µm. A container with spheroid wells having a diameter of about 500 µm and a depth of about 400 µm, a container with spheroid wells having a diameter of about 800 µm and a depth of about 400 µm, and a container with spheroid wells having a diameter of about 800 µm and a depth of about 300 µm are more preferred. A container with spheroid wells having a diameter of about 400 µm and a depth of 100 to 200 µm is most preferred.

As the container, one in which spheroid wells are uniformly processed without gaps on the culture surface (bottom) up to the wall surface is preferred. Such container does not have a flat surface between adjacent spheroid wells and seeded single cell suspension drops into any of the spheroid wells, thus forming uniform aggregates. More preferred is a micro-fabricated cell culture container (EZSPHERE (registered trade mark)) manufactured by AGC.

pMAC can be produced by either the method of embodiment 1 or embodiment 2, and is preferably produced by the method of embodiment 1. A highly pure cell population containing pMAC and containing few undesired cells can be stably produced by performing a step of expressing a gene that confers proliferating property after a step of deleting the gene that inhibits a macrophage-like function or suppressing expression of the gene. More specific procedures of embodiment 1 are as follows.
(1) a step of, in a pluripotent stem cell, deleting the gene that inhibits a macrophage-like function or suppressing expression of the gene (step 1-1),
   a step of seeding the pluripotent stem cell obtained in step 1-1, in which the gene that inhibits a macrophage-like function is deleted or expression of the gene is suppressed, in a container for cell mass-forming culture that has fine spheroid wells at the bottom, and does not have a flat surface between adjacent wells, to form an embryoid body (EB) (step 1-2),
   a step of obtaining a macrophage-like cell (MAC) by the following method (i) or (ii) from the EB obtained in step 1-2 (step 1-3),
      (i) performing a layered culture of the aforementioned EB on feeder cells in a medium containing VEGF, and further culturing same in a medium containing VEGF, SCF, and TPO, or
      (ii) performing a monolayer culture of the aforementioned EB in a medium containing BMP-4, VEGF, and SCF without feeder cells, and further culturing same in a medium containing VEGF, TPO, and GM-CSF,
(2) a step of expressing the gene that confers proliferating property in the MAC obtained in step 1-3 to obtain a proliferating macrophage-like cell (pMAC) (step 2).

As used herein, the gene that inhibits a macrophage-like function is the SIRPα gene.

The pMAC thus obtained has phagocytic ability like macrophages, and also has characteristics different from those of existing macrophages, such as proliferation in a cytokine-dependent manner.

### <pMAC containing chimeric antigen receptor and production method thereof>

The present invention provides pMAC containing a chimeric antigen receptor (CAR) (hereinafter also referred to as CAR-pMAC) and a production method thereof. In the production method of CAR-pMAC of the present invention, the CAR gene can be introduced into pMAC to which cytokine-dependent proliferating property has been conferred as described in the above-mentioned "production method of the pMAC of the present invention", and can also be introduced, for example, before deleting the gene that inhibits a macrophage-like function or suppressing expression of the gene in pluripotent stem cells, or after deleting the gene that inhibits a macrophage-like function or suppressing expression of the gene in pluripotent stem cells. The production method of CAR-pMAC of the present invention includes a step of expansion culturing the obtained CAR-pMAC.

One embodiment of the production method of CAR-pMAC of the present invention is the following method (embodiment I). A method for producing CAR-pMAC, including
(1) a step of, in a pluripotent stem cell, deleting the gene that inhibits a macrophage-like function or suppressing expression of the gene, and then inducing differentiation into a myeloid cell to obtain a macrophage-like cell (step 1),
(2) a step of expressing the gene that confers proliferating property in the macrophage-like cell obtained in step 1 to obtain a proliferating macrophage-like cell (pMAC) (step 2), and
(3) a step of introducing a chimeric antigen receptor (CAR) (step 3).

Step 1 and step 2 in this embodiment can be respectively performed in the same manner as step 1 and step 2 in embodiment 1 of the above-mentioned "production method of pMAC of the present invention".

Another embodiment of the production method of CAR-pMAC of the present invention is the following method (embodiment II).
(A) a step of expressing a gene that confers proliferating property in a myeloid cell differentiated from a pluripotent stem cell to obtain a proliferating myeloid cell (step A),
(B) a step of, in the proliferating myeloid cell obtained in step A, deleting the gene that inhibits a macrophage-like function or suppressing expression of the gene, and then inducing differentiation to obtain pMAC (step B), and
(C) a step of introducing a chimeric antigen receptor (CAR) (step C).

Step A and step B in this embodiment can be respectively performed in the same manner as step A and step B in embodiment 2 of the above-mentioned "production method of pMAC of the present invention".

Another embodiment of the production method of CAR-pMAC of the present invention is the following method (embodiment III).

A method for producing CAR-pMAC, including
(1') a step of introducing a chimeric antigen receptor (CAR) into a pluripotent stem cell (step 1'),
(2') a step of, in the pluripotent stem cell obtained in step 1' into which the CAR gene has been introduced, deleting the gene that inhibits a macrophage-like function or suppressing expression of the gene, and then inducing differentiation into a myeloid cell to obtain a macrophage-like cell into which the CAR gene has been introduced (step 2'), and
(3') a step of expressing the gene that confers proliferating property in the macrophage-like cell obtained in step 2' into which the CAR gene has been introduced to obtain a proliferating macrophage-like cell (CAR-pMAC) into which the CAR gene has been introduced (step 3').

Step 2' and step 3' in this embodiment can be respectively performed in the same manner as step 1 and step 2 in embodiment 1 of the above-mentioned "production method of pMAC of the present invention" except that the CAR gene has been introduced in advance into the target cells.

Another embodiment of the production method of CAR-pMAC of the present invention is the following method (embodiment IV).

A method for producing CAR-pMAC, including
(A') a step of introducing a chimeric antigen receptor (CAR) into a pluripotent stem cell (step A'),
(B') a step of expressing a gene that confers proliferating property in a myeloid cell differentiated from the pluripotent stem cell obtained in step A' into which the CAR gene has been introduced to obtain a proliferating myeloid cell into which the CAR gene has been introduced (step B'), and
(C') a step of, in the proliferating myeloid cell obtained in step B' into which the CAR gene has been introduced, deleting the gene that inhibits a macrophage-like function or suppressing expression of the gene, and then inducing differentiation to obtain a proliferating macrophage-like cell (CAR-pMAC) into which the CAR gene has been introduced (step C') .

Step A' and step B' in this embodiment can be respectively performed in the same manner as step A and step B in embodiment 2 of the above-mentioned "production method of pMAC of the present invention" except that the CAR gene has been introduced in advance into the target cells.

The introduction of a CAR gene into cells in step 3 in embodiment I and embodiment II, step 1' in embodiment III, and step A' in embodiment IV are described in detail below.

### Chimeric antigen receptor (also referred to as CAR in the present specification)

CAR is a structure that contains, from the N-terminal side to the C-terminal side of a protein, a target-specific extracellular domain, a transmembrane domain, and an intracellular signal domain for effector functions of immune cells, and CAR gene is a gene encoding this receptor. The extracellular domain contains an antigen recognition site that exhibits target-specific binding. The transmembrane domain exists between the extracellular domain and the intracellular signal domain. The intracellular signal domain transmits signals necessary for immune cells to exert their effector functions. That is, when the extracellular domain binds to a target antigen, an intracellular signal domain is used that is capable of transmitting a signal necessary for activating immune cells.

There have been several reports of experiments and clinical studies using CAR (e.g., Rossig C, et al. Mol Ther 10:5-18, 2004; Dotti G, et al. Hum Gene Ther 20:1229-1239, 2009; Ngo MC, et al. Hum Mol Genet 20 (R1):R93-99, 2011; Ahmed N, et al. Mol Ther 17:1779-1787, 2009; Pule MA, et al. Nat Med 14:1264-1270, 2008; Louis CU, et al. Blood 118:6050-6056, 2011; Kochenderfer JN, et al. Blood 116:4099-4102, 2010; Kochenderfer JN, et al. Blood 119:2709-2720, 2012; Porter DL, et al. N Engl J Med 365:725-733, 2011; Kalos M, et al. Sci Transl Med 3:95ra73, 2011; Brentjens RJ, et al. Blood 118:4817-4828, 2011; Brentjens RJ, et al. Sci Transl Med 5:177 ra38, 2013), and the CAR of the present invention can be constructed with reference to these reports.

### Introduction of CAR gene into pMAC

In the present invention, introduction of a CAR gene is performed by introducing the CAR gene into a desired cell by a CAR expression vector. The cells into which the CAR gene is introduced are, for example, pMAC provided by the above-mentioned present invention, and a pluripotent stem cell before or after deleting the gene that inhibits a macrophage-like function or suppressing expression of the gene. The CAR expression vector means a nucleic acid molecule capable of transporting a nucleic acid molecule encoding a CAR gene into target cells. It is not questioned whether it is DNA or RNA and is not particularly limited as to the form, origin, and the like, and various kinds of vectors can be used. The vector may be a viral vector or a non-viral vector. As the viral vector, retroviral vector, lentivirus vector, adenoviral vector, adeno-associated viral vector, herpes virus vector, sendai virus vector, vaccinia virus vector, and the like can be mentioned. Among these, with retroviral vectors, lentivirus vectors, and adeno-associated viral vectors, the target gene incorporated into the vector is integrated into the host chromosome, and stable and long-term expression can be expected. Each viral vector can be produced according to a conventional method or using a commercially available dedicated kit. Non-viral vectors include plasmid vectors, liposome vectors, positively charged liposome vectors (Felgner, P.L., Gadek, T.R., Holm, M. et al., Proc. Natl. Acad. Sci., 84:7413-7417, 1987), YAC vectors, BAC vectors, artificial chromosome vectors, and the like.

A CAR expression vector contains an expression unit for expressing a CAR gene, and the expression unit generally contains a promoter, a CAR gene, and a poly A addition signal. The expression unit is derived from various organisms or viruses or is composed of any sequence, and includes similar sequences and modified units thereof. Promoters that can be used in the CAR expression cassette include CAG promoter, CMV-IE (cytomegalovirus early gene-derived promoter), SV40ori, retrovirus LTRSRα, EF1α, β actin promoter, and the like. Examples of the poly A addition signal sequence include poly A addition sequence of SV40, poly A addition sequence of bovine-derived growth hormone gene, globulin poly A addition sequence, and the like. In order for the expression of the CAR gene to be controlled by the promoter, the CAR gene is generally linked directly to the 3'-terminal side of the promoter or via other sequence, and a poly A addition signal sequence is configured downstream of the CAR gene. The CAR gene is transcribed into messenger RNA (mRNA) by such expression unit, and CAR is translated from the mRNA and displayed on the cell surface.

The expression unit may contain detection genes (reporter genes, cell- or tissue-specific genes, selection marker genes, and the like) to enable detection of gene expression, an enhancer sequence to improve expression efficiency, a WRPE sequence, and the like.

The detection gene is used for determining the success or failure and efficiency of introduction of the CAR expression vector, detecting the expression of the CAR gene or determining the expression efficiency, and selecting and sorting cells in which the CAR gene is expressed, and the like. As the detection gene, the neo gene that confers resistance to neomycin, kmr gene and nptII gene that confer resistance to kanamycin and the like (Bernd Reiss et al. EMBO J. 3 (1984), 3317-3322), hph gene that confers resistance to hygromycin (Blochlinger & Diggelmann, Mol Cell Bio 4:2929-2931), DHFR gene that confers resistance to methotrexate (Bourouis et al., EMBO J.2(7)), and the like (all marker genes); genes for fluorescent proteins such as luciferase gene (Giacomin, P1. Sci. 116(1996), 59-72; Scikantha, J. Bact. 178(1996), 121), β-glucuronidase (GUS) gene, GFP (Gerdes, FEBS Lett. 389(1996), 44-47) or variants thereof (EGFP, d2EGFP, and the like), tdTomato or variants thereof (pTdTomato and the like), and the like (all reporter genes); genes such as the epidermal growth factor receptor (EGFR) gene, which lacks an intracellular domain, and the like can be used. The detection gene may be linked to the CAR gene via, for example, a bicistronic control sequence (e.g., internal ribosome entry site (IRES)) or a sequence encoding a self-cleavage peptide. As the self-cleavage peptide, 2A peptide (T2A) derived from Thosea asigna virus can be mentioned. Examples of other self-cleavage peptide include, but are not limited to, 2A peptide (F2A) derived from picornavirus, 2A peptide (F2A) derived from foot- and-mouth disease virus (FMDV), 2A peptide (E2A) derived from equine rhinitis A virus (ERAV), 2A peptide (P2A) derived from porcine teschovirus (PTV-1), and the like, 2A peptide derived from rotavirus, insect virus, aphthovirus, or trypanosoma virus, and the like. Similar sequences and partially modified sequences can also be mentioned.

The CAR gene expression vector prepared for gene transfer is introduced into pMAC by a conventional method. In the case of viral vectors, they are introduced into cells by infection with the virus. In the case of non-viral vectors such as plasmids, conventional methods such as electroporation method, liposomes method, calcium phosphate method, nucleofection method, and the like are used for introduction into cells, and electroporation method is preferably used for introduction.

In order to improve the efficiency of integration into the host chromosome, it is preferable to perform gene introduction by the transposon method. The transposon method is one of nonviral gene introduction methods that uses a pair of gene enzyme (transposase) and its specific recognition sequence to cause gene transposition, and can integrate any gene into the host chromosome. As the transposon method, for example, the piggyBac transposon method can be used. The piggyBac transposon method utilizes transposons isolated from insects (Fraser MJ et al., Insect Mol Biol. 1996 May; 5(2):141-51.; Wilson MH et al., Mol THER2007 Jan; 15(1):139-45.), and enables highly efficient integration into mammalian chromosomes. The piggyBac transposon method is actually used for gene introduction (e.g., Nakazawa Y, et al., J Immunother 32:826-836, 2009; Nakazawa Y et al., J Immunother 6:3-10, 2013, and the like).

The transposon method is not limited to those using piggyBac and may be, for example, transposon-based methods such as Sleeping Beauty (Ivics Z, Hackett PB, Plasterk RH, Izsvak Z (1997) Cell 91: 501-510.), Frog Prince (Miskey C, Izsvak Z, Plasterk RH, Ivics Z (2003) Nucleic Acids Res 31: 6873-6881.), Toll (Koga A, Inagaki H, Bessho Y, Hori H. Mol Gen Genet. 1995 Dec 10; 249(4):400-5.; Koga A, Shimada A, Kuroki T, Hori H, Kusumi J, Kyono-Hamaguchi Y, Hamaguchi S. J Hum Genet. 2007; 52(7):628-35. Epub 2007 Jun 7.), Tol2 (Koga A, Hori H, Sakaizumi M (2002) Mar Biotechnol 4: 6-11.; Johnson Hamlet MR, Yergeau DA, Kuliyev E, Takeda M, Taira M, Kawakami K, Mead PE (2006) Genesis 44: 438-445.; Choo BG, Kondrichin I, Parinov S, Emelyanov A, Go W, Toh WC, Korzh V (2006) BMC Dev Biol 6: 5.), and the like.

The operation of gene introduction using the transposon method can be performed by a conventional method. For example, for the piggyBac transposon method, a vector carrying a gene encoding piggyBac transposase (transposase plasmid) and a vector having a structure in which the CAR gene expression unit is sandwiched between piggyBac inverted repeat sequences (transposon plasmid) are prepared, and these vectors can be introduced into target cells by various techniques such as electroporation, nucleofection, lipofection, and calcium phosphate method.

As the treatment after CAR gene introduction, CAR-pMAC is expansion cultured to a necessary scale. The CAR-pMAC of the present invention can be proliferated in a cytokine-dependent manner. Therefore, in the production method of the CAR-pMAC of the present invention, the aforementioned expansion culture is preferably performed in the presence of at least one, preferably two, cytokines selected from the group consisting of GM-CSF and M-CSF.

The medium used when preparing CAR-pMAC is not particularly limited, and a medium used for normal cell culture, such as RPMI1640, MEM, X-VIVIO, IMDM, DMEM, DC medium, OptiMEM, and the like can be used. The medium may be a medium to which serum (human serum, fetal bovine serum, etc.) is added according to a conventional method, or a serum-free medium. It is preferable to use a serum-free medium because it is highly safe for clinical application and the culture efficiency is less likely to vary due to differences between serum lots. Examples of serum-free media include TexMACS TM (Miltenyi Biotec), AIM V (registered trademark) (Thermo Fisher Scientific), ALyS culture medium (Cell Science & Technology Institute, Inc.), and the like. When using serum, autologous serum, that is, serum collected from the individual from whom the CAR-expressing immune cells are derived (more specifically, a patient receiving administration of the cell population obtained by the production method of the present disclosure) may also be used, or it may also be an artificial serum. In the present invention, it is preferable to use autologous serum. As the basal medium, one suitable for cell culture may be used, and the aforementioned TexMACS TM (Miltenyi Biotec), AIM V (registered trademark), and ALyS culture medium (Cell Science & Technology Institute, Inc.) can be used. Other culture conditions may be those suitable for cell survival and proliferation, and general conditions may be employed. For example, culturing in a CO₂ incubator (CO₂ concentration 5%) set at 37°C, hypoxic culture (oxygen concentration 0 to 20%, preferably 0 to 10%, more preferably 1 to 5%), and the like can be mentioned.

By expansion culturing CAR-pMAC, a cell population containing CAR-pMAC can be obtained in sufficient quantity and quality for clinical use.

After expansion culture, CAR-pMAC is collected. The collection operation may be performed in a conventional manner. For example, the cells are collected by pipetting, centrifugation treatment, or the like. In a preferred embodiment, before the collection operation, a step of culturing the cells after expansion culture in the presence of a stimulating substance is performed. This step enables efficient expansion culture and also has the advantage of increasing cell survival rate. When desired, the CAR-pMAC population after expansion culture may be subjected to a cell separation step such as bead separation. By performing the cell separation step, the purity of CAR-pMAC with higher effect can be increased,

A cell population containing CAR-pMAC produced by the method of the present invention can be used for the treatment of diseases related to tumor or cancer, particularly for the treatment of cancer that expresses the target antigen of the CAR-expressing immune cells. Tumor or cancer-related disease may be a solid tumor or a blood tumor. Specific examples of cancer include, but are not limited to, various B-cell lymphomas (follicular malignant lymphoma, diffuse large B-cell malignant lymphoma, mantle cell lymphoma, MALT lymphoma, intravascular B-cell lymphoma, CD20-positive Hodgkin's lymphoma, etc.), myeloproliferative neoplasms, myelodysplastic/myeloproliferative neoplasms (CMML, JMML, CML, MDS/MPN-UC), myelodysplastic syndromes, acute myeloid leukemia, neuroblastoma, brain tumors, Ewing's sarcoma, osteosarcoma, retinoblastoma, small cell lung cancer, non-small cell lung cancer, melanoma, bone and soft tissue sarcoma, kidney cancer, pancreatic cancer, malignant mesothelioma, prostate cancer, breast cancer, uterine cancer, cervical cancer, ovarian cancer, and colorectal cancer. In a preferred embodiment, the cancer is a solid tumor. Examples of solid tumors include neuroblastoma, brain tumor, Ewing's sarcoma, osteosarcoma, retinoblastoma, small cell lung cancer, non-small cell lung cancer, melanoma, ovarian cancer, rhabdomyosarcoma, bone and soft tissue sarcoma, kidney cancer, pancreatic cancer, malignant mesothelioma, prostate cancer, breast cancer, uterine cancer, cervical cancer, ovarian cancer, and colorectal cancer.

Furthermore, the cell population containing CAR-pMAC produced by the method of the present invention can be used for the treatment of neurodegenerative diseases, inflammatory diseases, cardiovascular diseases, fibrotic diseases, and amyloidosis. Examples of the "neurodegenerative diseases" include tauopathy, α-synucleopathy, presenile dementia, senile dementia, Alzheimer's disease, Parkinsonism linked to chromosome 17 (FTDP-17), progressive supranuclear palsy (PSP), Pick's disease, primary progressive aphasia, frontotemporal dementia, corticobasal dementia, Parkinson's disease, Parkinson's disease with dementia, dementia with Lewy bodies, Down's syndrome, multiple system atrophy, amyotrophic lateral sclerosis (ALS), Hallervorden-Spatz syndrome, polyglutamine disease, trinucleotide repeat disease, prion disease, and the like. Examples of the "inflammatory diseases" include systemic lupus erythematosus, vasculitis, rheumatoid arthritis, periodontitis, ulcerative colitis, sinusitis, asthma, tuberculosis, Crohn's disease, chronic infections, hereditary periodic fevers, malignant tumors, systemic vasculitis, cystic fibrosis, bronchiectasis, epidermolysis bullosa, periodic neutropenia, acquired or hereditary immunodeficiency, injection drug use, and acne conglobata, Muckle-Wells (MWS) disease, and familial Mediterranean fever (FMF). Examples of the "cardiovascular diseases" include atherosclerosis, coronary artery disease, peripheral arterial disease, hypertensive heart disease, metabolic syndrome, hypertension, cerebrovascular disease, and heart failure. Examples of the "fibrosis diseases" include pulmonary fibrosis, idiopathic pulmonary fibrosis, cirrhosis, cystic fibrosis, scleroderma, cardiac fibrosis, radiation-induced lung injury, steatohepatitis, glomerulosclerosis, interstitial lung disease, hepatic fibrosis, mediastinal fibrosis, retroperitoneal fibrosis, myelofibrosis, and skin fibrosis. Examples of the "amyloidosis" include primary amyloidosis (AL), secondary amyloidosis (AA), familial amyloidosis (ATTR), other familial amyloidosis, beta-2 microglobulin amyloidosis, localized amyloidosis, heavy chain amyloidosis (AH), light chain amyloidosis (AL), primary systemic amyloidosis, ApoAI amyloidosis, ApoAII amyloidosis, ApoAIV amyloidosis, apolipoprotein C2 amyloidosis, apolipoprotein C3 amyloidosis, corneal lactoferrin amyloidosis, transthyretin-related amyloidosis, dialysis amyloidosis, fibrinogen amyloidosis, Lect2 amyloidosis (ALECT2), and lysozyme amyloidosis.

A cell population containing CAR-pMAC produced by the method of the present invention is administered in a therapeutically effective amount that is appropriately determined according to the age, body weight, body surface area, symptoms, and the like of the subject. The subject in the present disclosure is generally a human, preferably a cancer patient. A cell population containing CAR-pMAC produced by the method of the present invention can be administered at, for example, 1x10⁴ cells to 1x10¹⁰ cells per dose. The route of administration is not particularly limited, and the cells can be administered intratumorally, peritumorally, intraventricularly, intravenously, intraarterially, intraportally, intradermally, subcutaneously, intramuscularly, or intraperitoneally. The cell population of the present disclosure may be administered systemically or locally, and local administration includes direct injection into the target tissue, internal organ, or organ. The administration schedule is appropriately determined according to the age, body weight, body surface area, symptom, and the like of the subject, and may be a single administration or continuous or periodic multiple administrations.

The cell population containing CAR-pMAC produced by the method of the present invention can be mixed with a pharmaceutically acceptable carrier to form a pharmaceutical composition. Examples of the "pharmaceutically acceptable carrier" include components generally used in medicaments, such as conventional excipients, binders, buffers, water for injection, isotonicity agents, preservatives, and soothing agents. It may contain, in addition to the cell population to be administered to a subject, components such as dimethyl sulfoxide (DMSO), serum albumin, and the like for the purpose of cell protection, antibiotics and the like for the purpose of preventing bacterial contamination, various components (vitamins, cytokine, growth factor, steroid, etc.) for the purpose of cell activation, proliferation, or differentiation induction, and the like. The pharmaceutical composition can be prepared by conventional methods.

The present invention is described in detail below using examples, but the present invention is not limited in any way. Reagents and materials to be used are commercially available unless otherwise specified, or can be prepared from known literature and the like. In addition, those skilled in the art understand that other substances having similar effects and actions can be used alternatively.

### [Example]

### Example 1: Production of human iPSC

Human iPSC was produced by the method described in Kitayama, S. et al. (Stem Cell Reports. 6: 213-227, (2016)). The maintenance culture of human iPSC was performed using StemFit AK02N (Ajinomoto Healthy Supply), which is a medium for regenerative medicine, on polystyrene tissue culture plates coated with iMatrix511 (Nippi). The medium was changed every 1 or 2 days, and the cells were treated with the cell dissociation solution TrypLE Select (Life Technologies) for 4 to 5 minutes once a week depending on the growth of the cells, and collected as a single cell dispersion. The cell dispersion was seeded in a culture container with a suitable size and culture was continued. The seeded iPSCs were cultured overnight in the presence of 10 µM Y-27632 (FUJIFILM Wako Pure Chemical Corporation), a Rock inhibitor, and the medium was changed the next day to remove Y-27632.

### Example 2: Production of mouse iPSC

Mouse iPSC was produced by the method described in Araki, R. et al. (Nature, 494:100-104. (2013)). The maintenance culture of mouse iPSC was performed using complete medium complete ESM (cESM) prepared by adding 2-mercaptoethanol (Life Technologies) at a final concentration of 55 µM and 10⁴ U/mL Leukemia Inhibitory Factor (LIF) (Merck, trade name ESGROmLIF) to ES cell medium ESM (15% KSR (Life Technologies)-containing DMEM (FUJIFILM Wako Pure Chemical Corporation), on feeder cells of mouse fetal fibroblast MEF (ReproCELL) seeded on a 6-well tissue culture plate.

Feeder cells were prepared by seeding MEF (ReproCELL), whose cell proliferation had been stopped by mitomycin C treatment, on a gelatin-coated dish, and used for the maintenance culture of mouse iPSCs from the day after adhesion of MEFs.

### Example 3: Deletion of SIRPα gene by genome editing

Using sequence information from the database of the National Center for Biotechnology Information (NCBI), single guide RNAs of human SIRPα and mouse SIRPα were synthesized by nucleic acid synthesis. The single guide RNA of each gene was mixed with Guide-it^{™} Recombinant Cas9 protein (Takara Bio Inc.) to prepare a Cas9 protein-guide RNA complex (RNP), which was added to an iPSC single cell dispersion, and the RNA was introduced into the cells using an electroporation method. An iPSC clone deficient in the SIRPα gene was obtained by sequence analysis of the genomic DNA sequence targeted by the guide RNA and expression analysis by flow cytometry using differentiated cells obtained by differentiation induction in Example 5.

### Example 4: Production of iPSC-derived embryoid body

The embryoid body derived from human iPSC (wild-type or SIRPα-deficient type) (hereinafter to be indicated as human EB) was produced by seeding human iPSC at a given density prepared with StemFit containing 10 µM Y-27632 in a 6-well plate type cell mass-forming culture container EZ SPHERE SP (AGC Techno Glass). One to four days after the formation of embryoid bodies with a diameter of 50 to 200 µm, differentiation induction culture of Example 5 described later was performed.

The embryoid body derived from mouse iPSC (wild-type or SIRPα-deficient type) (hereinafter to be indicated as mouse EB) was produced by seeding mouse iPSC at a given density prepared with complete medium cESM in a 6-well plate type EZ SPHERE SP (AGC Techno Glass). One to four days after the formation of embryoid bodies with a diameter of 50 to 200 µm, differentiation induction culture of Example 5 described later was performed.

### Example 5: Differentiation induction of embryoid body into myeloid cell

### (1) Preparation of feeder cell

A mouse-derived cultured cell line C3H10T1/2 or OP9 was seeded on a gelatin-coated dish, and the cell line was used the next day. Feeder cells used for differentiation induction of human iPSC-derived embryoid body were irradiated with 60 Gy radiation to stop proliferation and then used. BME medium (Life Technologies) containing 10% FBS was used to culture C3H10T1/2 cells, and αMEM medium (Life Technologies) containing 20% FBS was used to culture OP9 cells.

### (2) On-feeder differentiation induction culture of mouse iPSC

After culturing mouse EB prepared from mouse iPSC (wild-type or SIRPα-deficient type) on feeder cells OP9 for 7 to 10 days using αMEM containing 20% FBS as a differentiation induction medium, the harvested cells were reseeded on freshly-prepared OP9 feeder. Differentiated cells containing myeloid cells were obtained by culturing for 7 to 10 days in the presence of 55 µM 2-mercaptoethanol and 50 ng/mL GM-CSF.

### (3) Feeder-free differentiation induction culture of human iPSC

Human EB produced from human iPSC (wild-type or SIRPα-deficient type) was cultured on a polystyrene tissue culture plate coated with iMatrix511 and Retronectin (Takara Bio Inc.) and then cultured for 5 to 7 days in differentiation induction medium X-VIVO15 (Lonza) in the presence of 50 ng/mL bFGF, 50 ng/mL BMP-4, 50 ng/mL VEGF, and 50 ng/mL SCF. Thereafter, the cells were cultured for 7 to 10 days in the presence of 50 ng/mL bFGF, 50 ng/mL VEGF, 50 ng/mL SCF, 50 ng/mL Flt-3L, 10 ng/mL TPO, 20 ng/mL IL-3, and 50 ng/mL GM-CSF. Thereafter, the cells were cultured for 7 to 10 days in the presence of 50 ng/mL GM-CSF, 50 ng/mL SCF, and 50 ng/mL Flt-3L to obtain differentiated cells containing myeloid cells. Although possibly affected at times by other growth factors and the like, any two concentrations within the range of 0.2 ng/mL to 200 ng/mL can be used as the upper limit and the lower limit. For example, any two concentrations from 0.2 ng/mL, 2 ng/mL, 5 ng/mL, 10 ng/mL, 20 ng/mL, 50 ng/mL, 100 ng/mL to 200 ng/mL, can be used as the upper limit and the lower limit.

### Example 6: Production of proliferating macrophage-like cell pMAC by introduction of proliferation gene

### (1) Preparation of lentiviral vector

Human c-MYC, BMI1, and MDM2 cDNAs were synthesized by gene synthesis using the sequence information in the database of the National Center for Biotechnology Information (NCBI). A cDNA fragment of each gene was inserted into the lentiviral vector pSL or CSII-EF-MSC-IRES2-Veneus. Using the lipofection method, each of the genes introduced into the lentiviral vector pSL or CSII-EF-MSC-IRES2-Veneus as prepared above, the packaging construct pCAG-HIVgp, and pCMV-VSV-G-RSV-Rev which is a construct of envelope and Rev was introduced into 293T cells, which are packaging cells (virus-producing cells).

Three days after gene transfer into 293T cells, the cell culture medium was collected, filtered through a filter with a pore size of 0.45 µm, and then concentrated with a Lenti-X concentrator (Clontech) to collect virus particles. After suspending the collected recombinant virus particles in a DMEM solution, they were dispensed into cryovials and stored under a -150°C environment.

### (2) Conferring proliferation potency by introducing proliferation gene into iPSC-derived myeloid cell

The myeloid cells prepared in the previous section were cultured in a 48-well tissue culture plate, and in the case of mouse myeloid cells, a suspension of lentivirus expressing c-MYC was added at the same time to infect them. In the case of human myeloid cells, a suspension of lentiviral expressing c-MYC, BMI1, and MDM2 was added at the same time to infect them. From the next day of the gene transfer, the culture scale was expanded while adding the culture medium according to the proliferation of the cells. Using 20% FBS-containing αMEM as a culture medium for myeloid cells after virus infection, the cells after infection were cultured in the presence of 50 ng/mL GM-CSF and 50 ng/mL M-CSF. The transfected cells continued to proliferate for more than 3 months. In each Example of the present specification, cells derived from wild-type iPSC are referred to as pMAC, and cells derived from pluripotent stem cell deficient in the SIRPα gene that inhibits macrophage-like function are referred to as SIRPα-deficient proliferating macrophage-like cells (pMAC-SIRPα-KO). In this Example, SIRPα-deficient iPSC was used as a pluripotent stem cell deficient in the gene that inhibits a macrophage-like function.

### (3) Preparation of mouse biologically derived macrophage

To prepare biologically derived macrophage from C57BL/6 mouse, bone marrow-derived monocyte (BM-derived monocyte) and peritoneal macrophage were collected. Bone marrow-derived monocytes were cultured in RPMI-1640 medium containing 10% fetal bovine serum in a 6-well tissue culture plate in the presence of 50 ng/mL M-CSF for 4 days to induce bone marrow-derived macrophage M0 (BM-derived macrophages M0). Thereafter, bone marrow-derived macrophage M1 (BM-derived macrophages M1) was induced by culturing for 24 hr in the presence of 20 ng/mL interferon-γ (Funakoshi) and 50 ng/mL lipopolysaccharide (FUJIFILM Wako Pure Chemical Corporation). Bone marrow-derived macrophage M2 (BM-derived macrophages M2) was induced by culturing bone marrow-derived macrophage M0 in the presence of 20 ng/mL IL-4 for 24 hr. In addition, 2 mL of thioglycollate medium (Merck) was administered intraperitoneally to the mice, and intraperitoneal macrophages were collected 3 days after administration.

### (4) Gene expression profiling of mouse pMAC

RNAseq analysis, which is a whole transcriptome analysis, (organism species: Mus musculus, reference genome mm10) was performed on pMACs produced from iPS cells and biologically derived macrophages to quantify the expression of transcripts. Total RNA was extracted from each cell using the QIAGEN RNeasy mini kit, and a sequence library was prepared by PCR amplification using primers containing index sequences and using the strand-specific library preparation method (dUTP method) with the NEBNext(R) Poly (A) mRNA Magnetic Isolation Module (model number E7490) and the NEBNext(R) Ultra^{™} II Directional RNA Library Prep Kit (model number E7760). The base sequences of the samples prepared from the libraries were obtained using a next-generation sequencer NovaSeq 6000 (Illumina), and data with a read length of PE150 (150 bp×2 paired-end), data volume of 4 G bases per sample, and read number of 26.7 M reads per sample (13.3 M pairs) were obtained.

Using the acquired data, informatics analysis was performed to perform principal component analysis (PCA). To confirm the quality of the sequence reads, the software FastQC (version 0.11.7) was used to calculate the quality scores (scores indicating the sequence information error rate) of R1 (Read1) and R2 (Read2), and no abnormalities were found (Entirely Normal, quality score> 28). Then, using the software Trimmomatic (version 0.38), the sequence reads were trimmed based on the settings (ILLUMINACLIP 2:30:10, LEADING=20, TRAILING=20, SLIDINGWINDW=4:15, MINLEN=36), and low-quality read ends, adapter sequences, short reads, and the like were removed based on the results of the FastQC quality check to obtain high-quality data. The trimmed sequence reads were mapped to the reference genome using the software HISAT2 (version 2.1.0) to calculate the read mapping rate, and it was confirmed that all samples were over 98%. Then, the raw read counts mapped to known exon regions for each gene were calculated using the software featureCounts (version 1.6.3). In addition, the mapped fragments were counted, and the Fragments Per Kilobase of exon per Million mapped fragments (FPKM) value, Fragments Per Kilobase of Transcript per Million mapped reads upper quartile (FPKM-UQ) value, and Transcripts Per Million (TPM) value were calculated. Using the software stats (version 3.6.1), a principal component analysis of the samples was performed based on the number of raw reads, FPKM value, and TPM value for all detected genes. The index that best reflects the data in the component with the greatest variability in each data was set as principal component (PC) 1 on the horizontal axis, and the index that best reflects the data in the component with the second greatest variability was set as PC2 on the vertical axis to create plot data for each sample (Fig. 1). Mouse pMAC was plotted in the range of PC1 (-100 to -75) and PC2 (-75 to -50), indicating that it exists in a different region from other biologically derived monocytes and macrophages.

### (5) Surface antigen analysis of mouse pMAC

Mouse bone marrow-derived monocyte (BM-derived monocyte) and pMAC were collected by pipetting. Bone marrow-derived macrophage M0 (BM-derived macrophages M0) and bone marrow-derived macrophage M2 (BM-derived macrophages M2) were treated with D-PBS (Nacalai Tesque) containing 2 mM EDTA (Nacalai Tesque), and bone marrow-derived macrophage M1 (BM-derived macrophages M1) was treated with Accutase (Nacalai Tesque) at 37°C for 15 min, and then collected by pipetting. The cells were stained with an anti-CD11b antibody, an anti-F4/80 antibody, an anti-CD16/32 antibody, an anti-CD64 antibody, an anti-I-A/E antibody, an anti-CD206 antibody, an anti-CD163 antibody, an anti-Ly6C antibody, an anti-CD80 antibody, an anti-CD86 antibody, an anti-SiglecF antibody, an anti-SIRPα antibody, an anti-TLR2 antibody, an anti-TLR4 antibody, an anti-CD124 antibody, an anti-CCR1 antibody, an anti-CCR2 antibody, an anti-CCR3 antibody, an anti-CCR4 antibody, an anti-CCR5 antibody, an anti-CXCR2 antibody and an anti-CXCR4 antibody, or an isotype-compatible control antibody. The cells were then washed twice with 2% FBS-containing PBS or PBS. The cells after washing were analyzed using a flow cytometer analyzer (trade name: BD Accuri C6 Flow Cutometer, manufactured by Beckton Dickinson). Fig. 2 shows the results of surface antigen analysis by a flow cytometer after antibody staining. From the results of this analysis, mouse pMAC was found to have properties of both M1 and M2 bone marrow-derived macrophages, which were used as comparison controls.

### (6) Morphology of mouse pMAC

Mouse pMAC and pMAC-SIRPα-KO showed no changes in cell morphology due to SIRPα deficiency, and retained the distorted morphology with protrusions characteristic of myeloid cells shown in Fig. 3.

### Example 7: Production of iPSC-derived chimeric antigen receptor-introduced macrophage-like cell (CAR-pMAC)

### (1) Preparation of lentiviral vector

The tdTomato gene and/or cDNA of a chimeric antigen receptor (CAR) having a GC33scFv domain and a CD3ζ domain were synthesized by gene synthesis, inserted into the lentiviral vector pSL in the same manner as in Example 6 (1), and then viral particles were prepared. Fig. 4 shows the plasmid map and CAR gene expression construct used in the preparation of the lentiviral vector. After suspending the collected recombinant virus particles in a DMEM solution, they were dispensed into cryovials and stored under a -150°C environment.

### (2) Transfer of CAR gene into iPSC-derived pMAC (human, mouse)

pMAC or pMAC-SIRPα-KO which was a human or mouse proliferating macrophage-like cell prepared in Example 6(2) was cultured in a 48-well tissue culture plate, and a suspension of lentivirus expressing GC33 CAR was added to infect same.

From the next day of the gene transfer, the culture scale was expanded while adding the culture medium according to the proliferation of the cells. Using 20% FBS-containing αMEM as a culture medium, the cells were cultured in the presence of 50 ng/mL GM-CSF and 50 ng/mL human M-CSF. As human pMAC, human GM-CSF was used and, as mouse pMAC, mouse GM-CSF was used.

### (4) Influence of genetic manipulation of mouse pMAC on cell proliferation

After gene transfer, human or mouse pMAC, SIRPα-deficient pMAC-SIRPα-KO, CAR-introduced pMAC (CAR-pMAC), and SIRPα-deficient CAR-introduced pMAC (CAR-pMAC-SIRPα-KO) were cultured for two weeks or more, and cell proliferation induced by GM-CSF and M-CSF was examined by MTT assay. pMAC, pMAC-SIRPα-KO, CAR-pMAC, and CAR-pMAC-SIRPα-KO were collected, seeded in a 96-well tissue culture plate (3×10³ cells/well), and cultured. Then, the proliferation rate was compared between a culture medium containing GM-CSF and M-CSF and a culture medium not containing GM-CSF and M-CSF. 0.5 mg/mL of 3-(4,5-dimethyl-2-thiazolyl)-2,5-diphenyl-2H-tetrazolium bromide (MTT) reagent (Merck) was added every 24 hr from immediately after the start of culture in a 96-well tissue culture plate, and metabolized formazan was measured 4 hr later by absorbance at a wavelength of 595 nm. The amount of metabolized formazan is proportional to the number of cells at the time of addition of the MTT reagent, that is, the rate of cell proliferation.

Fig. 5 shows the measurement results of the MTT assay of various mouse pMACs. pMAC proliferation required about 50 ng/mL of GM-CSF or M-CSF in the culture medium. In addition, the combined use of GM-CSF and M-CSF was effective in promoting proliferation. An influence of deletion of SIRPα gene which was performed to prepare pMAC and introduction of CAR gene on cell proliferation was not observed, indicating that CAR-pMAC has cell proliferation ability similar to that of pMAC.

### (5) Cell surface antigen analysis of mouse CAR-pMAC

The expression of tdTomato, GC33ζCAR, SIRPα in mouse pMAC, CAR-pMAC, pMAC-SIRPα-KO, CAR-pMAC-SIRPα-KO was examined using a flow cytometer. A fluorescent protein, tdTomato, was expressed via the T2A sequence in the nucleic acid sequence at the 3'-terminal side of the GC33ζCAR cDNA incorporated into the lentiviral vector. In addition, FITC-labeled human glypican 3 (hGPC3), which is the target antigen of CAR, was added to the cell sample. This makes it possible to analyze the expression of CAR by a flow cytometer using the fluorescence of tdTomato and hGPC3-FITC as an index. Fig. 6 shows the results of examining the expression of GC33ζCAR in pMAC, CAR-pMAC, pMAC-SIRPα-KO, and CAR-pMAC-SIRPα-KO using a flow cytometer. Compared to pMAC and pMAC-SIRPα-KO, CAR-pMAC and CAR-pMAC-SIRPα-KO show antigen-specific binding to hGPC. Compared to pMAC and CAR-pMAC, pMAC-SIRPα-KO and CAR-pMAC-SIRPα-KO show reduced expression of SIRPα. From each cytogram, it can be seen that the various cells prepared are a single cell population, contain almost no impurity cells, and are highly pure cells.

### (6) Cytokine production by mouse pMAC

Macrophages present in the body are divided into functional phenotypes, M1 and M2, depending on intercellular interactions in the microenvironment of cancer tissue. To compare with the phenotype of biologically-derived macrophages, cytokines produced by mouse pMAC, pMAC-SIRPα-KO, CAR-pMAC, and CAR-pMAC-SIRPα-KO were evaluated.

Mouse pMAC, pMAC-SIRPα-KO, CAR-pMAC, CAR-pMAC-SIRPα-KO, and mouse bone marrow-derived macrophages (2×10⁵ cells) were seeded into a 48-well tissue culture plate and cultured for 48 hr in the presence of 20 ng/mL interferon-γ (BioLegend) and 50 ng/mL LPS (FUJIFILM Wako Pure Chemical Corporation) to induce the M1 type. As the mouse bone marrow-derived macrophage, mouse bone marrow-derived macrophage M0 prepared in Example 6 (3) was used. Similarly, M2 type was induced by culturing for 48 hr in the presence of 20 ng/mL IL-4 (BioLegend). Using the obtained culture supernatant, the production of IL-6, TNFα, IL-12, and IL-10, which are known as M1 or M2 type markers, was quantified (Fig. 7). It was found that pMAC induced the production of IL-6, TNFα, and IL-10 by M1 stimulation, regardless of the presence or absence of SIRPα or CAR expression, whereas IL-12 was not produced.

### (7) Cell migration ability of mouse CAR-pMAC-SIRPα-KO

To examine the cell migration ability of mouse CAR-pMAC-SIRPα-KO, 160 µL of 10% FCS-containing RPMI-1640 medium containing cancer cell culture supernatant or recombinant chemokine was added to the lower chamber of a 16-well Boyden chamber type (transwell type) CIM-Plate 16 (Agilent) coated with bovine fibronectin (FUJIFILM Wako Pure Chemical Corporation), and mouse CAR-pMAC-SIRPα-KO (3×10⁵ cells) was seeded in the upper chamber in 20% FCS-containing αMEM containing GM-CSF and M-CSF and cultured at 37°C and 5% CO₂. The CAR-pMAC in the upper chamber passes through the pores of the transwell and is adsorbed to the electrode sensor on the back of the membrane by the attractant factor in the lower chamber, increasing the electrical resistance. The cell migration of mouse CAR-pMAC can be evaluated by measuring this electrical resistance using an xCELLigence RTCA DP system (Agilent).

To prepare mouse cancer cell culture supernatant, mouse MC38, LL/2, 4T1, and CT26 cells (1×10⁶ cells each) were seeded in a 6-well tissue culture plate. The next day, the medium was replaced with 2 mL of fresh RPMI-1640 medium containing 10% FCS, and the cells were further cultured for 24 hr to prepare the culture supernatant. As normal cells, T cells and B cells (1×10⁶ cells) isolated from the spleen of a c57BL/6 mouse were used to prepare culture supernatant.

The results of measuring the cell migration activity of CAR-pMAC-SIRPα-KO against the culture supernatant of cancer cells are shown in the upper row of Fig. 8. The electrical resistance Cell Index value increased against the culture supernatant of mouse cancer cells MC38, LL/2, CT26, and 4T1, indicating that mouse CAR-pMAC-SIRPα-KO has migration activity (Fig. 8, upper row, left). When the culture supernatant of normal mouse spleen-derived B and T cells was used as a negative control, the Cell Index value was low, similar to that of 10% FCS-containing RPMI1640 (medium) containing no attractant factor, and CAR-pMAC did not show cell migration activity (Fig. 8, upper row, center). In addition, when CAR-pMAC-SIRPα-KO was cultured in the presence of 200 ng/mL of cytochalasin D (FUJIFILM Wako Pure Chemical Corporation) or Pertussis Toxin (FUJIFILM Wako Pure Chemical Corporation), which are known to be inhibitors of cell migration, the increase in the Cell Index value in the culture supernatant of cancer cells MC38 was partially reduced by cytochalasin D (CytD) and completely suppressed by Pertussis Toxin (PTX) (Fig. 8, upper row, right). These results indicate that CAR-pMAC-SIRPα-KO has cell migration ability in response to attractant factors produced by multiple cancer cells.

Then, in order to examine the cell migration ability of CAR-pMAC-SIRPα-KO in response to recombinant chemokines, mouse recombinant chemokines CCL2, CCL3, CCL4, CCL5, CCL7, CCL8, CX3CL1, CXCL1, CXCL2, CXCL5, CXCL12SDF-1α, and CXCL12SDF-1β (all manufactured by BioLegend) were used as candidate attractant factors, all at a concentration of 100 ng/mL. The results are shown in the lower row of Fig. 8.

As a result of evaluation of cell migration ability of mouse CAR-pMAC-SIRPα-KO against mouse recombinant chemokines, it is clear that it responds to CCL3, CCL4, and CCL7 (Fig. 8, lower row, left and center).

### (8) Cytokine production by mouse pMAC co-cultured with cancer cells

Cytokine production was evaluated by co-culture of cancer antigen hGPC3-expressing or non-expressing cancer cells with mouse pMAC, pMAC-SIRPα-KO, CAR-pMAC, and CAR-pMAC-SIRPα-KO. Mouse cancer cell hGPC3-introduced or non-expressing 4T1, hGPC3-introduced or non-expressing CT26, hGPC3-introduced or non-expressing LL/2, hGPC3-introduced or non-expressing MC38 (all 5×10⁴ cells), and mouse pMAC, pMAC-SIRPα-KO, CAR-pMAC, CAR-pMAC-SIRPα-KO (all 2×10⁵ cells) were seeded in 48-well tissue culture plates using RPMI-1640 containing 10% FCS and cultured at 37°C, 5% CO₂ for 48 hr. Using the obtained culture supernatant, the production of IL-6 (Fig. 9-1), TNFα (Fig. 9-2), IL-10 (Fig. 9-3), IL-12 (Fig. 9-4) was quantified. It was found that the production of IL-6 and TNFα was induced by co-culture of 4T1-GPC3, CT26-GPC3, LL/2-GPC3, and MC38-GPC3 with CAR-pMAC-SIRPα-KO. No difference in the amount of IL-10 produced was observed under the conditions tested, and IL-12 was not detected.

### (9) Antigen-specific cancer cell phagocytosis by mouse CAR-pMAC

Mouse pMAC, CAR-pMAC, pMAC-SIRPα-KO, and CAR-pMAC-SIRPα-KO (3×10⁴ cells/well) were seeded as effector cells in a 96-well tissue culture plate, and co-cultured for 2 days with the target cells: mouse cancer cell hGPC3-introduces or non-expressing 4T1, hGPC3-introduced or non-expressing CT26, hGPC3-introduced or non-expressing LL/2, and hGPC3-introduced or non-expressing MC38, each transfected with luciferase gene (all 3×10³ cells/well), using RPMI-1640 containing 10% FBS. To evaluate the phagocytosis of cancer cells specific to the CAR target antigen, cancer cells introduced with the cancer antigen hGPC3 were used. Fig. 10 shows the results of investigating the survival of target cells by quantifying the bioluminescence obtained by adding the luciferase substrate Bright-Glo Luciferase Assay (Promega) to the 96-well tissue culture plate after co-culture. The cell killing effect of target cells was about 20% in co-culture with pMAC, CAR-pMAC, and pMAC-SIRPα-KO, but it was improved to about 60% in co-culture with CAR-pMAC-SIRPα-KO. In addition, the cell killing effect of CAR-pMAC-SIRPα-KO was not observed in hGPC3 non-expressing target cells, indicating that CAR-pMAC-SIRPα-KO has the ability to phagocytose cancer cells specific to the target antigen.

Mouse pMAC or CAR-pMAC-SIRPα-KO that expresses tdTomato (5×10⁴ cells/well) and hGPC3-introduced colorectal cancer cell MC38 that expresses Venus (5×10³ cells/well) were co-cultured in a 48-well tissue culture plate using RPMI-1640 medium containing 10% FBS, and fluorescence observation was performed 2 and 4 days later, using a fluorescence microscope (Keyence BZ-X810). The results are shown in Fig. 11. Cancer cells are detected by green fluorescence (excitation wavelength 470 nm, absorption wavelength 525 nm) derived from the fluorescent protein Venus, and pMAC or CAR-pMAC-SIRPα-KO is detected by red fluorescence (excitation wavelength 545 nm, absorption wavelength 605 nm) derived from the fluorescent protein tdTomato. A bar graph plotting the area of the region where the green fluorescent signal of cancer cells and the red fluorescent signal of pMAC or CAR-pMAC-SIRPα-KO are overlapped (Fig. 11, lower left). A bar graph plotting the area of the region where the green fluorescent signal of cancer cells is detected (Fig. 11, lower right). It was found that cancer cells co-cultured with pMAC proliferated, whereas cancer cells co-cultured with CAR-pMAC-SIRPα-KO were surrounded and phagocytosed by CAR-pMAC-SIRPα-KO, and eliminated.

### (10) Cancer cell cytotoxicity of mouse CAR-pMAC

To examine the cancer cell cytotoxicity of mouse CAR-pMAC, cancer cells and CAR-pMAC-SIRPα-KO were co-cultured in a 16-well plate E-Plate 16 (Agilent) at 37°C, 5% CO₂. Cancer cells that adhere to the electrode sensor on the plate by preculture are phagocytosed by co-culture with CAR-pMAC-SIRPα-KO, and show weakened cell adhesion and decreased electrical resistance. The cancer cell cytotoxicity of CAR-pMAC-SIRPα-KO can be evaluated by measuring this electrical resistance using an xCELLigence RTCA DP system (Agilent).

Target mouse cancer cell hGPC3-introduced or non-expressing CT26 (5×10³ cells), hGPC3-introduced or non-expressing MC38 (1×10⁴ cells), hGPC3-introduced or non-expressing LL/2 (1×10⁴ cells), hGPC3-introduced or non-expressing 4T1 (5×10³ cells) were seeded on an E-Plate 16 coated with iMatrix-511 and cultured at 37°C, 5% CO₂ for 24 hr, after which they were co-cultured with 1-fold, 5-fold, or 10-fold amount of effector cancer cells targeting mouse CAR-pMAC-SIRPα-KO. The cancer cell cytotoxicity (% Cytolysis) of CAR-pMAC-SIRPα-KO at effector:target ratio (E:T ratio) of 1:1, 5:1, and 10:1, 6 hr after the start of co-culture (T=30 hr) showed more superior activity with a higher cancer antigen hGPC3 expression and E:T ratio, regardless of which cancer cell was targeted (Fig. 12-1). Time course changes of the adhesion signal (Normalized Cell Index value; CI value) indicating cancer cell survival when cancer cells are co-cultured with CAR-pMAC-SIRPα-KO, and the cytotoxic activity (% Cytolysis) of cancer cells standardized to that of cancer cells cultured alone are shown in Fig. 12-2 and Fig. 12-3. It can be seen that the CI value of cancer cells decreases over time and the % Cytolysis increases depending on the expression of the cancer antigen hGPC3 and the number of CAR-pMAC-SIRPα-KO cells (E:T ratio).

### (11) Examination of importance of SIRPα gene deficiency or GC33-ζ-CAR introduction in cytotoxicity by mouse CAR-pMAC

To investigate the importance of SIRPα gene deficiency or GC33-ζ-CAR introduction in the cancer cell cytotoxicity of CAR-pMAC, human or mouse pMAC, pMAC-SIRPα-KO, CAR-pMAC, and CAR-pMAC-SIRPα-KO were used as effector cells to evaluate the cancer cell cytotoxicity at an E:T ratio of 10:1 using the xCELLigence RTCA DP system.

Mouse cancer cells hGPC-introduced or non-expressing CT26 (5×10³ cells) were seeded on an E-Plate 16 coated with iMatrix-511 and cultured at 37°C for 24 hr. Then, effector cells mouse pMAC, pMAC-SIRPα-KO, CAR-pMAC, CAR-pMAC-SIRPα-KO (5×10⁴ cells) were added and co-cultured. It can be seen that antigen-specific cytotoxic activity (% Cytolysis) is improved by SIRPα deficiency and GC33-ζ-CAR introduction compared to hGPC3-introduced CT26 (Fig. 13).

### (12) Time-lapse observation of cancer cell phagocytosis by mouse CAR-pMAC

To evaluate the phagocytosis of cancer cells by CAR-pMAC, cancer cell MC38-GPC3-Luc-Venus (5×10⁵ cells/mL) labeled with the pH-sensitive red fluorescent dye pHrodo Deep Red (Thermo Fisher Scientific) at 20°C for 2 hr was co-cultured with 1 mL each of pMAC, pMAC-SIRPα-KO, CAR-pMAC, or CAR-pMAC-SIRPα-KO (each 5×10⁶ cells/mL), using 10% FCS-containing RPMI-1640 medium and 12-well tissue culture plate. A 8-hour time-lapse observation was performed by photographing four specific fields 50 times every 10 minutes using a fluorescence microscope (Keyence BZ-X810). The results are shown in Fig. 14. When pHrodo-labeled cancer cells are phagocytosed and taken into pMAC cells, an image is obtained in which red fluorescence (excitation wavelength 620 nm, absorption wavelength 700 nm) derived from pHrodo is detected in response to a drop in pH. Image analysis (time-series cell counting) was performed using the BZ-X810 analyzer analysis application to measure changes in the number of phagocytic cells over time. It can be seen that CAR-pMAC-SIRPα-KO has improved phagocytic ability compared to pMAC, pMAC-SIRPα-KO, and CAR-pMAC.

### (13) Pharmacokinetics of mouse CAR-pMAC in tumor-bearing mice

A colorectal cancer peritoneal dissemination model (n=6) was created by transplanting hGPC3-introduced MC38 (5×10⁵ cells) into the peritoneal cavity of C57BL6/N mice. CAR-pMAC-SIRPα-KO (1×10⁷ cells) was then administered intraperitoneally to the mice. Using 100 µg of genomic DNA extracted from each organ removed before treatment, and 1 and 3 days after treatment as a template, a custom-made PrimeTime real-time PCR probe (Integrated DNA Technologies) that specifically recognizes CAR (GC33-ζ) was used to measure the amount of CAR-pMAC present in the lungs, liver, kidneys, heart, spleen, bone marrow, tumor, and peripheral blood with a real-time PCR device 7500Fast (Thermo Fisher Scientific) (Fig. 15). No CAR signals were detected in the mice before treatment, and one day after intraperitoneal administration, CAR-pMAC-SIRPα-KO was detected in the lungs, liver, kidneys, heart, spleen and tumor, but not in bone marrow or peripheral blood. Three days after intraperitoneal administration, the number of mice in which CAR-pMAC-SIRPα-KO was detected in normal tissues of the lungs, kidneys, heart and spleen had decreased, but CAR-pMAC was detected in tumor tissue in five out of six mice, indicating that it was present in large amounts.

### (14) Antitumor effect of CAR-pMAC in vivo

A colorectal cancer peritoneal dissemination model was created by transplanting hGPC3-introduced MC38 (5×10⁵ cells) into the peritoneal cavity of C57BL6/N mice. CAR-pMAC-SIRPα-KO (3×10⁶ cells) irradiated with 10 Gy was administered intraperitoneally a total of six times and the therapeutic effect was evaluated. Mice that lost 20% or more of their body weight or whose body temperature fell 30°C or lower were determined to be dead, and a survival curve was created. Cell viability of hGPC3-introduced MC38 in vivo was measured by biochemiluminescence with an IVIS imaging system (PerkinElmer) every 3 to 4 days. Fig. 16 shows the photographed images of in vivo imaging and the time course changes of the total flux of luciferase luminescence. The no treatment group, which did not receive CAR-pMAC-SIRPα-KO, showed remarkable tumor growth after cancer cell transplantation. In contrast, CAR-pMAC-SIRPα-KO remarkably suppressed tumor growth and significantly extended survival.

### Example 8 Functional analysis of human CAR-pMAC

### (1) Preparation of human biological macrophage and iPS cell-derived macrophage

Normal human donor blood collection-derived CD14 monocytes (Lonza) were seeded (2×10⁶ cells/well) in 6-well tissue culture plates in the presence of 50 ng/mL human M-CSF using ImmunoCult-SF Macrophage Differentiation Medium (Stemcell Technologies) and cultured for 4 days to induce CD14 monocyte-derived macrophage M0 (Human CD14-derived macrophages M0). Thereafter, the cells were cultured for 2 days in the presence of 50 ng/mL lipopolysaccharide, 50 ng/mL human interferon γ to induce CD14 monocyte-derived macrophage M1 (Human CD14-derived macrophages M1). In addition, CD14 monocyte-derived macrophage M0 (Human CD14-derived macrophages M0) was cultured for 2 days in the presence of 50 ng/mL IL-4 (BioLegend) to induce CD14 monocyte-derived macrophage M2 (Human CD14-derived macrophages M2).

Human iPS cell-derived macrophages were induced by culturing the differentiated cells including myeloid cells obtained in Example 5(3) for 7 to 14 days in the presence of 50 ng/mL M-CSF and 20 ng/mL IL-3.

### (2) Gene expression profiling of human pMAC

RNAseq analysis, which is a whole transcriptome analysis, (organism species: Human, reference genome hg38) was performed on pMACs produced from iPS cells, biologically derived macrophages, and iPSC-derived macrophages to quantify the expression of transcripts. Total RNA was extracted from each cell using the QIAGEN RNeasy mini kit, and a sequence library was prepared by PCR amplification using primers containing index sequences and using the strand-specific library preparation method (dUTP method) with the NEBNext(R) Poly (A) mRNA Magnetic Isolation Module (model number E7490) and the NEBNext(R) Ultra^{™} II Directional RNA Library Prep Kit (model number E7760). The base sequences of the samples prepared from the libraries were obtained using a next-generation sequencer NovaSeq 6000 (Illumina), and data with a read length of PE150 (150 bp×2 paired-end), data volume of 4 G bases per sample, and read number of 26.7 M reads per sample (13.3 M pairs) were obtained.

Using the acquired data, informatics analysis was performed to perform principal component analysis (PCA). To confirm the quality of the sequence reads, the software FastQC (version 0.11.7) was used to calculate the quality scores (scores indicating the sequence information error rate) of R1 (Read1) and R2 (Read2), and no abnormalities were found (Entirely Normal, quality score> 28). Then, using the software Trimmomatic (version 0.38), the sequence reads were trimmed based on the settings (ILLUMINACLIP 2:30:10, LEADING=20, TRAILING=20, SLIDINGWINDW=4:15, MINLEN=36), and low-quality read ends, adapter sequences, short reads, and the like were removed based on the results of the FastQC quality check to obtain high-quality data. The trimmed sequence reads were mapped to the reference genome using the software HISAT2 (version 2.1.0) to calculate the read mapping rate, and it was confirmed that all samples were over 98%. Then, the raw read counts mapped to known exon regions for each gene were calculated using the software featureCounts (version 1.6.3). In addition, the mapped fragments were counted, and the Fragments Per Kilobase of exon per Million mapped fragments (FPKM) value, Fragments Per Kilobase of Transcript per Million mapped reads upper quartile (FPKM-UQ) value, and Transcripts Per Million (TPM) value were calculated. Using the software stats (version 3.6.1), a principal component analysis of the samples was performed based on the number of raw reads, FPKM value, and TPM value for all detected genes. The index that best reflects the data in the component with the greatest variability in each data was set as principal component (PC) 1 on the horizontal axis, and the index that best reflects the data in the component with the second greatest variability was set as PC2 on the vertical axis to create plot data for each sample (Fig. 17). Human pMAC was plotted in the range of PC1 (150 to 200) and PC2 (-20 to -40), indicating that it exists in a different region from other biologically derived CD14 positive cell-derived monocyte, CD14 positive cell-derived macrophages (M0, M1, M2 types), and further, iPS cell-derived myeloid cells, and iPS cell-derived macrophages.

### (3) Surface antigen analysis of human iPSC-pMAC

Human pMAC, pMAC-SIRPα-KO, CAR-pMAC-SIRPα-KO, blood collection-derived CD14 positive monocyte (CD14-derived monocyte), iPSC-derived myeloid cell (iPSC-derived Myeloid cells), and iPSC-derived macrophage (iPSC-macrophages) were collected by pipetting. CD14 monocyte-derived macrophage M0 (CD14-derived macrophages M0) and CD14 monocyte-derived macrophage M2 (CD14-derived macrophages M2) were treated with D-PBS (Nacalai Tesque) containing 2 mM EDTA (Nacalai Tesque), and CD14 monocyte-derived macrophage M1 (CD14-derived macrophages M1) was treated with Accutase (Nacalai Tesque) at 37°C for 15 min, and then collected by pipetting.

The cells were stained with an anti-HLA-ABC antibody, an anti-HLA-DR antibody, an anti-CD40 antibody, an anti-CD80 antibody, an anti-CD83 antibody, an anti-CD86 antibody, an anti-CD68 antibody, an anti-CD163 antibody, an anti-CD206 antibody, an anti-SIRPα antibody, an anti-CCR1 antibody, an anti-CCR2 antibody, an anti-CCR3 antibody, an anti-CCR4 antibody, an anti-CCR5 antibody, an anti-CCR7 antibody, an anti-CXCR2 antibody, an anti-CXCR4 antibody and an anti-CD14 antibody, or an isotype-compatible control antibody. The cells were then washed twice with 2% FBS-containing PBS or PBS. The cells after washing were analyzed using a flow cytometer analyzer (trade name: BD Accuri C6 Flow Cutometer, manufactured by Beckton Dickinson). Fig. 18 shows the results of surface antigen analysis by a flow cytometer after antibody staining. From the results of this analysis, it was found that human pMAC (pMAC, pMAC-SIRPα-KO, CAR-pMAC-SIRPα-KO) are negative for CD83 and CD14, which are expressed in CD14-positive cell-derived macrophages (M0, M1, and M2) and iPSC-derived macrophages.

### (4) Morphology of human pMAC

Phase contrast microscopic images of human CD14 positive monocyte, iPSC-derived myeloid cell, pMAC, CD14 positive cell-derived macrophage (unstimulated M0, M1 and M2 stimulated conditions) are shown in Fig. 19-1, and phase contrast microscopic images of iPS cell-derived macrophage, pMAC, pMAC-SIRPα-KO, CAR-pMAC, CAR-pMAC-SIRPα-KO (unstimulated M0, M1 and M2 stimulated conditions) are shown in Fig. 19-2. pMAC, pMAC-SIRPα-KO, CAR-pMAC, CAR-pMAC-SIRPα-KO showed no changes in cell morphology due to SIRPα deficiency or CAR introduction, and it can be seen that they have the distorted morphology with protrusions exhibited by blood collection-derived CD14 positive cell-derived macrophages shown in Fig. 19-1.

### (5) Cell proliferation of human pMAC

After gene transfer, human pMAC, pMAC-SIRPα-KO, and iPS cell-derived macrophage as a comparison control were cultured for two weeks or more, and cell proliferation induced by GM-CSF and M-CSF was examined by MTT assay. pMAC and pMAC-SIRPα-KO were collected, seeded in a 96-well tissue culture plate (3×10³ cells/well), and cultured. Then, the proliferation rate was compared between a culture medium containing GM-CSF and M-CSF and a culture medium not containing GM-CSF and M-CSF.

Fig. 20 shows the measurement results of the MTT assay. Proliferation of human pMAC and pMAC-SIRPα-KO required about 50 ng/mL of GM-CSF or M-CSF in the culture medium. In addition, the combined use of GM-CSF and M-CSF was effective in promoting proliferation. On the other hand, cytokine-dependent cell proliferation was not observed in iPS cell-derived macrophages, indicating that pMAC has high cell proliferation ability.

### (6) Cell surface antigen analysis of human CAR-pMAC

The expression of tdTomato, GC33ζCAR, SIRPα in human pMAC, pMAC-SIRPα-KO, CAR-pMAC, CAR-pMAC-SIRPα-KO was examined using a flow cytometer. A fluorescent protein, tdTomato, was expressed via the T2A sequence in the nucleic acid sequence at the 3'-terminal side of the GC33ζCAR cDNA incorporated into the lentiviral vector. In addition, FITC-labeled human glypican 3 (hGPC3), which is the target antigen of CAR, was added to the cell sample. This makes it possible to analyze the expression of CAR by a flow cytometer using the fluorescence of tdTomato and hGPC3-FITC as an index. Fig. 21 shows the results of examining the expression of GC33ζCAR in human pMAC, pMAC-SIRPα-KO, CAR-pMAC, and CAR-pMAC-SIRPα-KO using a flow cytometer. Compared to pMAC and pMAC-SIRPα-KO, CAR-pMAC and CAR-pMAC-SIRPα-KO show antigen-specific binding to hGPC. Compared to pMAC and CAR-pMAC, pMAC-SIRPα-KO and CAR-pMAC-SIRPα-KO show reduced expression of SIRPα. From each cytogram, it can be seen that the various cells prepared are a single cell population, contain almost no impurity cells, and are highly pure cells.

### (7) Cytokine production by human CAR-pMAC

Human CD14 monocyte-derived macrophage M0 type and human iPS cell-derived macrophage produced in Example 8(1), human pMAC and pMAC-SIRPα-KO produced in Example 6(2), and CAR-pMAC, CAR-pMAC-SIRPα-KO (2×10⁵ cells) produced in Example 7(2) were seeded into a 48-well tissue culture plate and cultured for 48 hr in the presence of 50 ng/mL interferon-γ (BioLegend) and 50 ng/mL LPS (FUJIFILM Wako Pure Chemical Corporation) to induce the M1 type. Similarly, M2 type was induced by culturing for 48 hr in the presence of 50 ng/mL IL-4 (BioLegend). Using the obtained culture supernatant, the production of IL-6, TNFα, IL-12, and IL-10, which are known as M1 or M2 type markers, was quantified (Fig. 22). It can be seen that the production of IL-6, TNFα, and IL-10 is induced in pMAC, pMAC-SIRPα-KO, CAR-pMAC, CAR-pMAC-SIRPα-KO by stimulation with interferon γ and LPS. On the other hand, production of IL-12 was not detected.

### (8) Cell migration ability of human CAR-pMAC

To examine the cell migration ability of human CAR-pMAC-SIRPα-KO, 160 µL of 10% FCS-containing RPMI-1640 medium containing cancer cell culture supernatant or recombinant chemokine was added to the lower chamber of a 16-well Boyden chamber type (transwell type) CIM-Plate 16 (Agilent) coated with bovine fibronectin (FUJIFILM Wako Pure Chemical Corporation), and human CAR-pMAC-SIRPα-KO (3×10⁵ cells) was seeded in the upper chamber in 20% FCS-containing αMEM containing GM-CSF and M-CSF and cultured at 37°C and 5% CO₂. The CAR-pMAC in the upper chamber passes through the pores of the transwell and is adsorbed to the electrode sensor on the back of the membrane by the attractant factor in the lower chamber, increasing the electrical resistance. The cell migration of human CAR-pMAC can be evaluated by measuring this electrical resistance using an xCELLigence RTCA DP system (Agilent).

To prepare human cancer cell culture supernatant, human HepG2, JHH-7, KOC7c cells (2×10⁶ cells each) and SK-Hep1 (5×10⁵ cells) were seeded in a 6-well tissue culture plate. The next day, the medium was replaced with 2 mL of fresh RPMI-1640 medium containing 10% FCS, and the cells were further cultured for 24 hr to prepare the culture supernatant. As normal cells, HEK293 (5×10⁵ cells) were used to prepare culture supernatant.

The results of measuring the cell migration activity of human CAR-pMAC-SIRPα-KO against the culture supernatant of cancer cells are shown in Fig. 23. Cell Index value increased against the culture supernatant of human cancer cells HepG2, SK-Hep1, and JHH-7, indicating that human CAR-pMAC-SIRPα-KO has migration activity. The migration ability towards the cancer cell KOC7c was low, and the Cell Index value was similar to that of the normal cell HEK293 (Fig. 23, upper row, left). In addition, when CAR-pMAC-SIRPα-KO was cultured in the presence of 200 ng/mL of cytochalasin D (FUJIFILM Wako Pure Chemical Corporation) or Pertussis Toxin (FUJIFILM Wako Pure Chemical Corporation), which are known to be inhibitors of cell migration, the increase in the Cell Index value in the culture supernatant of cancer cells MC38 was partially reduced by cytochalasin D (CytD) and completely suppressed by Pertussis Toxin (PTX) (Fig. 23, upper row, center). When the cell migration ability of human iPS-macrophage toward the culture supernatant of cancer cells was measured, the cancer cells HepG2, SK-Hep1, and KOC7c all showed CI values similar to those of the negative control Medium and normal cells HEK293 (Fig. 23, upper row, right). These results indicate that CAR-pMAC-SIRPα-KO has cell migration ability higher than that of iPS-macrophage, in response to attractant factors produced by multiple cancer cells.

Then, in order to examine the cell migration ability of CAR-pMAC-SIRPα-KO in response to recombinant chemokines, human recombinant chemokines CCL2 (BioLegend), CCL3 (BioLegend), CCL3LI (BioLegend), CCL4 (BioLegend), CCL5 (BioLegend), CCL7 (R&D Systems), CCL8 (BioLegend), CCL13 (R&D Systems), CCL14 (Novus Biologicals), CCL15 (R&D Systems), CCL23 (BioLegend), CX3CL1 (R&D Systems), CXCL1 (BioLegend), CXCL2 (BioLegend), CXCL5 (BioLegend), CXCL12SDF-1α (BioLegend), and CXCL12SDF-1β (BioLegend) were used as candidate attractant factors, all at a concentration of 100 ng/mL.

As a result of evaluation of cell migration ability of human CAR-pMAC-SIRPα-KO against human recombinant chemokines, it is clear that it responds to CCL2, CCL3LI, CCL5, CCL7, CCL23 and SDF-1α (CXCL12) (Fig. 23, second row and third row from the top). Furthermore, cell migration of CAR-pMAC and CAR-pMAC-SIRPα-KO toward culture supernatant of cancer cells HepG2 was examined (Fig. 23, bottom). It can be seen that the lack of SIRPα improves the cell migration ability of CAR-pMAC-SIRPα-KO compared to CAR-pMAC derived from wild-type iPS cells.

### (9) Cytokine production by human CAR-pMAC (co-culture with cancer cells)

Human pMAC, pMAC-SIRPα-KO, CAR-pMAC, CAR-pMAC-SIRPα-KO (all 2×10⁵ cells) were seeded in a 48-well tissue culture plate using RPMI-1640 containing 10% FCS and cultured at 37°C, 5% CO₂ for 48 hr. Using the obtained culture supernatant, the production of IL-6, TNFα, IL-10, IL-12 was quantified (Fig. 24-1). In addition, human pMAC, pMAC-SIRPα-KO, CAR-pMAC, and CAR-pMAC-SIRPα-KO were co-cultured with cultured cancer antigen hGPC3-expressing or non-expressing cancer cells and cytokine production was evaluated. Using culture supernatant obtained by coculturing human cancer cell hGPC3-introduced or non-expressing SK-Hep1, hGPC3-expressing or -deficient JHH-7 (both 5×10⁴ cells) with human pMAC, pMAC-SIRPα-KO, CAR-pMAC, CAR-pMAC-SIRPα-KO (all 2×10⁵ cells), the production of IL-6 (Fig. 24-2), TNFα (Fig. 24-3), IL-10 (Fig. 24-2), IL-12 (Fig. 24-3) was quantified.

It can be seen that human CAR-pMAC, CAR-pMAC-SIRPα-KO produce IL-6, TNFα, and IL-10 upon introduction of CAR. It can be seen that antigen-specific cytokine production is not induced by co-culture with human cancer cell SK-Hep1 (hGPC3-introduced or non-expressing) and JHH-7 (hGPC3-expressing or - deficient). IL-12 was not detected.

### (10) Cancer cell cytotoxicity by human CAR-pMAC

Human pMAC, CAR-pMAC, pMAC-SIRPα-KO, CAR-pMAC-SIRPα-KO (3×10⁴ cells/well) were seeded as effector cells in a 96-well tissue culture plate, and co-cultured for 2 days with the target cells: cancer cells JHH7-Luc, SK-Hep1-GPC3-Luc-Venus, KOC7c-GPC3-Luc, each transfected with luciferase gene (all 3×10³ cells/well), using RPMI-1640 containing 10% FBS. To evaluate the phagocytosis of cancer cells specific to the CAR target antigen, cancer antigen hGPC3-endogenously expressing or -transfected cancer cells were used. Fig. 25 shows the results of investigating the survival of target cells by quantifying the bioluminescence obtained by adding the luciferase substrate Bright-Glo Luciferase Assay (Promega) to the 96-well tissue culture plate after co-culture. The cell killing effect of target cells was about 20 to 40% in co-culture with pMAC, CAR-pMAC, and pMAC-SIRPα-KO, but it was improved to about 60 to 90% in co-culture with CAR-pMAC-SIRPα-KO. In addition, the cell killing effect of CAR-pMAC-SIRPα-KO was not observed in hGPC3 non-expressing target cell SK-Hep1-mock-Luc-V, indicating that CAR-pMAC-SIRPα-KO has the ability to phagocytose cancer cells specific to the target antigen.

### (11) Cancer cell cytotoxicity of human CAR-pMAC-SIRPα-KO (real-time measurement)

Using human CAR-pMAC-SIRPα-KO as an effector cell, the cytotoxicity against human cancer cells was evaluated using the xCELLigence RTCA DP system (Fig. 26-1 and Fig. 26-2). hGPC3-introduced or non-expressing SK-Hep1 (5×10⁴ cells) were seeded on E-Plate16 and cultured at 37°C, 5% CO₂ for 24 hr, after which human CAR-pMAC-SIRPα-KO was added at E:T ratios of 1:1, 5:1, and 10:1 and the cells were co-cultured. It can be seen that human CAR-pMAC-SIRPα-KO shows superior cytotoxic activity as the hGPC3 expression and E:T ratio increase. Time course changes of the adhesion signal (Cell Index value; CI value) indicating cancer cell survival when cancer cells are co-cultured with CAR-pMAC-STIRPα-KO, and the cytotoxic activity (% Cytolysis) of cancer cells standardized to that of cancer cells cultured alone are shown in Fig. 26-2. It can be seen that the CI value of cancer cells decreases over time and the % Cytolysis increases depending on the expression of the cancer antigen hGPC3 and the number of CAR-pMAC-SIRPα-KO cells (E:T ratio).

### (12) Examination of importance of SIRPα gene deficiency or GC33-ζ-CAR introduction in cytotoxicity by CAR-pMAC

To investigate the importance of SIRPα gene deficiency or GC33-ζ-CAR introduction in the cancer cell cytotoxicity of CAR-pMAC, human pMAC, pMAC-SIRPα-KO, CAR-pMAC, and CAR-pMAC-SIRPα-KO were used as effector cells to evaluate the cancer cell cytotoxicity at an E:T ratio of 10:1 using the xCELLigence RTCA DP system.

Human cancer cells hGPC-introduced or non-expressing SK-Hep1 (5×10⁴ cells) were seeded on an E-Plate 16 and cultured at 37°C for 24 hr. Then, effector cells human pMAC, pMAC-SIRPα-KO, CAR-pMAC, CAR-pMAC-SIRPα-KO (5×10⁵ cells) were added and co-cultured. It can be seen that cytotoxic activity (% Cytolysis) is improved by GC33-ζ-CAR introduction compared to hGPC3-introduced SK-Hep1 (Fig. 27, top left). It can be seen that the cytotoxic activity is remarkably higher than that against SK-Hep1, which does not express cancer antigen hPGC3 (Fig. 27, upper row, right). Time course changes of the adhesion signal (Normalized Cell Index value; CI value) indicating cancer cell survival when cancer cells are co-cultured with human pMAC, pMAC-SIRPα-KO, CAR-pMAC, CAR-pMAC-SIRPα-KO, and the cytotoxic activity (% Cytolysis) of cancer cells standardized to that of cancer cells cultured alone are shown in Fig. 27, middle row and lower row. GC33-ζ-CAR introduction and SIRPα deficiency most remarkably reduced the CI value of cancer cells over time and most remarkably increased % Cytolysis.

### [Industrial Applicability]

According to the method of the present invention, it is possible to provide a novel CAR-loaded platform that is stable in quality, can be supplied stably, and is superior in economic efficiency. The novel CAR-loaded platform uses proliferating macrophage-like cells (pMACs). The CAR-loaded pMAC is also effective in treating solid cancers.

This application is based on patent application No. 2022-127477 filed in Japan (filing date: August 9, 2022), the contents of which are encompassed in full herein.

## Claims

1. A proliferating macrophage-like cell (pMAC) that proliferates in a cytokine-dependent manner.

2. The pMAC according to claim 1, wherein the cytokine is a granulocyte macrophage colony stimulating factor (GM-CSF) and/or a macrophage colony stimulating factor (M-CSF).

3. The pMAC according to claim 1, wherein CD14 and CD83 are negative.

4. The pMAC according to claim 1, wherein a gene that inhibits a macrophage-like function is deleted or suppressed in expression.

5. The pMAC according to claim 4, wherein the aforementioned gene that inhibits a macrophage-like function is a signal regulatory protein α (SIRPα) gene.

6. The pMAC according to any one of claims 1 to 5, wherein the aforementioned pMAC further comprises a chimeric antigen receptor (CAR).

7. A method for producing a proliferating macrophage-like cell (pMAC), comprising a step of deleting a gene that inhibits a macrophage-like function or suppressing expression of the gene, and a step of expressing a gene that confers proliferating property.

8. The production method according to claim 7, comprising
(1) a step of, in a pluripotent stem cell, deleting the gene that inhibits a macrophage-like function or suppressing expression of the gene, and then inducing differentiation into a myeloid cell to obtain a macrophage-like cell (MAC) (step 1), and
(2) a step of expressing the gene that confers proliferating property in the MAC obtained in step 1 to obtain a proliferating macrophage-like cell (pMAC) (step 2).

9. The production method according to claim 7, comprising (A) a step of expressing a gene that confers proliferating property in a myeloid cell differentiated from a pluripotent stem cell to obtain a proliferating myeloid cell (step A), and (B) a step of, in the proliferating myeloid cell obtained in step A, deleting the gene that inhibits a macrophage-like function or suppressing expression of the gene, and then inducing differentiation to obtain a proliferating macrophage-like cell (pMAC) (step B).

10. The production method according to claim 7, wherein the gene that inhibits a macrophage-like function is a signal regulatory protein α (SIRPα) gene.

11. The production method according to claim 7, wherein the gene that confers proliferating property is at least one type selected from the group consisting of c-MYC, BMI1 and MDM2.

12. A method for producing CAR-pMAC, comprising introducing a chimeric antigen receptor (CAR) into the pMAC obtained by the production method according to any one of claims 7 to 11.

13. A method for producing CAR-pMAC, comprising
(1) a step of, in a pluripotent stem cell, deleting the gene that inhibits a macrophage-like function or suppressing expression of the gene, and then inducing differentiation into a myeloid cell to obtain a macrophage-like cell (MAC) (step 1),
(2) a step of expressing the gene that confers proliferating property in the MAC obtained in step 1 to obtain a proliferating macrophage-like cell (pMAC) (step 2), and
(3) a step of introducing a chimeric antigen receptor (CAR) (step 3).

14. The production method according to claim 13, wherein the differentiation induction into the myeloid cell in step 1 is performed by the following method (i) or (ii).
(i) performing a layered culture of embryoid body (EB) induced from the pluripotent stem cells on feeder cells in a medium containing VEGF, and further culturing same in a medium containing VEGF, SCF, and TPO, or
(ii) performing a monolayer culture of embryoid body (EB) induced from the pluripotent stem cells in a medium containing BMP-4, VEGF, and SCF without feeder cells, and further culturing same in a medium containing VEGF, TPO, and GM-CSF.

15. The production method according to claim 14, wherein the induction from the pluripotent stem cell into the embryoid body (EB) is performed by the following methods (a) and (b): (a) seeding pluripotent stem cells in a container for cell mass-forming culture to form an embryoid body (EB), wherein (b) the aforementioned container has fine spheroid wells at the bottom, and does not have a flat surface between adjacent wells.

16. A method for producing CAR-pMAC, comprising
(1) a step of, in a pluripotent stem cell, deleting the gene that inhibits a macrophage-like function or suppressing expression of the gene (step 1-1),
a step of seeding the pluripotent stem cell obtained in step 1-1, in which the gene that inhibits a macrophage-like function is deleted or expression of the gene is suppressed, in a container for cell mass-forming culture that has fine spheroid wells at the bottom, and does not have a flat surface between adjacent wells, to form an embryoid body (EB) (step 1-2),
a step of obtaining a macrophage-like cell (MAC) by the following method (i) or (ii) from the EB obtained in step 1-2 (step 1-3),
(i) performing a layered culture of the aforementioned EB on feeder cells in a medium containing VEGF, and further culturing same in a medium containing VEGF, SCF, and TPO, or
(ii) performing a monolayer culture of the aforementioned EB in a medium containing BMP-4, VEGF, and SCF without feeder cells, and further culturing same in a medium containing VEGF, TPO, and GM-CSF,
(2) a step of expressing the gene that confers proliferating property in the MAC obtained in step 1-3 to obtain a proliferating macrophage-like cell (pMAC) (step 2), and
(3) a step of introducing a chimeric antigen receptor (CAR) into the pMAC obtained in step 2 to obtain CAR-pMAC (step 3).

17. The production method according to any one of claims 13 to 16, wherein the gene that inhibits a macrophage-like function is a signal regulatory protein α (SIRPα) gene.

18. The production method according to any one of claims 13 to 16, wherein the gene that confers proliferating property is at least one type selected from the group consisting of c-MYC, BMI1 and MDM2.

19. A proliferating macrophage-like cell that satisfies the following conditions when a main component analysis based on the gene expression profile obtained by performing a whole-transcriptome analysis using, as a comparison target, a cell population along the macrophage cell lineage consisting of an iPS cell-derived myeloid cell, an iPS cell-derived macrophage, a biologically derived monocyte cell, a biologically derived macrophage M1, a biologically derived macrophage M2, and a biologically derived macrophage M0 is performed and the top two components with high contribution rates are selected as the analysis targets:
condition **1:** no overlapping with plots of known macrophage cell lineages
condition **2:** PC1 is 100 or more, and PC2 is plotted in the range of -80 to **0.**

20. A pharmaceutical composition comprising the proliferating macrophage-like cell according to claim 6 and a pharmaceutically acceptable carrier.

21. The pharmaceutical composition according to claim 20, which is for treating at least one disease selected from the group consisting of a disease associated with a tumor or cancer, a neurodegenerative disease, an inflammatory disease, a cardiovascular disease, a fibrotic disease, and amyloidosis.
